# FASCICULE DE BREVET EUROPEEN

(11) **EP 0 923 929 B1**
(45) Date de publication et mention de la délivrance du brevet: **12.05.2004**
(21) Numéro de dépôt: 98402939.7
(22) Date de dépôt: 25.11.1998
(51) Int. Cl.: A61K 7/13, C07D 487/04

(54) **COMPOSITIONS DE TEINTURE DES FIBRES KERATINIQUES CONTENANT DES PYRAZOLO-AZOLES; LEUR UTILISATION POUR LA TEINTURE COMME BASE D'OXYDATION, PROCEDE DE TEINTURE; NOUVEAUX PYRAZOLO-AZOLES.**
ZUSAMMENSETZUNG ZUR FÄRBUNG VON KERATINISCHEN FASERN ENTHALTEND PYRAOLOAZOLE ALS OXIDATIONSMITTEL, FÄRBEVERFAHREN UND PYRAZOLOAZOLE
KERATIN FIBRE DYE COMPOSITION CONTAINING PYRAZOLO-AZOLE COMPOUNDS, USE THEREOF AS OXIDATION AGENTS, AND DYEING METHOD; PYRAZOLOAZOLES

(30) Priorité: 16.12.1997 FR 9715947
(43) Date de publication de la demande: 23.06.1999
(73) Titulaire: L'OREAL, 75008 Paris (FR)
(72) Inventeur: Vidal, Laurent, 75013 Paris (FR); Maubru, Mireille, 78400 Chatou (FR)
(74) Mandataire: Goulard, Sophie

(56) Documents cités:
- WO-A-93/07849
- WO-A-97/35551

## Description

L'invention a pour objet une composition pour la teinture d'oxydation des fibres kératiniques, et en particulier des fibres kératiniques humaines telles que les cheveux humains contenant au moins un composé pyrazolo-azole à titre de base d'oxydation, le procédé de teinture mettant en oeuvre cette composition, ainsi que certains nouveaux pyrazolo-azoles.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est-à-dire permettre d'obtenir des écarts de coloration les plus faibles possible tout au long d'une même fibre kératinique, qui peut être en effet, différemment sensibilisée (i.e. abîmée) entre sa pointe et sa racine. Ils doivent également présenter une bonne stabilité chimique dans les formulations et un bon profil toxicologique.

Or, la demanderesse vient maintenant de découvrir qu'il est possible d'obtenir de nouvelles teintures puissantes, peu sélectives et particulièrement résistantes aux diverses agressions que peuvent subir les fibres kératiniques, capables d'engendrer des colorations intenses dans des nuances variées, en utilisant à titre de base d'oxydation des composés pyrazolo-azoles de formule (I) ci-après définie.

Il a déjà été proposé de teindre les fibres kératiniques avec des compositions comprenant un composé pyrazole substitué par un cycle saturé pouvant contenir 5 ou 6 chaînons, notamment dans la demande de brevet WO 93 107 849.

Il est également connu de teindre les fibres kératiniques avec des compositions comprenant un composé diamino ou triamino pyrazole à titre de base d'oxydation en association avec un méta-aminophénol halogéné en ortho du phénol à titre de coupleur, notamment dans la demande de brevet WO 99/11 231.

Par ailleurs, il est connu d'utiliser des compositions comprenant un composé pyrazolo-azole particulier à titre de coupleur pour la teinture des fibres kératiniques, notamment dans la demande de brevet WO 97/35551.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :
- à titre de base d'oxydation, au moins un composé pyrazolo-azole de formule (I) et/ou au moins l'un de ses sels d'addition avec un acide : 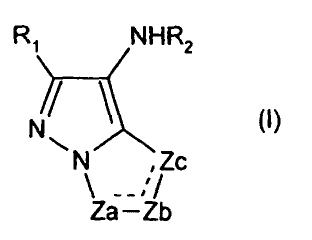 dans laquelle :
- R₁ représente : un atome d'hydrogène ; un radical alkyle en C₁-C₂₀, linéaire ou ramifié, éventuellement substitué par 1 ou 2 radicaux R choisis dans le groupe constitué par halogène, nitro, cyano, hydroxyle, alcoxy, aryloxy, amino, alkylamino, acylamino, carbamoyle, sulfonamido, sulfamoyle, imido, alkylthio, arylthio, aryle tel que phényle ou naphtyle, alcoxy(C₁-C₄)carbonyle, acyle ; un radical aryle (tel que phényle ou naphtyle), éventuellement substitué par 1 ou 2 radicaux R tels que précédemment définis ; un hétérocycle à 5 ou 6 chaînons possédant au moins un atome d'azote, d'oxygène ou de soufre (tel que pyridyle, quinolyle, pyrrolyle, morpholyle, furanyle, tétrahydrofuranyle, pyrazolyle, triazolyle, tétrazolyle, thiazolyle, oxazolyle, imidazolyle ou thiadiazolyle), ledit hétérocycle pouvant être substitué par 1 ou 2 radicaux R tels que définis précédemment ; un radical trifluoromethyle; un radical phenyle substitué par un trifluoromethyle; un cycle phényle substitué par un radical alkyle en C₁-C₄; lorsque R₁ désigne un radical alkyle en C₁-C₄, un radical aryle ou un hétérocycle à 5 ou 6 chaînons (tels que définis ci-dessus), il peut alors être relié à l'atome de carbone du noyau pyrazole par l'intermédiaire d'un atome d'oxygène, d'azote ou de soufre (dans ce cas, R₁ devient XR₁ avec X = O, NH ou S) ;
   R₁ peut en outre désigner un atome d'halogène (tel que le brome, le chlore ou le fluor) ; un radical acyle ; un radical sulfonyle un radical sulfinyle ; un radical phosphonyle, un radical carbamoyle ; un radical sulfamoyle ; un radical cyano ; un radical siloxy ; un radical amino ; un radical acylamino ; un radical acyloxy ; un radical carbamoyloxy ; un radical sulfonamide ; un radical imide ; un radical uréido ; un radical sulfamoylamino ; un radical alcoxy(C₁-C₄)carbonylamino ; un radical aryloxycarbonylamino ; un radical alcoxy(C₁-C₄)carbonyle ; un radical aryloxycarbonyle ; un radical carboxyle ; un radical hydroxyle;
   un radical (CH₂)ₚ-X-(CH₂)_{q}-OR' dans lequel p et q sont des nombres entiers, identiques ou différents, compris entre 1 et 3 inclusivement, R' représente un atome d'hydrogène ou un radical méthyle et X désigne un atome d'oxygène ou un groupe NR" dans lequel R" désigne un atome d'hydrogène ou un radical méthyle
- R₂ désigne un atome d'hydrogène ; un radical alkyle en C₁-C₄ ; un radical monohydroxyalkyle en C₁-C₄ ; un radical polyhydroxyalkyle en C₂-C₄ ; un radical aminoalkyle en C₁-C₄ ; un radical (CH₂)ₚ-X-(CH₂)_{q}OR' dans lequel p et q sont des nombres entiers, identiques ou différents, compris entre 1 et 3 inclusivement, R' représente un atome d'hydrogène ou un radical méthyle, X désigne un atome d'oxygène ou un groupement NR" dans lequel R" représente un atome d'hydrogène ou un radical méthyle ; un radical alcoxy(C₁-C₄)alkyle en C₂-C₄ ; un radical di-alkyl(C₁-C₄)aminoalkyle en C₁-C₄ ;
- Zₐ, Z_{b}, Z_{c} représentent, indépendamment l'un de l'autre, un atome d'azote, un atome de carbone portant un radical R₃ ou R₄, R₃ et R₄ ayant les mêmes significations que celles indiquées ci-dessus pour le radical R₁ ainsi que phenyle substitué par un radical alkyle en C₁-C₄; un radical methane sulfonyle; sous réserve que l'un au moins des radicaux Zₐ, Z_{b} et Z_{c} soit différent d'un atome de carbone ; R₃ et R₄ peuvent également former ensemble un cycle aromatique substitué ou non tel qu'un cycle phényle.

Parmi les radicaux R₁ des composés de formule (I) définie ci-dessus, on préfère les radicaux choisis dans le groupe constitué par un atome d'hydrogène ; un radical alkyle en C₁-C₄, linéaire ou ramifié ; un cycle phényle ; un cycle phényle substitué par un atome d'halogène, un radical alkyle en C₁-C₄, un radical alcoxy en C₁-C₄, un groupe nitro, un groupe amino, un groupe trifluorométhyle ou par un radical alkylamino en C₁-C₄ ; un radical benzyle ; un radical benzyle substitué par un atome d'halogène, un radical alkyle en C₁-C₄, un radical alcoxy en C₁-C₄, un groupe nitro, un groupe amino, un groupe trifluorométhyle ou par un radical alkylamino en C₁-C₄ ; un hétérocycle choisi parmi les cycles thiophène, furane ou pyridine ; un radical trifluorométhyle ; un radical (CH₂)ₚ-X-(CH₂)_{q}-OR' dans lequel p et q sont des nombres entiers, identiques ou différents, compris entre 1 et 3 inclusivement, R' représente un atome d'hydrogène ou un radical méthyle et X désigne un atome d'oxygène ou un groupe NR" dans lequel R" désigne un atome d'hydrogène ou un radical méthyle ; un radical hydroxyalkyle en C₁-C₄ ; un radical aminoalkyle en C₁-C₄ ; un radical alkylamino en C₁-C₄ ; un radical dialkylamino en C₁-C₄ ; un radical arylamino ; un radical alcoxy choisi parmi méthoxy, éthoxy et phénoxy ; un atome d'halogène choisi parmi le brome, le chlore et le fluor ; un groupe carboxyle un radical alcoxycarbonyle en C₁-C₄ ; un radical phényloxy-carbonyle ; un radical méthylthio ; un radical éthylthio un radical phénylthio ; un radical méthanesulfonyle ; un radical cyano ; un radical amino ; un radical hydroxyle.

Parmi les radicaux R₁ des composés de formule (I) définie ci-dessus, on préfère plus particulièrement les radicaux choisis dans le groupe constitué par un atome d'hydrogène ; un radical alkyle choisi parmi les radicaux méthyle, éthyle, isopropyle et ter-butyle ; un atome d'halogène choisi parmi le chlore et le fluor ; phényle ; toluyle ; 4-chlorophényle ; 4-méthoxyphényle ; 3-méthoxyphényle ; 2-méthoxyphényle ; benzyle ; un hétérocycle choisi parmi les cycles pyridyle, furyle et thiènyle ; trifluorométhyle ; hydroxyméthyle ; aminométhyle ; méthoxy ; éthoxy ; méthylamino ; éthylamino ; diméthylamino ; carboxyle ; méthoxycarbonyle ; éthoxycarbonyle et cyano.

Encore plus particulièrement, on préfère les radicaux R₁ choisis dans le groupe constitué par : un atome d'hydrogène ; un atome de chlore ; un radical méthyle ; éthyle ; phényle ; toluyle ; 4-chlorophényle ; 4-méthoxyphényle ; benzyle ; trifluorométhyle ; méthoxy ; éthoxy ; carboxyle ; méthylamino ; diméthylamino et cyano.

Parmi les radicaux R₂ des composés de formule (I) définie ci-dessus, on préfère les radicaux choisis dans le groupe constitué par un atome d'hydrogène ; un radical méthyle et un radical β-hydroxyéthyle.

Encore plus particulièrement, on préfère que le radical R₂ représente un atome d'hydrogène.

Parmi les radicaux R₃ et R₄ des composés de formule (I) définie ci-dessus, on préfère les radicaux choisis dans le groupe constitué par un atome d'hydrogène ; un radical hydroxyle ; un radical amino ; un radical alkyle en C₁-C₄, linéaire ou ramifié ; un radical trifluorométhyle ; un cycle phényle ; un cycle phényle substitué par un ou deux radicaux R choisis parmi un atome d'halogène, un radical alkyle en C₁-C₄, un radical alcoxy en C₁-C₄, un radical hydroxyle, un radical carboxyle, un groupe nitro, un radical alkylthio en C₁-C₄, un groupe méthylènedioxy, un groupe amino, un groupe trifluorométhyle et un radical alkylamino en C₁-C₄ ; un radical benzyle ; un radical benzyle substitué par un atome d'halogène, un radical méthyle ou isopropyle, un radical méthoxy; un radical monohydroxyalkyle en C₁-C₄ ; un radical aminoalkyle en C₁-C₄ ; un radical alkyl(C₁-C₄)aminoalkyle en C₁-C₄ ; un radical alcoxy choisi parmi les radicaux méthoxy, éthoxy et phénoxy ; un radical méthylthio ; un radical éthylthio ; un radical phénylthio ; un radical méthanesulfonyle ; un cycle aromatique substitué ou non et formé conjointement par les radicaux R₃ et R₄ tel qu'un cycle phényle, pyridyle ou phényle substitué par un radical sulfonyle, un atome d'halogène, un radical alcoxy en C₁-C₄, un radical alkyle en C₁-C₄, un groupe nitro, un groupe cyano, un radical amino, un radical alkylamino en C₁-C₄, ou par un radical trifluorométhyle.

Parmi les radicaux R₃ et R₄ des composés de formule (I) définie ci-dessus, on préfère plus particulièrement les radicaux choisis dans le groupe constitué par un atome d'hydrogène ; un radical alkyle choisi parmi les radicaux méthyle, éthyle, isopropyle, n-propyle et ter-butyle ; un cycle phényle ; un cycle toluyle ; un cycle 2-, 3- ou 4-chlorophényle ; un cycle 3- ou 4-hydroxyphényle ; un cycle 3- ou 4-aminophényle ; un cycle 3- ou 4-méthoxyphényle ; un cycle 4-trifluorométhylphényle ; un cycle benzyle ; un radical trifluorométhyle ; un radical hydroxyméthyle ; un radical hydroxyéthyle ; un radical hydroxyisopropyle ; un radical aminométhyle ou aminoéthyle ; un radical méthoxy ou éthoxy ; un radical méthylthio ou éthylthio ; un cycle aromatique substitué ou non et formé conjointement par les radicaux R₃ et R₄ tel qu'un cycle phényle, toluyle, sulfonylphényle et chlorophényle.

Encore plus particulièrement, on préfère que les radicaux R₃ et R₄ soient choisis dans le groupe constitué par un atome d'hydrogène ; un radical méthyle ; un radical éthyle ; un radical isopropyle ; un cycle phényle ; un cycle 4-chlorophényle ; un cycle 4-méthoxyphényle ; un cycle 4-aminophényle ; un radical méthoxy ou éthoxy ; un radical méthylthio ou éthylthio et un cycle phényle formé conjointement par les radicaux R₃ et R₄.

Parmi les composés de formule (I) conformes à l'invention, on peut plus particulièrement citer :
i) les pyrazolo [1,5-b] 1,2,4-triazoles de formule particulière (la) suivante, et leurs sels d'addition avec un acide : 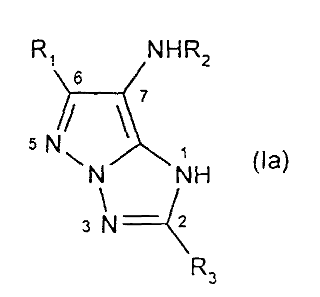
ii) les pyrazolo [3,2-c] 1,2,4-triazoles de formule particulière (Ib) suivante, et leurs sels d'addition avec un acide : 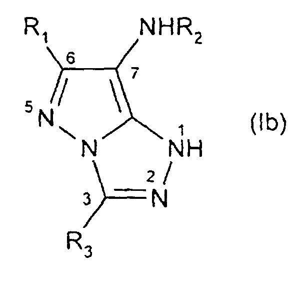
iii) les pyrazolotétrazoles de formule particulière (Ic) suivante, et leurs sels d'addition avec un acide : 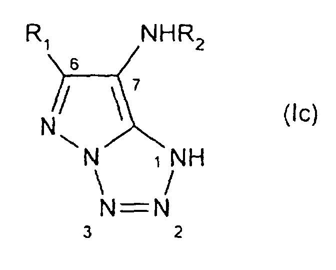
iv) les pyrazolo [1,5-a] imidazoles de formule particulière (Id) suivante, et leurs sels d'addition avec un acide : 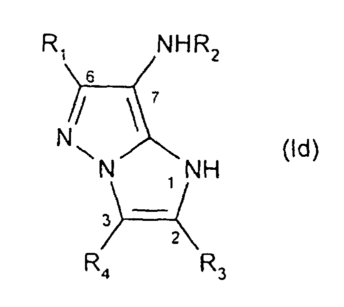
v) les pyrazolo [5,1-e] pyrazoles de formule particulière (le) suivante, et leurs sels d'addition avec un acide : 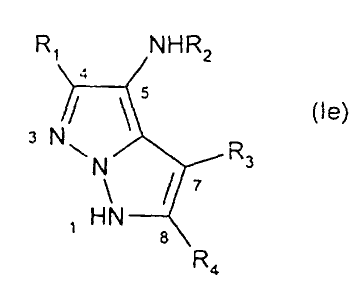
vi) les pyrazolo [5,1-e] 1,2,3-triazoles de formule particulière (If) suivante, et leurs sels d'addition avec un acide : 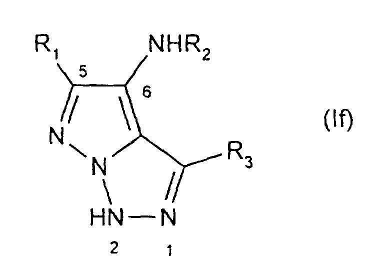
formules dans lesquelles R₁, R₂, R₃ et R₄ ont les mêmes significations que celles indiquées ci-dessus pour la formule (I).

A titre d'exemples de composés de formules (la) ou (Ib), on peut citer en particulier ceux pour lesquels :
- R₁ désigne un atome d'hydrogène, un radical méthyle, trifluorométhyle, carboxyle, phényle, éthoxy, cyano, éthylthio, amino ou hydroxyle ;
- R₂ désigne un atome d'hydrogène ;
- R₃ désigne un atome d'hydrogène, un radical méthyle, β-aminoéthyle, éthyle, isopropyle, phényle, β-hydroxyéthyle, méthylthio ou éthoxy.

A titre d'exemples de composés de formule (Ic), on peut citer en particulier ceux pour lesquels :
- R₁ désigne un atome d'hydrogène, un radical méthyle, trifluorométhyle, carboxyle, phényle, éthoxy ou cyano ;
- R₂ désigne un atome d'hydrogène.

A titre d'exemples de composés de formule (Id), on peut citer en particulier ceux pour lesquels :
- R₁ désigne un atome d'hydrogène, un radical méthyle, trifluorométhyle, carboxyle, phényle, éthoxy, cyano, amino, éthylthio ou hydroxyle ;
- R₂ désigne un atome d'hydrogène ;
- R₃ et R₄ désignent respectivement un atome d'hydrogène et un atome d'hydrogène, un atome d'hydrogène et un radical méthyle, un radical méthyle et un atome d'hydrogène, un atome d'hydrogène et un radical phényle, un radical hydroxyle et un atome d'hydrogène, ou bien R₃ et R₄ forment conjointement un cycle phényle.

A titre d'exemples de composés de formule (le), on peut citer en particulier ceux pour lesquels :
- R₁ désigne un atome d'hydrogène, un radical méthyle, trifluorométhyle, carboxyle, phényle, éthoxy ou cyano ;
- R₂ désigne un atome d'hydrogène ;
- R₃ et R₄ désignent respectivement un atome d'hydrogène et un radical méthyle, un radical méthyle et un atome d'hydrogène, un radical méthyle et un radical méthyle, un atome d'hydrogène et un radical phényle, un atome d'hydrogène et un radical amino.

A titre d'exemples de composés de formule (If), on peut citer en particulier ceux pour lesquels :
- R₁ désigne un atome d'hydrogène, un radical méthyle, trifluorométhyle, carboxyle, phényle, éthoxy ou cyano ;
- R₂ désigne un atome d'hydrogène ;
- R₃ désigne un atome d'hydrogène ou un radical méthyle.

Parmi les composés de formules (la) utilisables dans les compositions tinctoriales conformes à l'invention, on préfère plus particulièrement :
- le 7-amino-2-méthyl pyrazolo [1,5-b]-1,2,4-triazole ;
- le 7-amino-2-éthyl pyrazolo [1,5-b]-1,2,4-triazole ;
- le 7-amino-2-isopropyl pyrazolo [1,5-b]-1,2,4-triazole ;
- le 7-amino-2-phényl pyrazolo [1,5-b]-1,2,4-triazole ;
- le 7-amino-2,6-diméthyl pyrazolo [1,5-b]-1,2,4-triazole ;
- le 7-amino-6-méthyl-2-éthyl pyrazolo [1,5-b]-1,2,4-triazole ;
- le 7-amino-6-méthyl-2-isopropyl pyrazolo [1,5-b]-1,2,4-triazole ;
- le 7-amino- 6-méthyl-2-phényl pyrazolo [1,5-b]-1,2,4-triazole ;
- le 7-amino-6-carboxy-2-méthyl pyrazolo [1,5-b]-1,2,4-triazole ;
- le 7-amino-6-carboxy-2-éthyl pyrazolo [1,5-b]-1,2,4-triazole ;
- le7-amino- 6-carboxy-2-isopropyl pyrazolo [1,5-b]-1,2,4-triazole ;
- le 7-amino-6-carboxy-2-phényl pyrazolo [1,5-b]-1,2,4-triazole ;
- le 7-amino-6-phényl-2-méthyl pyrazolo [1,5-b]-1,2,4-triazole ;
- le 7-amino-6-phényl-2-éthyl pyrazolo [1,5-b]-1,2,4-triazole ;
- le 7-amino-6-phényl-2-isopropyl pyrazolo [1,5-b]-1,2,4-triazole ;
- le 6,7-diamino-2-méthyl pyrazolo [1,5-b]-1,2,4-triazole ;
- le 6,7-diamino-2-éthyl pyrazolo [1,5-b]-1,2,4-triazole ;
- le 6,7-diamino-2-isopropyl pyrazolo [1,5-b]-1,2,4-triazole ;
- le 6,7-diamino-2-phényl pyrazolo [1,5-b]-1,2,4-triazole ;
- le 7-amino-6-éthylthio-2-méthyl pyrazolo [1,5-b]-1,2,4-triazole ;
- le 7-amino-6-éthylthio-2-éthyl pyrazolo [1,5-b]-1,2,4-triazole ;
- le 7-amino-6-éthylthio-2-isopropyl pyrazolo [1,5-b]-1,2,4-triazole ;
- le 7-amino-6-éthylthio-2-phényl pyrazolo [1,5-b]-1,2,4-triazole ;
- le 7-amino-6-éthoxy-2-méthyl pyrazolo [1,5-b]-1,2,4-triazole ;
- le 7-amino-6-éthoxy-2-éthyl pyrazolo [1,5-b]-1,2,4-triazole ;
- le 7-amino-6-éthoxy-2-isopropyl pyrazolo [1,5-b]-1,2,4-triazole ;
- le 7-amino-6-éthoxy-2-phényl pyrazolo [1,5-b]-1,2,4-triazole ;
- le 7-amino-6-méthyl-2-(2'-aminoéthyl) pyrazolo [1,5-b]-1,2,4-triazole ;
- le 7-amino-6-carboxy-2-(2'-aminoéthyl) pyrazolo [1,5-b]-1,2,4-triazole ;
- le 7-amino-6-phényl-2-(2'-aminoéthyl) pyrazolo [1,5-b]-1,2,4-triazole ;
- le 7-amino-6-éthylthio-2-(2'-aminoéthyl) pyrazolo [1,5-b]-1,2,4-triazole ;
- le 7-amino-2-(2'-aminoéthyl) pyrazolo [1,5-b]-1,2,4-triazole ;
- le 7-amino-2-(2'-hydroxyéthyl) pyrazolo [1,5-b]-1,2,4-triazole ;
- le 7-amino-6-méthyl-2-(2'-hydroxyéthyl) pyrazolo [1,5-b]-1,2,4-triazole ;
- le 7-amino-6-éthylthio-2-(2'-hydroxyéthyl) pyrazolo [1,5-b]-1,2,4-triazole ;
- le 7-amino-6-carboxy-2-(2'-hydroxyéthyl) pyrazolo [1,5-b]-1,2,4-triazole ;
- le 7-amino-6-phényl-2-(2'-hydroxyéthyl) pyrazolo [1,5-b]-1,2,4-triazole ;
- le 6,7-diamino-2-(2'-aminoéthyl)-pyrazolo [1,5-b]-1,2,4-triazole ;
- le 6,7-diamino-2-(2'-hydroxyéthyl)-pyrazolo [1,5-b]-1,2,4-triazole ;
- le 7-amino-6-éthoxy-2-(2'-aminoéthyl)-pyrazolo [1,5-b]-1,2,4-triazole ;
- le 7-amino-6-éthoxy-2-(2'-hydroxyéthyl)-pyrazolo [1,5-b]-1,2,4-triazole ;
et leurs sels d'addition avec un acide.

Parmi les composés de formules (Ib) utilisables dans les compositions tinctoriales conformes à l'invention, on préfère plus particulièrement :
- le 7-amino-3-méthyl pyrazolo [3,2-c]-1,2,4-triazole ;
- le 7-amino-3-éthyl pyrazolo [3,2-c]-1,2,4-triazole ;
- le 7-amino-3-isopropyl pyrazolo [3,2-c]-1 ,2,4-triazole ;
- le 7-amino-3-phényl pyrazolo [3,2-c]-1,2,4-triazole ;
- le 7-amino-3-(2'-aminoéthyl) pyrazolo [3,2-c]-1,2,4-triazole ;
- le 7-amino-3-(2'-hydroxyéthyl) pyrazolo [3,2-c]-1,2,4-triazole ;
- le 7-amino-6-méthyl-3-éthyl pyrazolo [3,2-c]-1,2,4-triazole ;
- le 7-amino-3,6-diméthyl pyrazolo [3,2-c]-1,2,4-triazole ;
- le 7-amino-6-méthyl-3-isopropyl pyrazolo [3,2-c]-1,2,4-triazole ;
- le 7-amino-6-méthyl-3-phényl pyrazolo [3,2-c]-1,2,4-triazole ;
- le 7-amino-6-méthyl-3-(2'-aminoéthyl) pyrazolo [3,2-c]-1,2,4-triazole ;
- le 7-amino-6-méthyl-3-(2'-hydroxyéthyl) pyrazolo [3,2-c]-1,2,4-triazole ;
- le 7-amino-6-méthyl-3-méthylthio pyrazolo [3,2-c]-1,2,4-triazole ;
- le 7-amino-6-phényl-3-méthyl pyrazolo [3,2-c]-1,2,4-triazole ;
- le 7-amino-6-phényl-3-éthyl pyrazolo [3,2-c]-1,2,4-triazole ;
- le 7-amino-6-phényl-3-isopropyl pyrazolo [3,2-c]-1,2,4-triazole ;
- le 7-amino-6-phényl-3-phényl pyrazolo [3,2-c]-1,2,4-triazole ;
- le 7-amino-6-phényl-3-(2'-aminoéthyl) pyrazolo [3,2-c]-1,2,4-triazole ;
- le 7-amino-6-phényl-3-(2'-hydroxyéthyl) pyrazolo [3,2-c]-1,2,4-triazole ;
- le 7-amino-6-phényl-3-méthylthio pyrazolo [3,2-c]-1,2,4-triazole ;
- le 7-amino-6-éthylthio-3- méthyl pyrazolo [3,2-c]-1,2,4-triazole ;
- le 7-amino-6-éthylthio-3- éthyl pyrazolo [3,2-c]-1,2,4-triazole ;
- le 7-amino-6-éthylthio-3-isopropyl pyrazolo [3,2-c]-1,2,4-triazole ;
- le 7-amino-6-éthylthio-3-phényl pyrazolo [3,2-c]-1,2,4-triazole ;
- le 7-amino-6-éthylthio-3-(2'-aminoéthyl) pyrazolo [3,2-c]-1,2,4-triazole ;
- le 7-amino-6-éthylthio-3-(2'-hydroxyéthyl) pyrazolo [3,2-c]-1,2,4-triazole ;
- le 7-amino-6-trifluorométhyl-3-méthylthio pyrazolo [3,2-c]-1,2,4-triazole ;
- le 7-amino-6-trifluorométhyl pyrazolo [3,2-c]-1,2,4-triazole ;
- le 7-amino-6-carboxy-3-méthyl pyrazolo [3,2-c]-1,2,4-triazole ;
- le 7-amino-6-carboxy-3-éthyl pyrazolo [3,2-c]-1,2,4-triazole ;
- le 7-amino-6-carboxy-3-isopropyl pyrazolo [3,2-c]-1,2,4-triazole ;
- le 7-amino-6-carboxy-3-phényl pyrazolo [3,2-c]-1,2,4-triazole ;
- le 7-amino-6-carboxy-3-(2'-aminoéthyl) pyrazolo [3,2-c]-1,2,4-triazole ;
- le 7-amino-6-carboxy-3-(2'-hydroxyéthyl) pyrazolo [3,2-c]-1,2,4-triazole ;
- le 6,7-diamino-3-méthyl pyrazolo [3,2-c]-1,2,4-triazole ;
- le 6,7-diamino-3-éthyl pyrazolo [3,2-c]-1,2,4-triazole ;
- le 6,7-diamino-3-phényl pyrazolo [3,2-c]-1,2,4-triazole ;
- le 6,7-diamino-3-isopropyl pyrazolo [3,2-c]-1,2,4-triazole ;
- le 7-amino-6-éthoxy-3-méthyl pyrazolo [3,2-c]-1,2,4-triazole ;
- le 7-amino-6-éthoxy-3-éthyl pyrazolo [3,2-c]-1,2,4-triazole ;
- le 7-amino-6-éthoxy-3-isopropyl pyrazolo [3,2-c]-1,2,4-triazole ;
- le 7-amino-6-éthoxy-3-phényl pyrazolo [3,2-c]-1,2,4-triazole ;
- le 7-amino-6-éthoxy-3-(2'-aminoéthyl) pyrazolo [3,2-c]-1,2,4-triazole ;
- le 7-amino-6-éthoxy-3-(2'-hydroxyéthyl) pyrazolo [3,2-c]-1,2,4-triazole ;
- le 6,7-diamino-3-(2'-aminoéthyl) pyrazolo [3,2-c]-1,2,4-triazole ;
- le 6,7-diamino-3-(2'-hydroxyéthyl) pyrazolo [3,2-c]-1,2,4-triazole ;
et leurs sels d'addition avec un acide.

Parmi les composés de formules (Ic) utilisables dans les compositions tinctoriales conformes à l'invention, on préfère plus particulièrement :
- le 7-amino-6-méthyl pyrazolo [5,1-e] tétrazole ;
- le 7-amino-6-phényl pyrazolo [5,1-e] tétrazole ;
- le 7-amino-6-carboxy pyrazolo [5,1-e] tétrazole ;
et leurs sels d'addition avec un acide.

Parmi les composés de formules (Id) utilisables dans les compositions tinctoriales conformes à l'invention, on préfère plus particulièrement :
- le 7-amino-2-méthyl pyrazolo [1,5-a] imidazole ;
- le 7-amino-2-phényl pyrazolo [1,5-a] imidazole ;
- le 7-amino pyrazolo [1,5-a] benzimidazole ;
- le 7-amino-6-méthyl pyrazolo [1,5-a] imidazole ;
- le 7-amino-2,6-diméthyl pyrazolo [1,5-a] imidazole ;
- le 7-amino-6-méthyl-2-phényl pyrazolo [1,5-a] imidazole ;
- le 7-amino-6-méthyl pyrazolo [1,5-a] benzimidazole ;
- le 7-amino-6-phényl pyrazolo [1,5-a] imidazole ;
- le 7-amino-6-phényl-2-méthyl pyrazolo [1,5-a] imidazole ;
- le 7-amino-2,6-diphényl pyrazolo [1,5-a] imidazole ;
- le 7-amino-6-phényl pyrazolo [1,5-a] benzimidazole ;
- le 7-amino-6-carboxy pyrazolo [1,5-a] imidazole ;
- le 7-amino-6-carboxy-2-méthyl pyrazolo [1,5-a] imidazole ;
- le 7-amino-6-carboxy-2-phényl pyrazolo [1,5-a] imidazole ;
- le 7-amino-6-carboxy pyrazolo [1,5-a] benzimidazole ;
- le 7-amino-6-éthoxy pyrazolo [1,5-a] imidazole ;
- le 7-amino-6-éthoxy-2-méthyl pyrazolo [1,5-a] imidazole ;
- le 7-amino-6-éthoxy-2-phényl pyrazolo [1,5-a] imidazole ;
- le 7-amino-6-éthoxy pyrazolo [1,5-a] benzimidazole ;
- le 7-amino-6-trifluorométhyl pyrazolo [1,5-a] benzimidazole ;
- le 6,7-diamino pyrazolo [1,5-a] imidazole ;
- le 6,7-diamino-2-méthyl pyrazolo [1,5-a] imidazole ;
- le 6,7-diamino-2-phényl pyrazolo [1,5-a] imidazole ;
- le 6,7-diamino pyrazolo [1,5-a] benzimidazole ;
- le 7-amino-6-éthylthio pyrazolo [1,5-a] imidazole ;
- le 7-amino-6-éthylthio-2-méthyl pyrazolo [1,5-a] imidazole ;
- le 7-amino-6-éthylthio-2-phényl pyrazolo [1,5-a] imidazole ;
- le 7-amino-6-méthyl-2-hydroxy pyrazolo [1,5-a] imidazole ;
- le 7-amino-6-phényl-2-hydroxy pyrazolo [1,5-a] imidazole ;
- le 7-amino-6-éthoxy-2-hydroxy pyrazolo [1,5-a] imidazole ;
- le 7-amino-6-éthylthio-2-hydroxy pyrazolo [1,5-a] imidazole ;
- le 6,7-diamino-2-hydroxy pyrazolo [1,5-a] imidazole ;
et leurs sels d'addition avec un acide.

Parmi les composés de formules (le) utilisables dans les compositions tinctoriales conformes à l'invention, on préfère plus particulièrement :
- le 5,8-diamino-4-méthyl pyrazolo [5,1-e] pyrazole ;
- le 5,7,8-triamino-4-méthyl pyrazolo [5,1-e] pyrazole ;
et leurs sels d'addition avec un acide.

Parmi les composés de formules (If) utilisables dans les compositions tinctoriales conformes à l'invention, on préfère plus particulièrement :
- le 6-amino-5-méthyl pyrazolo [5,1-e]-1,2,3-triazole ;
- le 6-amino-5-phényl pyrazolo [5,1-e]-1,2,3-triazole ;
et leurs sels d'addition avec un acide.

Le ou les composés pyrazolo-azoles de formule (I) ci-dessus, représentent de préférence de 0,0005 à 12 % en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le mono-méthyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 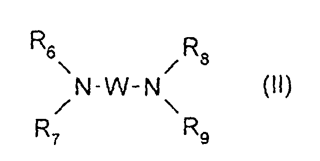 dans laquelle W est un reste propylène éventuellement substitué par un groupement hydroxyle ou un radical alkyle en C₁-C₄ ; R₆, R₇, R₈ et R₉, identiques ou différents, représentent un atome d'hydrogène, un radical alkyle en C₁-C₄ ou hydroxyalkyle en C₁-C₄.

Selon une forme de réalisation préférée, la composition tinctoriale conforme à l'invention renferme en outre un ou plusieurs coupleurs de façon à modifier ou à enrichir en reflets les nuances obtenues en mettant en oeuvre les composés de formule (I).

Les coupleurs utilisables dans la composition tinctoriale conforme à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthyl)amino 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, la 1H 3-méthyl pyrazol 5-one, la 1-phényl 3-méthyl pyrazol 5-one, le 2,6-diméthyl pyrazolo [1,5-b]-1,2,4-triazole, le 2,6-diméthyl [3,2-c]-1,2,4-triazole, le 6-méthyl pyrazolo [1,5-a] benzimidazole, et leurs sels d'addition avec un acide.

Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut encore contenir, en plus des colorants définis ci-dessus, au moins une base d'oxydation additionnelle qui peut être choisie parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et des bases hétérocycliques différentes des composés pyrazolo-azoles de formule (I) utilisés conformément à l'invention.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-(β-hydroxyéthyl) paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-(β-hydroxyéthyloxy) paraphénylènediamine, la 2-(β-acétylaminoéthyloxy) paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-(β-hydroxyéthyl) paraphénylènediamine, la 2-(β-hydroxyéthyloxy) paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-(β-acétylaminoéthyloxy) paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino 2-propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Lorsqu'elles sont utilisées, la ou les bases d'oxydation additionnelles représentent de préférence de 0,0005 à 12 % en poids environ du poids total de la composition tinctoriale, et encore plus préférentiellement de 0,005 à 6 % en poids environ de ce poids.

D'une manière générale, les sels d'addition avec un acide utilisables dans le cadre des compositions tinctoriales de l'invention (composés de formule (I), bases d'oxydation additionnelles et coupleurs) sont notamment choisis parmi les chlorhydrates, les bromhydrates, les sulfates, les citrates, les succinates, les tartrates, les tosylates, les benzènesulfonates, les lactates et les acétates.

La composition tinctoriale conforme à l'invention peut en outre renfermer un ou plusieurs colorants directs pouvant notamment être choisis parmi les colorants nitrés de la série benzéniques.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet l'utilisation des composés de formule (I) telle que définie précédemment à titre de base d'oxydation dans des compositions pour la teinture d'oxydation des fibres kératiniques et en particulier des fibres kératiniques humaines telles que les cheveux.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, pendant un temps suffisant pour développer la coloration désirée, soit à l'air, soit à l'aide d'un agent oxydant.

Selon une première forme de mise en oeuvre du procédé de l'invention, la coloration des fibres peut être effectuée sans addition d'un agent oxydant, au seul contact de l'oxygène de l'air.

Dans ce cas, la composition tinctoriale peut alors éventuellement contenir des catalyseurs d'oxydation, afin d'accélérer le processus d'oxydation.

A titre de catalyseurs d'oxydation, on peut plus particulièrement citer les sels métalliques tels que les sels de manganèse, de cobalt, de cuivre, de fer, d'argent et de zinc.

De tels composés sont, par exemple, le diacétate de manganèse tétrahydrate, le dichlorure de manganèse, le triacétate de manganèse et ses hydrates, le trichlorure de manganèse, le dichlorure de zinc, le diacétate de zinc dihydrate, le carbonate de zinc, le dinitrate de zinc, le sulfate de zinc, le dichlorure de fer, le sulfate de fer, le diacétate de fer, le diacétate de cobalt tétrahydrate, le carbonate de cobalt, le dichlorure de cobalt, le sulfate de cobalt heptahydrate, le chlorure cuivrique, le nitrate d'argent ammoniacal.

Lorsqu'ils sont utilisés, ces sels métalliques sont généralement mis en oeuvre dans des proportions variant entre 0,001 et 4 % en poids d'équivalent métal par rapport au poids total de la composition tinctoriale et de préférence entre 0,005 et 2 % en poids d'équivalent métal par rapport au poids total de la composition tinctoriale.

Selon une deuxième forme de mise en oeuvre du procédé de l'invention, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon cette deuxième forme de mise en oeuvre du procédé de teinture de l'invention, on mélange de préférence, au moment de l'emploi, la composition tinctoriale décrite ci-dessus avec une composition oxydante contenant, dans un milieu appropriée pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates et les enzymes telles que les peroxydases et les oxydo-réductases à 2 électrons. Le peroxyde d'hydrogène est particulièrement préféré.

Le pH de la composition renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la Demanderesse.

Certains composés de formule (I), utilisés à titre de base d'oxydation, sont nouveaux et, à ce titre, constituent un autre objet de l'invention.

Ces nouveaux composés de formule (I), ainsi que leurs sels d'addition avec un acide, répondent aux formules (l'a) à (I'f) suivantes : 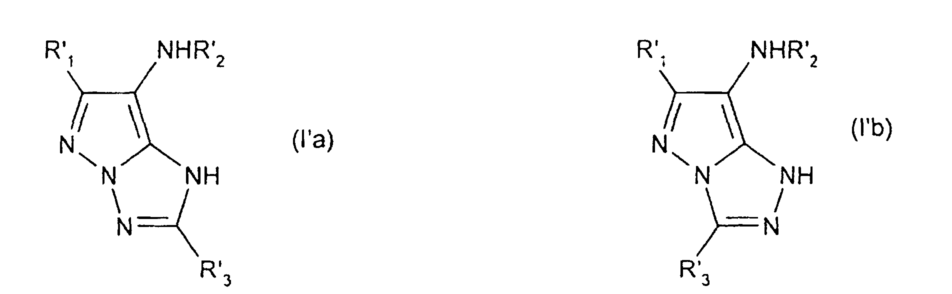 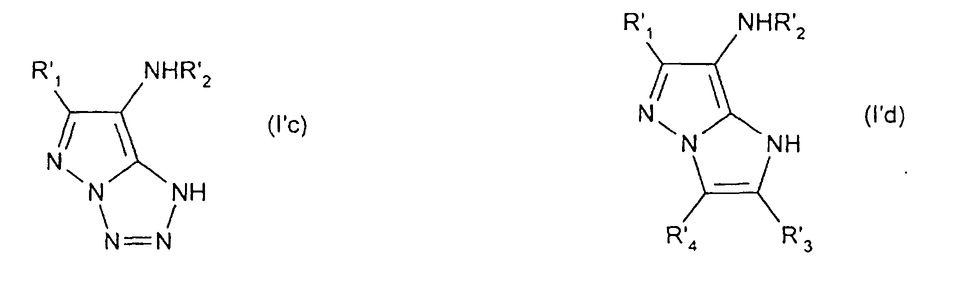 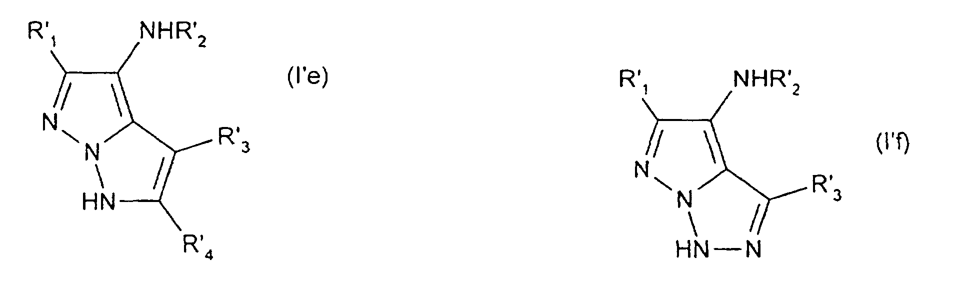 dans lesquelles les radicaux R'₁, R'₂, R'₃ et R'₄ ont les mêmes significations que celles indiquées précédemment pour les radicaux R₁, R₂, R₃ et R₄ de la formule (I) ; étant entendu que :
- dans les composés de formule (I'b) :
   i) lorsque R'₁ représente un radical méthyle, et que R'₂ représente un atome d'hydrogène, alors le radical R'₃ est différent d'un radical méthyle ou d'un radical (CH₂)₃-SO₂-(CH₂)₁₅-CH₃, ou CH(CH₃)-CH₂-SO₂-(CH₂)₁₁-CH₃ ;
   ii) lorsque R'₁ représente un radical ter-butyle, et que R'₂ représente un atome d'hydrogène, alors R'₃ est différent d'un radical (CH₂)₃-SO₂-(CH₂)₁₁-CH₃ ;
- dans les composés de formule (I'd) :
   - lorsque R'₃ et R'₄ forment ensemble un noyau benzénique, et que R'₂ représente un atome d'hydrogène, alors R'₁ est différent d'un radical méthyle, -CONH₂ ou carboxyle ;
   - lorsque R'₃ et R'₄ forment ensemble un noyau benzénique substitué par un radical sulfonyle en position 4, et que R'₂ représente un atome d'hydrogène, alors R'₁ est différent d'un radical phényle, d'un radical -(CH₂)₁₆-CH₃ ; ou d'un radical -COOH ;
   - lorsque R'₃ et R'₄ forment ensemble un noyau benzénique substitué par un radical carboxylique en position 4, et que R'₂ représente un atome d'hydrogène, alors R'₁ est différent d'un radical carboxylique.

Les différents composés exclus par les réserves ci-dessus sont connus dans le domaine photographique ou comme réactifs analytiques (voir notamment JP 02188748, JP 63074055, JP 61160745, et JP 60172982).

Parmi les nouveaux composés de formule (l'a) ci-dessus, on peut plus particulièrement citer :
- le 7-amino-2-méthyl pyrazolo [1,5-b]-1,2,4-triazole ;
- le 7-amino-2-éthyl pyrazolo [1,5-b]-1,2,4-triazole ;
- le 7-amino-2-isopropyl pyrazolo [1,5-b]-1,2,4-triazole ;
- le 7-amino-2-phényl pyrazolo [1,5-b]-1,2,4-triazole ;
- le 7-amino-2,6-diméthyl pyrazolo [1,5-b]- 1,2,4-triazole ;
- le 7-amino-6-méthyl-2-éthyl pyrazolo [1,5-b]-1,2,4-triazole ;
- le 7-amino-6-méthyl-2-isopropyl pyrazolo [1,5-b]-1,2,4-triazole ;
- le 7-amino- 6-méthyl-2-phényl pyrazolo [1,5-b]-1,2,4-triazole ;
- le 7-amino-6-carboxy-2-méthyl pyrazolo [1,5-b]-1,2,4-triazole ;
- le 7-amino-6-carboxy-2-éthyl pyrazolo [1,5-b]-1,2,4-triazole ;
- le7-amino- 6-carboxy-2-isopropyl pyrazolo [1,5-b]-1,2,4-triazole ;
- le 7-amino-6-carboxy-2-phényl pyrazolo [1,5-b]-1,2,4-triazole ;
- le 7-amino-6-phényl-2-méthyl pyrazolo [1,5-b]-1,2,4-triazole ;
- le 7-amino-6-phényl-2-éthyl pyrazolo [1,5-b]-1,2,4-triazole ;
- le 7-amino-6-phényl-2-isopropyl pyrazolo [1,5-b]-1,2,4-triazole ;
- le 6,7-diamino-2-méthyl pyrazolo [1,5-b]-1,2,4-triazole ;
- le 6,7-diamino-2-éthyl pyrazolo [1,5-b]-1,2,4-triazole ;
- le 6,7-diamino-2-isopropyl pyrazolo [1,5-b]-1,2,4-triazole ;
- le 6,7-diamino-2-phényl pyrazolo [1,5-b]-1,2,4-triazole ;
- le 7-amino-6-éthylthio-2-méthyl pyrazolo [1,5-b]-1,2,4-triazole ;
- le 7-amino-6-éthylthio-2-éthyl pyrazolo [1,5-b]-1,2,4-triazole ;
- le 7-amino-6-éthylthio-2-isopropyl pyrazolo [1,5-b]-1,2,4-triazole ;
- le 7-amino-6-éthylthio-2-phényl pyrazolo [1,5-b]-1,2,4-triazole ;
- le 7-amino-6-éthoxy-2-méthyl pyrazolo [1,5-b]-1,2,4-triazole ;
- le 7-amino-6-éthoxy-2-éthyl pyrazolo [1,5-b]-1,2,4-triazole ;
- le 7-amino-6-éthoxy-2-isopropyl pyrazolo [1,5-b]-1,2,4-triazole ;
- le 7-amino-6-éthoxy-2-phényl pyrazolo [1,5-b]-1,2,4-triazole ;
- le 7-amino-6-méthyl-2-(2'-aminoéthyl) pyrazolo [1,5-b]-1,2,4-triazole ;
- le 7-amino-6-carboxy-2-(2'-aminoéthyl) pyrazolo [1,5-b]-1,2,4-triazole ;
- le 7-amino-6-phényl-2-(2'-aminoéthyl) pyrazolo [1,5-b]-1,2,4-triazole ;
- le 7-amino-6-éthylthio-2-(2'-aminoéthyl) pyrazolo [1,5-b]-1,2,4-triazole ;
- le 7-amino-2-(2'-aminoéthyl) pyrazolo [1,5-b]-1,2,4-triazole ;
- le 7-amino-2-(2'-hydroxyéthyl) pyrazolo [1,5-b]-1,2,4-triazole ;
- le 7-amino-6-méthyl-2-(2'-hydroxyéthyl) pyrazolo [1,5-b]-1,2,4-triazole ;
- le 7-amino-6-éthylthio-2-(2'-hydroxyéthyl) pyrazolo [1,5-b]-1,2,4-triazole ;
- le 7-amino-6-carboxy-2-(2'-hydroxyéthyl) pyrazolo [1,5-b]-1,2,4-triazole ;
- le 7-amino-6-phényl-2-(2'-hydroxyéthyl) pyrazolo [1,5-b]-1,2,4-triazole ;
- le 6,7-diamino-2-(2'-aminoéthyl) pyrazolo [1,5-b]-1,2,4-triazole ;
- le 6,7-diamino-2-(2'-hydroxyéthyl) pyrazolo [1,5-b]-1,2,4-triazole ;
- le 7-amino-6-éthoxy-2-(2'-aminoéthyl) pyrazolo [1,5-b]-1,2,4-triazole ;
- le 7-amino-6-éthoxy-2-(2'-hydroxyéthyl) pyrazolo [1,5-b]-1,2,4-triazole ;
et leurs sels d'addition avec un acide.

Parmi les nouveaux composés de formule (I'b), on peut plus particulièrement citer
- le 7-amino-3-méthyl pyrazolo [3,2-c]-1,2,4-triazole ;
- le 7-amino-3-éthyl pyrazolo [3,2-c]-1,2,4-triazole ;
- le 7-amino-3-isopropyl pyrazolo [3,2-c]-1,2,4-triazole ;
- le 7-amino-3-phényl pyrazolo [3,2-c]-1,2,4-triazole ;
- le 7-amino-3-(2'-aminoéthyl) pyrazolo [3,2-c]-1,2,4-triazole ;
- le 7-amino-3-(2'-hydroxyéthyl) pyrazolo [3,2-c]-1,2,4-triazole ;
- le 7-amino-6-méthyl-3-éthyl pyrazolo [3,2-c]-1,2,4-triazole ;
- le 7-amino-3,6-diméthyl pyrazolo [3,2-c]-1,2,4-triazole ;
- le 7-amino-6-méthyl-3-isopropyl pyrazolo [3,2-c]-1,2,4-triazole ;
- le 7-amino-6-méthyl-3-phényl pyrazolo [3,2-c]-1,2,4-triazole ;
- le 7-amino-6-méthyl-3-(2'-aminoéthyl) pyrazolo [3,2-c]-1,2,4-triazole ;
- le 7-amino-6-méthyl-3-(2'-hydroxyéthyl) pyrazolo [3,2-c]-1,2,4-triazole ;
- le 7-amino-6-méthyl-3-méthylthio pyrazolo [3,2-c]-1,2,4-triazole ;
- le 7-amino-6-phényl-3-méthyl pyrazolo [3,2-c]-1,2,4-triazole ;
- le 7-amino-6-phényl-3-éthyl pyrazolo [3,2-c]-1,2,4-triazole ;
- le 7-amino-6-phényl-3-isopropyl pyrazolo [3,2-c]-1,2,4-triazole ;
- le 7-amino-6-phényl-3-phényl pyrazolo [3,2-c]-1,2,4-triazole ;
- le 7-amino-6-phényl-3-(2'-aminoéthyl) pyrazolo [3,2-c]-1,2,4-triazole ;
- le 7-amino-6-phényl-3-(2'-hydroxyéthyl) pyrazolo [3,2-c]-1,2,4-triazole ;
- le 7-amino-6-phényl-3-méthylthio pyrazolo [3,2-c]-1,2,4-triazole ;
- le 7-amino-6-éthylthio-3-méthyl pyrazolo [3,2-c]-1,2,4-triazole ;
- le 7-amino-6-éthylthio-3-éthyl pyrazolo [3,2-c]-1,2,4-triazole ;
- le 7-amino-6-éthylthio-3-isopropyl pyrazolo [3,2-c]-1,2,4-triazole ;
- le 7-amino-6-éthylthio-3-phényl pyrazolo [3,2-c]-1,2,4-triazole ;
- le 7-amino-6-éthylthio-3-(2'-aminoéthyl) pyrazolo [3,2-c]-1,2,4-triazole ;
- le 7-amino-6-éthylthio-3-(2'-hydroxyéthyl) pyrazolo [3,2-c]-1,2,4-triazole ;
- le 7-amino-6-trifluorométhyl-3-méthylthio pyrazolo [3,2-c]-1,2,4-triazole ;
- le 7-amino-6-trifluorométhyl pyrazolo [3,2-c]-1,2,4-triazole ;
- le 7-amino-6-carboxy-3-méthyl pyrazolo [3,2-c]-1,2,4-triazole ;
- le 7-amino-6-carboxy-3-éthyl pyrazolo [3,2-c]-1,2,4-triazole ;
- le 7-amino-6-carboxy-3-isopropyl pyrazolo [3,2-c]-1,2,4-triazole ;
- le 7-amino-6-carboxy-3-phényl pyrazolo [3,2-c]-1,2,4-triazole ;
- le 7-amino-6-carboxy-3-(2'-aminoéthyl) pyrazolo [3,2-c]-1,2,4-triazole ;
- le 7-amino-6-carboxy-3-(2'-hydroxyéthyl) pyrazolo [3,2-c]-1,2,4-triazole ;
- le 6,7-diamino-3-méthyl pyrazolo [3,2-c]-1,2,4-triazole ;
- le 6,7-diamino-3-éthyl pyrazolo [3,2-c]-1,2,4-triazole ;
- le 6,7-diamino-3-phényl pyrazolo [3,2-c]-1,2,4-triazole ;
- le 6,7-diamino-3-isopropyl pyrazolo [3,2-c]-1,2,4-triazole ;
- le 7-amino-6-éthoxy-3-méthyl pyrazolo [3,2-c]-1,2,4-triazole ;
- le 7-amino-6-éthoxy-3-éthyl pyrazolo [3,2-c]-1,2,4-triazole ;
- le 7-amino-6-éthoxy-3-isopropyl pyrazolo [3,2-c]-1,2,4-triazole ;
- le 7-amino-6-éthoxy-3-phényl pyrazolo [3,2-c]-1,2,4-triazole ;
- le 7-amino-6-éthoxy-3-(2'-aminoéthyl) pyrazolo [3,2-c]-1,2,4-triazole ;
- le 7-amino-6-éthoxy-3-(2'-hydroxyéthyl) pyrazolo [3,2-c]-1,2,4-triazole ;
- le 6,7-diamino-3-(2'-aminoéthyl) pyrazolo [3,2-c]-1,2,4-triazole ;
- le 6,7-diamino-3-(2'-hydroxyéthyl) pyrazolo [3,2-c]-1,2,4-triazole ;
et leurs sels d'addition avec un acide.

Parmi les nouveaux composés de formule (I'c), on peut plus particulièrement citer :
- le 7-amino-6-méthyl pyrazolo [5,1-e] tétrazole ;
- le 7-amino-6-phényl pyrazolo [5,1-e] tétrazole ;
- le 7-amino-6-carboxy pyrazolo [5,1-e] tétrazole ;
et leurs sels d'addition avec un acide.

Parmi les nouveaux composés de formule (I'd), on peut plus particulièrement citer :
- le 7-amino pyrazolo [1,5-a] imidazole ;
- le 7-amino-2-méthyl pyrazolo [1,5-a] imidazole ;
- le 7-amino-2-phényl pyrazolo [1,5-a] imidazole ;
- le 7-amino pyrazolo [1,5-a] benzimidazole ;
- le 7-amino-6-méthyl pyrazolo [1,5-a] imidazole ;
- le 7-amino-2,6-diméthyl pyrazolo [1,5-a] imidazole ;
- le 7-amino-6-méthyl-2-phényl pyrazolo [1,5-a] imidazole ;
- le 7-amino-6-phényl pyrazolo [1,5-a] imidazole ;
- le 7-amino-6-phényl-2-méthyl pyrazolo [1,5-a] imidazole ;
- le 7-amino-2,6-diphényl pyrazolo [1,5-a] imidazole ;
- le 7-amino-6-phényl pyrazolo [1,5-a] benzimidazole ;
- le 7-amino-6-carboxy pyrazolo [1,5-a] imidazole ;
- le 7-amino-6-carboxy-2-méthyl pyrazolo [1,5-a] imidazole ;
- le 7-amino-6-carboxy-2-phényl pyrazolo [1,5-a] imidazole ;
- le 7-amino-6-éthoxy pyrazolo [1,5-a] imidazole ;
- le 7-amino-6-éthoxy-2-méthyl pyrazolo [1,5-a] imidazole ;
- le 7-amino-6-éthoxy-2-phényl pyrazolo [1,5-a] imidazole ;
- le 7-amino-6-éthoxy pyrazolo [1,5-a] benzimidazole ;
- le 7-amino-6-trifluorométhyl pyrazolo [1,5-a] benzimidazole ;
- le 6,7-diamino pyrazolo [1,5-a] imidazole ;
- le 6,7-diamino-2-méthyl pyrazolo [1,5-a] imidazole ;
- le 6,7-diamino-2-phényl pyrazolo [1,5-a] imidazole ;
- le 6,7-diamino pyrazolo [1,5-a] benzimidazole ;
- le 7-amino-6-éthylthio pyrazolo [1,5-a] imidazole ;
- le 7-amino-6-éthylthio-2-méthyl pyrazolo [1,5-a] imidazole ;
- le 7-amino-6-éthylthio-2-phényl pyrazolo [1,5-a] imidazole ;
- le 7-amino-6-méthyl-2-hydroxy pyrazolo [1,5-a] imidazole ;
- le 7-amino-6-phényl-2-hydroxy pyrazolo [1,5-a] imidazole ;
- le 7-amino-6-éthoxy-2-hydroxy pyrazolo [1,5-a] imidazole ;
- le 7-amino-6-éthylthio-2-hydroxy pyrazolo [1,5-a] imidazole ;
- le 6,7-diamino-2-hydroxy pyrazolo [1,5-a] imidazole ;
et leurs sels d'addition avec un acide.

Parmi les nouveaux composés de formule (l'e), on peut plus particulièrement citer :
- le 5,8-diamino-4-méthyl pyrazolo [5,1-e] pyrazole ;
- le 5,7,8-triamino-4-méthyl pyrazolo [5,1-e] pyrazole ;
et leurs sels d'addition avec un acide.

Parmi les nouveaux composés de formule (I'f), on peut plus particulièrement citer :
- le 6-amino-5-méthyl pyrazolo [5,1-e]-1,2,3-triazole ;
- le 6-amino-5-phényl pyrazolo [5,1-e]-1,2,3-triazole ;
et leurs sels d'addition avec un acide.

Les nouveaux composés de formule (I') conformes à la présente invention, peuvent être préparés selon les différents procédés de préparation décrits dans les demandes de brevets et brevets suivants : FR 2 075 583, EP-A-119 860, EP-A-285 274, EP-A-244 160, EP-A-578 248, GB 1 458 377, US 3 227 554, US 3 419 391, US 3 061 432, US 4 500 630, US 3 725 067, US 3 926 631, US 5 457 210, JP 84/99437, JP 83/42045, JP 84/162548, JP 84/171956, JP 85/33552, JP 85/43659, JP 85/172982, JP 85/190779, JP 41/33053, EP 433854, JP 62/129851, JP 60/140241, ainsi que dans les publications suivantes :
- Chem. Ber. 32, 797 (1899), Chem. Ber. 89, 2550, (1956),
- J. Chem. Soc. Perkin trans 1, 2047, (1977),
- J. Prakt. Chem., 320, 533, (1978).

Les exemples qui suivent sont destinés à illustrer l'invention, sans pour autant lui apporter un caractère limitatif.

### EXEMPLES DE PREPARATION

### EXEMPLE DE PREPARATION A : Synthèse du chlorhydrate du 1H-7-amino-2,6-diméthyl pyrazolo [1,5-b]-1,2,4-triazole

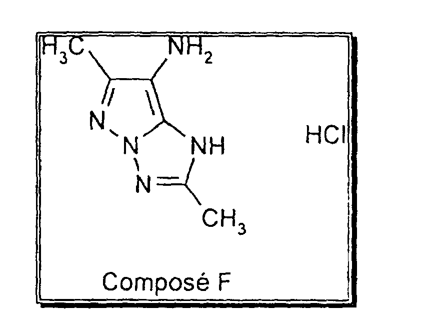

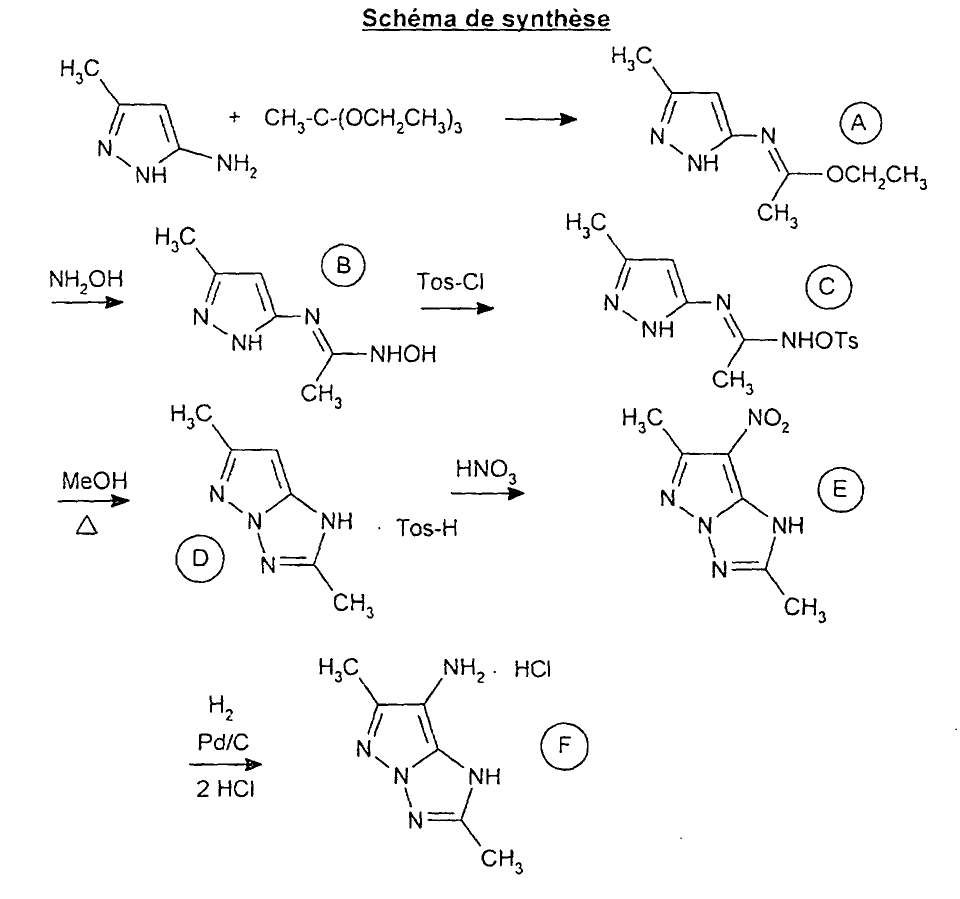

A une solution de 100 g de 5-amino-3-méthyl pyrazole (1,03 moles) dans 500 cm³ de diglyme, on a ajouté 200 g (1,23 moles) d'orthoacétate d'éthyle en 20 minutes à température ambiante. Le milieu réactionnel a été chauffé à 95°C, puis concentré à 55°C sous 300 Pa pour obtenir 164 g d'une huile orange correspondant au produit **A**.

A une solution de 71,7 g (1,03 mole) de chlorhydrate d'hydroxylamine dans 1,5 l d'éthanol absolu ont été ajoutés 180 cm³ d'une solution de méthylate de sodium (5,7 M dans le méthanol) à température ambiante. Après 30 minutes d'agitation, le milieu réactionnel a été refroidi à 5°C et filtré. Les sels ont été lavés par 2 fois avec 100 cm³ d'éthanol absolu et les deux solutions éthanoliques ont été rassemblées. A cette solution ont été ajoutés 164 g (0,98 mole) de composé **A**, préparé comme ci-dessus, dissous dans 150 cm³ d'éthanol à une température de 5°C.

On a laissé le milieu réactionnel se réchauffer jusqu'à la température ambiante et agité pendant 1h supplémentaire. Le milieu réactionnel a alors été refroidi à 5°C et le précipité obtenu a été essoré, lavé par 2 fois avec 100 cm³ d'éther isopropylique et séché à 50° C sous 5300 Pa. On a ainsi obtenu 52 g d'un premier jet de solide blanc correspondant au composé **B** dont le point de fusion était de 197° C.

Le filtrat éthanolique et la phase éthérée de lavage ont été réunis puis concentrés à sec avant d'être réempatés dans 300 cm³ de tétrahydrofurane. On a essoré ce deuxième précipité, lavé à l'éther isopropylique et séché à 50°C sous vide pour obtenir 16 g d'un deuxième jet de précipité blanc de point de fusion 197° C correspondant au composé **B**.

A une solution de 20 g (0,13 mole) de composé **B**, préparé ci-dessus, dans 9,5 litres de tétrahydrofurane anhydre, on a ajouté 20,05 cm³ (0,143 mole) de triéthylamine anhydre puis 27,2 g (0,143 mole) du chlorure d'acide de l'acide paratoluènesulfonique. Le milieu réactionnel a été agité pendant 2 heures à température ambiante puis refroidi à 0°C. Le chlorhydrate de triéthylamine a été séparé par filtration et le filtrat a été concentré sous un vide de 8 kPa à environ 50°C jusqu'au début de la cristallisation. On a refroidi à 0°C et le précipité a été essoré sur un verre fritté, lavé avec du tétrahydrofurane glacé, puis séché sous vide à une température de 40°C. On a ainsi obtenu 38,2 g de composé **C** sous la forme d'un solide blanc dont le point de fusion était compris entre 105°C et 128°C (décomposition).

On a chauffé pendant 2 heures au reflux une solution de 35 g (0,113 mole) de composé **C** obtenu ci-dessus dans 1 litre de méthanol puis on a évaporé à sec cette solution. On a obtenu une huile qui a cristallisé quand on a ajouté 100 cm³ d'éther isopropylique. Les cristaux ont été essorés sur un verre fritté puis recristallisés dans un mélange d'isopropanol et d'heptane. On a ainsi obtenu, après séchage sous vide à 40°C, 19 g de tosylate du 1H-2,6-diméthyl pyrazolo [1,5-b]-1,2,4-triazole (composé **D**) sous la forme d'un solide blanc dont le point de fusion était de 157°C.

A une solution de 19 g (0,062 mole) de composé **D** dans 35 cm³ d'acide sulfurique concentré, à une température d'environ 0°C - 5°C, ont été ajoutés 8,9 cm³ d'acide nitrique fumant en veillant à ne pas dépasser une température de 5°C. Le milieu réactionnel a ensuite été chauffé à température ambiante, agité pendant 4 heures, puis versé lentement sur 400 g de glace pilée. Le solide blanc, qui a précipité, a été essoré, lavé par 2 fois avec 15 cm³ d'eau glacée et séché à 40° C sous vide. On a ainsi obtenu 8,5 g de solide blanc correspondant au composé **E** et de point de fusion 273° C.

A une solution de 8 g (0,044 mole) de composé **E** dans 300 cm³ de méthanol ont été ajoutés 1,4 g de Palladium sur charbon à 5% en poids et contenant 50 % d'eau. La suspension a été placée dans un hydrogénateur, sous une pression de 13 bars d'hydrogène et à une température de 70° C pendant 5 heures. Le contenu de l'hydrogénateur a été prélevé et filtré sur verre fritté. Le filtrat a ensuite été coulé dans 100 cm³ d'une solution d'éthanol chlorhydrique 4M. On a ajouté 350 cm³ d'éther éthylique à cette solution et le précipité qui s'est formé a été essoré, lavé 2 fois par 20 cm³ d'éther éthylique, et séché à 40° C sous vide pour obtenir 7,2 g de solide beige correspondant au composé **F** et de point de fusion 213°C.

L'analyse élémentaire pour C₆H₉N₅, HCl était :

| **%** | **C** | **H** | **N** | **Cl** |
|---|---|---|---|---|
| Calculé | 38,41 | 5,37 | 37,32 | 18,89 |
| Trouvé | 38,22 | 5,14 | 36,57 | 19,03 |

### EXEMPLE DE PREPARATION B : Synthèse du dichlorhydrate du 1H-7-amino-3,6-diméthyl pyrazolo [3,2-c]-1,2,4-triazole

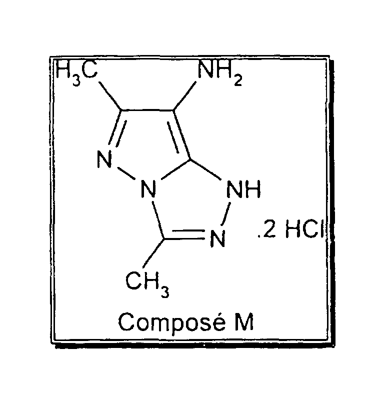

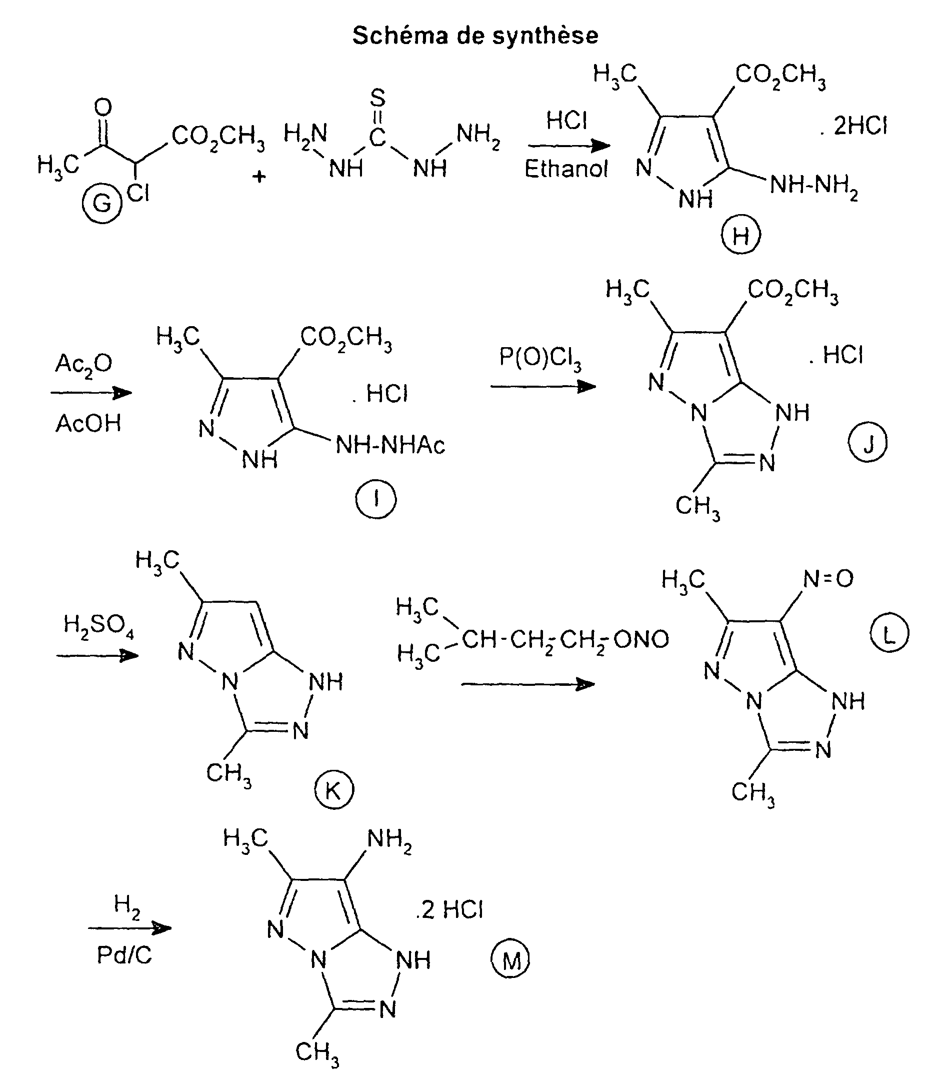

A une solution de 80 g de thiocarbazide (0,754 mole) dans 2 litres d'éthanol et 700 cm³ d'acide chlorhydrique 12 N, ont été ajoutés 113,5 g (0,754 mole) de 2-chloroacétoacétate de méthyle (composé **G**), à une température de 80°C. Le milieu réactionnel a été chauffé au reflux pendant 1 heure, puis refroidi à 0°C. Le solide formé a été essoré, lavé 2 fois avec 200 cm³ d'éther isopropylique, puis séché à 40°C sous vide. Le solide a alors été dissous dans 800 cm³ de méthanol au reflux et la solution a été glacée à 0°C. Le solide ainsi formé a été essoré, lavé deux fois avec 100 cm³ d'éther isopropylique et séché à 40°C sous vide. On a ainsi obtenu 12,65 g de poudre beige correspondant au composé **H** et de point de fusion 199°C.

Le filtrat de recristallisation a été concentré de moitié et 700 cm³ d'éther isopropylique ont été rajoutés à ce concentrat. Le solide ainsi formé a été essoré, lavé 2 fois avec 100 cm³ d'éther isopropylique et séché à 40°C sous vide. On a ainsi obtenu 9,14 g de solide beige correspondant au composé **H** et de point de fusion 200°C. La même opération a été recommencée sur ce dernier filtrat et 6,9 g d'un solide beige a été obtenu, dont le point de fusion était de 199°C et correspondant au composé **H**.

A une suspension de 25 g (0,103 mole) de composé **H** dans 750 cm³ d'acide acétique ont été ajoutés 9,7 cm³ d'anhydride acétique. Le milieu réactionnel a été chauffé au reflux pendant 1 heure, puis refroidi à 16°C. Le précipité blanc a alors été essoré, lavé par 250 cm³ d'éther diisopropylique et séché à 40°C sous vide. On a ainsi obtenu 19 g de solide blanc correspondant au composé **I** et de point de fusion 216°C. Le filtrat a été concentré jusqu'à un volume d'environ 30 cm³, puis refroidi à 16°C. A cette solution, ont été ajoutés 15 cm³ d'acétone et 45 cm³ d'éther isopropylique. Le précipité a alors été essoré, lavé deux fois avec 50 cm³ d'ether isopropylique et séché à 40°C sous vide. On a ainsi obtenu 4,7 g d'un deuxième jet de précipité blanc correspondant au composé **I** et de point de fusion 216°C.

A une suspension de 23 g de composé **I** (0,092 mole) dans 1,2 litre de toluène ont été ajoutés 22 cm³ de chlorure de phosphoryle à température ambiante et sous vive agitation. Le milieu réactionnel a été chauffé au reflux pendant 5 heures, puis refroidi à 0°C. Le précipité obtenu a été essoré, lavé 2 fois avec 200 cm³ d'heptane et séché sous une pression de 40 mm de mercure à 40° C. On a ainsi obtenu 11 g de précipité jaune qui ont été recristallisés dans 30 cm³ d'éthanol absolu pour conduire à 5 g de poudre jaune correspondant au composé **J** et de point de fusion 192°C.

A une solution de 3,8 g (0,016 mole) de composé **J** dans 20 cm³ d'eau ont été ajoutés 1,7 cm³ de soude 10 N à une température de 5° C. A cette solution ont été ajoutés 60 cm³ d'acide sulfurique concentré à 98% en veillant à ce que la température reste comprise entre 7 et 10°C. Le milieu réactionnel a ensuite été chauffé à 100°C-120° C pendant 1 h 30. Après avoir été refroidi à 0°C, le milieu réactionnel a été versé dans 200 g de glace et son pH a été ajusté à pH 8 à l'aide de soude 10 N en veillant à ce que la température n'ait pas dépassé pas 10°C.

La phase aqueuse a ensuite été extraite 3 fois par 300 cm³ d'acétate d'éthyle. La phase organique a été séchée sur sulfate de sodium et concentrée jusqu'à un volume d'environ 30 cm³. Le précipité jaune obtenu a été essoré, lavé 2 fois avec 50 cm³ d'heptane, séché à 40°C sous vide. On a ainsi obtenu 1,2 g de poudre jaune correspondant au composé **K** et de point de fusion 118° C.

A une solution de 1 g de composé **K** (7,3 mmoles), dans 20 cm³ d'isopropanol et 20 cm³ d'éthanol absolu a été ajouté de l'acide chlorhydrique 4N jusqu'à ce que le pH de la solution ait été de 5. Le milieu réactionnel a été refroidi à 5°C et 1 cm³ de nitrite d'isopentyle y a été ajouté. La température du milieu réactionnel a été ramenée à 25°C. Après 1 heure d'agitation, le milieu réactionnel a été refroidi à 0°C et le précipité a été essoré, lavé par de l'éther diisopropylique et séché à 30°C sous vide. On a obtenu ainsi 1,2 g de solide beige correspondant au composé **L** et de point de fusion 210°C.

A une solution de 1 g (6 mmoles) de composé **L** dans 200 cm³ de méthanol a été ajouté 0,4 g de Palladium sur charbon à 5 % en poids et contenant 50 % d'eau. La suspension a été placée dans un hydrogénateur, sous une pression de 3,5 bars d'hydrogène à 30°C pendant 1 heure. Le contenu de l'hydrogénateur a été ensuite prélevé et filtré. Le filtrat a été coulé dans une solution contenant 200 cm³ d'éther diisopropylique et 10 cm³ de solution d'éthanol chlorhydrique 5M. Le précipité formé a été essoré, lavé à l'éther diisopropylique et séché à 40°C sous vide. On a ainsi obtenu 0,9 g de poudre beige clair correspondant au composé **M** et de point de fusion 206°C et nommée avec les conventions de numérotation définies précédemment le dichlorhydrate de 7-amino-3,6-diméthyl pyrazolo [3,2-c]-1,2,4-triazole.

L'analyse élémentaire pour C₆H₉N₅, 2HCl, H₂O était :

| % | **C** | **H** | **N** | **Cl** |
|---|---|---|---|---|
| **Calculé** | 29,77 | 5,41 | 28,93 | 29,29 |
| **Trouvé** | 29,07 | 5,19 | 28,04 | 29,48 |

### EXEMPLE DE PREPARATION C : Synthèse du dichlorhydrate du 1H-7-amino-6-méthylpyrazolo[1,5-a]imidazole

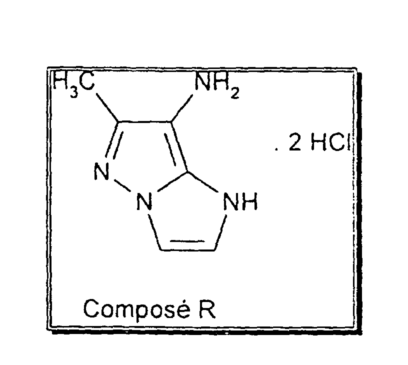

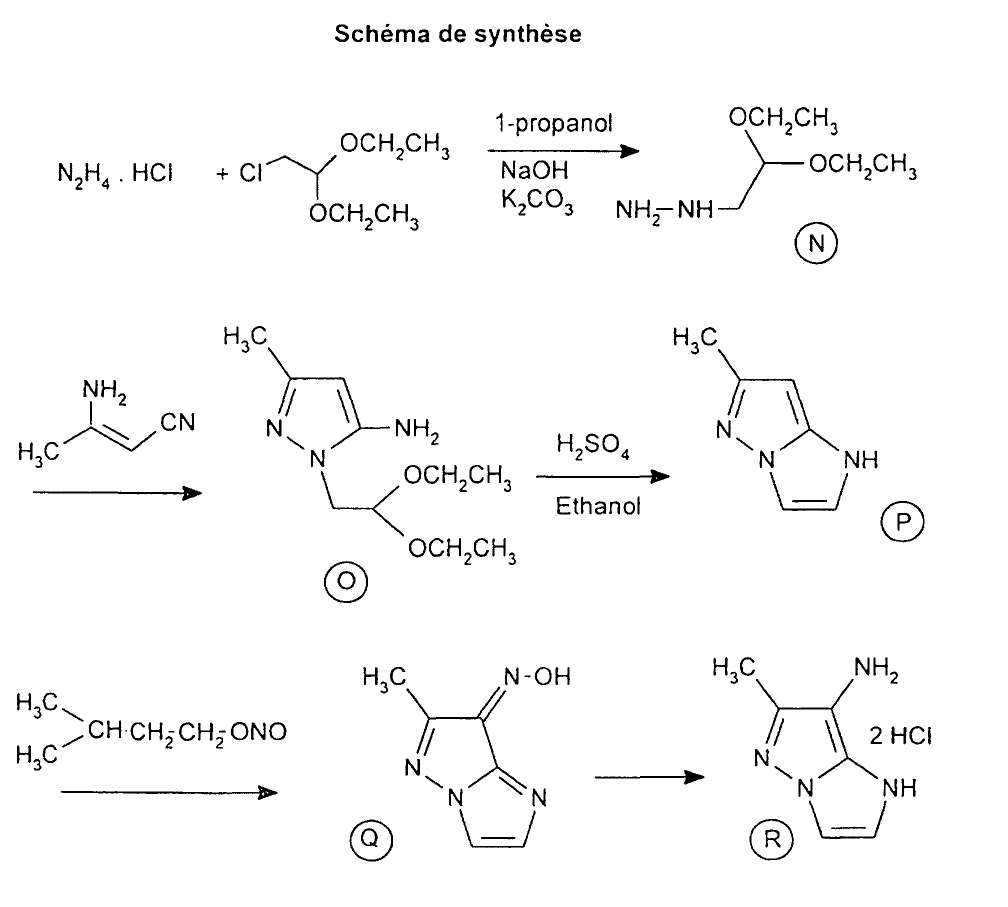

A une solution de 410 g de soude dans 6 litres de n-propanol à 25°C, ont été ajoutés 685 g de chlorhydrate d'hydrazine (10 moles). Après 1 heure d'agitation ont été introduits, à température ambiante, 452 g de carbonate de potassium (3,27 moles), puis 1 kg de diéthylacétal de chloroacétaldéhyde. Le milieu réactionnel a été chauffé à ébullition pendant 40 heures, puis refroidi à 5°C. Les sels précipités ont été filtrés et lavés 2 fois avec 200 cm³ d'éthanol absolu. Le filtrat et la phase éthanolique rassemblés ont été concentrés jusqu'à obtention d'une huile jaune qui a été distillée sous une pression de 1,3 Pa à une température de 78-82°C. On a ainsi isolé 446 g d'huile incolore correspondant au composé **N**.

A une solution de 8,2 g (0,1 mole) de 3-aminocrotononitrile dans 100 cm³ de n-pentanol ont été ajoutés 14,8 g (0,1 mole) de composé **N** préalablement dilués dans 20 cm³ de n-pentanol à 25°C. Le milieu réactionnel a été chauffé au reflux pendant 5 heures, puis concentré de façon à distiller le n-pentanol sous une pression de 5,3 kPa. L'huile marron, qui a été obtenue, a ensuite été distillée sous un vide de 3 Pa à une température de 117-119°C pour isoler une huile jaune pâle. On a ainsi obtenu 17,8 g d'huile correspondant au composé **O**.

A une solution de 17 g (0,08 mole) de composé **O** dans 300 cm³ d'éthanol absolu ont été additionnés 200 cm³ d'acide sulfurique à 20% aqueux à température ambiante. Le milieu réactionnel a ensuite été chauffé au reflux pendant 3 heures, puis refroidi à 5°C. Le pH du milieu réactionnel a été ajusté à pH 8 à l'aide d'une solution saturée d'hydrogènocarbonate de sodium dans l'eau. Le sel de sulfate de sodium a été filtré du milieu réactionnel et le filtrat a été concentré de façon à éliminer l'éthanol. La phase aqueuse ainsi obtenue a été extraite 3 fois par 300 cm³ d'acétate d'éthyle, puis par 300 cm³ d'un mélange acétate d'éthyle / méthanol dans les proportions 90/10. Les phases organiques réunies ont été séchées sur sulfate de magnésium, puis concentrées à sec. Le solide orange obtenu a été ensuite réempaté dans 200 cm³ d'heptane, essoré et lavé par 50 cm³ d'heptane, puis séché à 40°C sous vide. On a ainsi obtenu 9,8 g de solide orange correspondant au composé **P** et de point de fusion 138° C.

A une solution de 2 g (0,016 mole) de composé **P** dans 10 cm³ d'éthanol absolu à 5°C a été ajouté de l'acide chlorhydrique 12 N jusqu'à ce que le milieu réactionnel ait atteint un pH de 5-6. Puis, 2,2 cm³ (0,016 mole) de nitrite d'isoamyle ont été ajoutés à cette solution à 5°C et la température du milieu réactionnel a progressivement augmenté jusqu'à environ 20°C. Après 1 heure d'agitation, la suspension a été refroidie à 5°C, le précipité a été essoré, lavé 2 fois avec 20 cm³ d'heptane et séché à 35°C sous vide. On a ainsi obtenu 1,4 g de solide marron-rouge correspondant au composé **Q** et de point de fusion 216° C.

A une solution de 1 g (6,66 mmoles) de composé **Q** dans 150 cm³ de méthanol ont été ajoutés 0,3 g de palladium sur charbon à 5% et contenant 50 % d'eau. La suspension a été placée dans un hydrogénateur, sous une pression de 1,5 bars à 30°C pendant 1 h 30. Le contenu de l'hydrogénateur a ensuite été prélevé et filtré. Le filtrat a été coulé dans une solution contenant 100 cm³ d'alcool isopropylique et 10 cm³ d'éthanol chlorhydrique 5M. La solution a été concentrée jusqu'à un volume correspondant au début de la cristallisation du produit réduit. La suspension a alors été glacée, le précipité a été essoré, lavé par de l'isopropanol glacé et séché à 40°C sous vide. On a ainsi obtenu 0,55 g de solide beige clair correspondant au composé **R** et de point de décomposition compris entre 230°-250° C et nommé avec les conventions de numérotation définies précédemment le dichlorhydrate de 7-amino-6-méthyl pyrazolo [1,5-a] imidazole.

L'analyse élémentaire pour C₆ H₈ N₄. 2HCl était :

| % | **C** | **H** | **N** | **Cl** |
|---|---|---|---|---|
| **Calculé** | 34,47 | 4,82 | 26,80 | 33,91 |
| **Trouvé** | 33,99 | 4,84 | 25,69 | 35,08 |

### EXEMPLES DE TEINTURE

### EXEMPLE 1 DE TEINTURE EN MILIEU ALCALIN

On a préparé la composition tinctoriale suivante :
- Dichlorhydrate de 7-amino-3,6-diméthyl pyrazolo [3,2-c]-1,2,4-triazole, monohydrate (composé de formule (I)) 0,672 g
- Résorcine 0,330 g
- Alcool benzylique 2 g
- Polyéthylène glycol à 6 moles d'oxyde d'éthylène 3 g
- Ethanol 18 g
- Alkyl (C₈-C₁₀) polyglucoside en solution aqueuse à 60% de matière active tamponné par du citrate d'ammonium vendu sous la dénomination ORAMIX CG110 ® par SEPPIC 6 g
- Ammoniaque à 20% de NH₃ 10 g
- Métabisulfite de sodium 0,208 g
- Séquestrant q.s.
- Eau déminéralisée q.s.p. 100 g

Au moment de l'emploi, la composition tinctoriale ci-dessus a été mélangée poids pour poids avec une composition oxydante constituée par une solution aqueuse contenant 6.10⁻³ mol% de persulfate d'ammonium.
Le mélange obtenu présentait un pH d'environ 9,7 et a été appliqué sur des mèches de cheveux gris naturels à 90% de blancs, pendant 30 minutes.
Les cheveux ont ensuite été rincés, lavés avec un shampooing standard et séchés.

Les cheveux ont été teints dans une nuance irisé cendré.

### EXEMPLES 2 ET 3 DE TEINTURE EN MILIEU ALCALIN

On a préparé les compositions tinctoriales suivantes (teneurs en grammes) :

| **EXEMPLE** | **2** | **3** |
|---|---|---|
| 7-amino-6-méthyl pyrazolo [1,5-a] benzimidazole, 2HCl (Composé de formule (I)) | 1,43 | - |
| 7-amino-3,6-diméthyl pyrazolo [3,2-c] 1,2,4-triazole, 2HCl, H₂O (Composé de formule (I)) | - | 1,12 |
| Support de teinture commun n°1 | (*) | (*) |
| Eau déminéralisée q.s.p. | 100 g | 100 g |

| | | |
|---|---|---|
| (*) Support de teinture commun n°1 : | | |

- Alcool benzylique 3 g
- Polyéthylène glycol à 6 moles d'oxyde d'éthylène 4,5 g
- Ethanol 15 g
- Alkyl (C₈-C₁₀) polyglucoside en solution aqueuse à 60% de matière active tamponné par du citrate d'ammonium vendu sous la dénomination ORAMIX CG110 ® par SEPPIC 9 g
- Tampon ammoniacal (NH₄OH 1 M / NH₄Cl 1M) 24 g

Ces compositions présentaient un pH d'environ 9,5 et ont été appliquées sur des mèches de cheveux gris naturels à 90 % de blancs pendant 30 minutes.
La coloration s'est développée sans autre agent oxydant que l'oxygène de l'air. Les cheveux ont ensuite été rincés, lavés avec un shampooing standard et séchés.

Les cheveux ont été teints dans une nuance figurant dans le tableau ci-après :

| **EXEMPLE** | **NUANCE OBTENUE** |
|---|---|
| 2 | Rouge irisé rabattu |
| 3 | Doré cuivré |

### EXEMPLE 4 DE TEINTURE EN MILIEU NEUTRE

On a préparé la composition tinctoriale suivante :
- 7-amino-3,6-diméthyl pyrazolo [3,2-c] 1,2,4-triazole, 2 HCl, H₂O (composé de formule (I)) 1,12 g
- Alcool benzylique 3 g
- Polyéthylène glycol à 6 moles d'oxyde d'éthylène 4,5 g
- Ethanol 15 g
- Alkyl (C₈-C₁₀) polyglucoside en solution aqueuse à 60% de matière active tamponné par du citrate d'ammonium vendu sous la dénomination ORAMIX CG110 ® par SEPPIC 9 g
- Tampon phosphate (K₂HPO₄ 1.5M / KH₂PO₄ 1 M) 24 g
- Eau déminéralisée q.s.p. 100 g

Cette composition présentait un pH d'environ 7 et a été appliquée sur des mèches de cheveux gris naturels à 90 % de blancs pendant 30 minutes.
La coloration s'est développé sans autre agent oxydant que l'oxygène de l'air. Les cheveux ont ensuite été rincés, lavés avec un shampooing standard et séchés.

Les cheveux ont été teints dans une nuance cuivré doré.

### EXEMPLES 5 ET 6 DE TEINTURE EN MILIEU ALCALIN

On a préparé les compositions tinctoriales suivantes (teneurs en grammes) :

| EXEMPLE | 5 | 6 |
|---|---|---|
| 7-amino 2,6-diméthyl pyrazolo [1,5-b] 1,2,4-triazole, HCl (composé de formule (I)) | 0,726 | 0,726 |
| 2-méthyl 5-aminophénol (coupleur) | 0,369 | - |
| Méta-aminophénol | - | 0,327 |
| Support de teinture commun n°2 | (**) | (**) |
| Eau déminéralisée q.s.p. | 100 g | 100 g |

| | | |
|---|---|---|
| (**) Support de teinture commun n° 2 : | | |

- Ethanol 30 g
- Acétate d'ammonium 0,8 g
- Métabisulfite de sodium en solution aqueuse à 35% 1,3 g
- Ammoniaque à 20% de NH₃ q.s. pH 10

Au moment de l'emploi la composition tinctoriale de l'exemple 5 a été mélangée poids pour poids avec une solution de peroxyde d'hydrogène à 20 volumes (6% en poids) et de pH 3. Le mélange obtenu présentait un pH d'environ 9,9.

Au moment de l'emploi la compositicn tinctoriale de l'exemple 6 a été mélangée poids pour poids avec une solution aqueuse contenant 6.10⁻³ mol% de persulfate d'ammonium. Le mélange obtenu présentait un pH d'environ 9,7.

Chacun des mélanges obtenu a été appliqué pendant 30 minutes sur des mèches de cheveux gris naturels à 90 % de blancs. Les cheveux ont ensuite rincés, lavés avec un shampooing standard, rincés à nouveau puis séchés.

Les cheveux ont été teints dans une nuance figurant dans le tableau ci-après :

| **EXEMPLE** | **NUANCE OBTENUE** |
|---|---|
| **5** | Saumon |
| **6** | Fuchsia |

### EXEMPLES 7 A 12 DE TEINTURE EN MILIEU ACIDE

On a préparé les compositions tinctoriales suivantes (teneurs en grammes) :

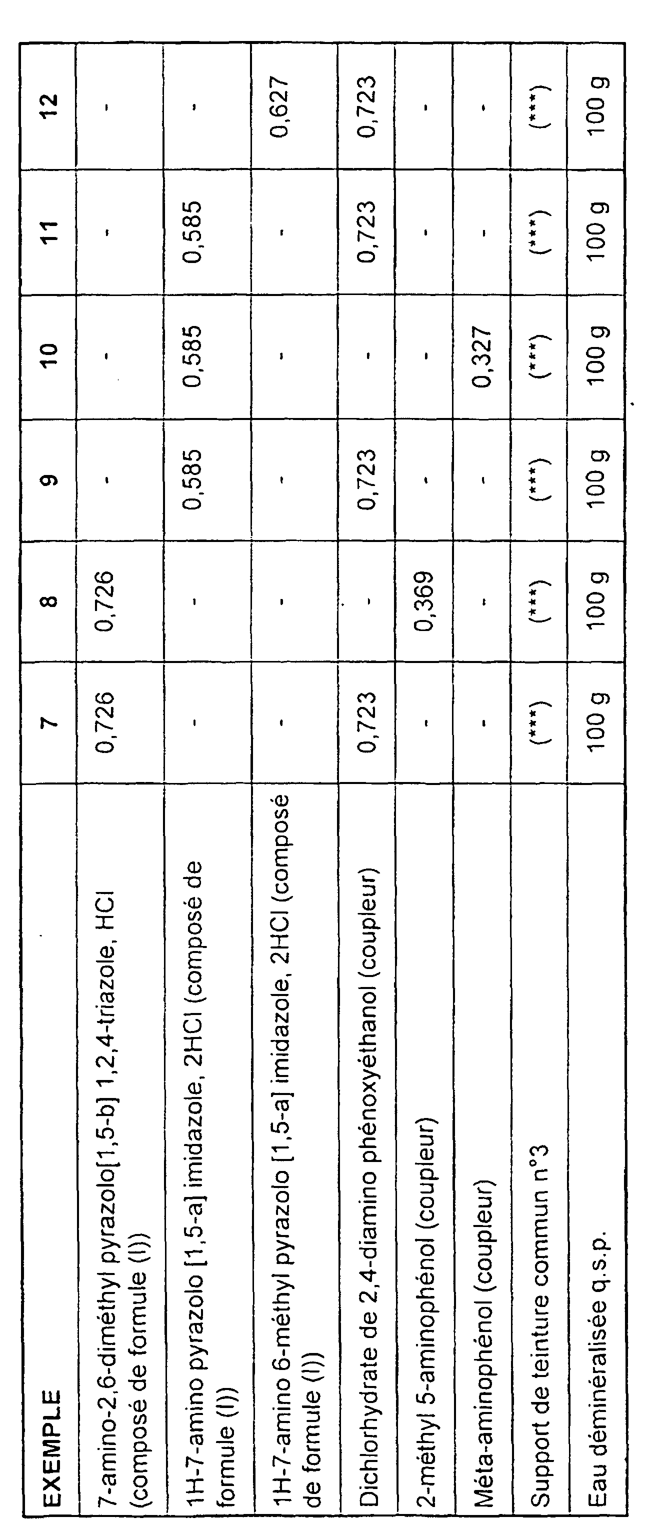

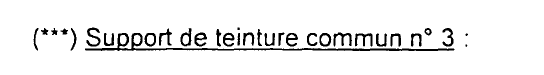

- Ethanol 20 g
- Tampon K₂HPO₄ / KH₂PO₄ (1.5 M / 1 M) 10 g
- Métabisulfite de sodium en solution aqueuse à 50 % 1,3 g

Au moment de l'emploi les compositions tinctoriales des exemples 7, 8, et 10 à 12 ont été mélangées poids pour poids avec une solution de peroxyde d'hydrogène à 20 volumes (6% en poids).

Au moment de l'emploi la composition tinctoriale de l'exemple 9 a été mélangée poids pour poids avec une solution aqueuse contenant 6.10⁻³ mol% de persulfate d'ammonium.

Chacun des mélanges obtenu a été appliqué pendant 30 minutes sur des mèches de cheveux gris naturels à 90 % de blancs. Les cheveux ont ensuite rincés , lavés avec un shampooing standard, rincés à nouveau puis séchés.

Les cheveux ont été teints dans une nuance figurant dans le tableau ci-après :

| **EXEMPLE** | **pH de teinture** | **Nuance obtenue** |
|---|---|---|
| 7 | 6,4 ± 0,2 | Violet |
| 8 | 6,1 ± 0,2 | Orangé rosé |
| 9 | 3,2 ± 0,2 | Cendré violacé |
| 10 | 6,2 ± 0,2 | Rouge cuivré |
| 11 | 5,5 ± 0,2 | Violet profond |
| 12 | 5,5 ± 0,2 | Violet profond |

## Revendications

1. Composition pour la teinture d'oxydation des fibres kératiniques et en particulier des fibres kératiniques humaines telles que les cheveux, **caractérisée par le fait qu'**elle comprend, dans un milieu approprié pour la teinture :
- à titre de base d'oxydation, au moins un composé pyrazolo-azole de formule (I) et/ou au moins l'un de ses sels d'addition avec un acide : 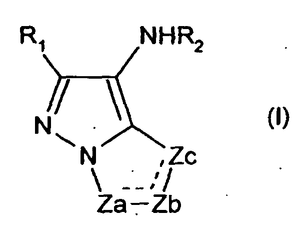
dans laquelle:
. R₁ représente : un atome d'hydrogène ; un radical alkyle en C₁-C₂₀, linéaire ou ramifié, éventuellement substitué par 1 ou 2 radicaux R choisis dans le groupe constitué par halogène, nitro, cyano, hydroxyle, alcoxy, aryloxy, amino, alkylamino, acylamino, carbamoyle, sulfonamido, sulfamoyle, imido, alkylthio, arylthio, aryle tel que phényle ou naphtyle, alcoxy(C₁-C₄)carbonyle, acyle un radical aryle (tel que phényle ou naphtyle), éventuellement substitué par 1 ou 2 radicaux R tels que précédemment définis ; un hétérocycle à 5 ou 6 chaînons possédant au moins un atome d'azote, d'oxygène ou de soufre (tel que pyridyle, quinolyle, pyrrolyle, morpholyle, furanyle, tétrahydrofuranyle, pyrazolyle, triazolyle, tétrazolyle, thiazolyle, oxazolyle, imidazolyle ou thiadiazolyle), ledit hétérocycle pouvant être substitué par 1 ou 2 radicaux R tels que définis précédemment ; un radical trifluoromethyle; un radical phenyle substitué par un trifluoromethyle; un cycle phényle substitué par un radical alkyle en C₁-C₄;
lorsque R₁ désigne un radical alkyle en C₁-C₄, un radical aryle ou un hétérocycle à 5 ou. 6 chaînons (tels que définis ci-dessus), il peut alors être relié à l'atome de carbone du noyau pyrazole par l'intermédiaire d'un atome d'oxygène, d'azote ou de soufre (dans ce cas, R₁ devient XR₁ avec X = O, NH ou S)
R₁ peut en outre désigner un atome d'halogène (tel que le brome, le chlore ou le fluor) ; un radical acyle ; un radical sulfonyle ; un radical sulfinyle ; un radical phosphonyle, un radical carbamoyle ; un radical sulfamoyle ; un radical cyano ; un radical siloxy ; un radical amino ; un radical acylamino ; un radical acyloxy ; un radical carbamoyloxy ; un radical sulfonamide ; un radical imide ; un radical uréido ; un radical sulfamoylamino ; un radical alcoxy(C₁-C₄)carbonylamino ; un radical aryloxycarbonylamino ; un radical alcoxy(C₁-C₄)carbonyle ; un radical aryloxycarbonyle ; un radical carboxyle ; un radical hydroxyle ;
un radical (CH₂)ₚ-X-(CH₂)_{q}-OR' dans lequel p et q sont des nombres entiers, identiques ou différents, compris entre 1 et 3 inclusivement, R' représente un atome d'hydrogène ou un radical méthyle et X désigne un atome d'oxygène ou un groupe NR" dans lequel R" désigne un atome d'hydrogène ou un radical méthyle ;
. R₂ désigne un atome d'hydrogène un radical alkyle en C₁-C₄ ; un radical monohydroxyalkyle en C₁-C₄ ; un radical polyhydroxyalkyle en C₂-C₄ ; un radical aminoalkyle en C₁-C₄ ; un radical (CH₂)ₚ-X-(CH₂)_{q}OR' dans lequel p et q sont des nombres entiers, identiques ou différents, compris entre 1 et 3 inclusivement, R' représente un atome d'hydrogène ou un radical méthyle, X désigne un atome d'oxygène ou un groupement NR" dans lequel R" représente un atome d'hydrogène ou un radical méthyle ; un radical alcoxy(C₁-C₄)alkyle en C₂-C₄ ; un radical di-alkyl(C₁-C₄)aminoalkyle en C₁-C₄ ;
. Zₐ, Z_{b}, Z_{c} représentent, indépendamment l'un de l'autre, un atome d'azote, un atome de carbone portant un radical R₃ ou R₄, R₃ et R₄ ayant les mêmes significations que celles indiquées ci-dessus pour le radical R₁ ainsi que phenyle substitué par un radical alkyl en C₁-C₄; sur radical methanesulfonyle ; sous réserve que l'un au moins des radicaux Zₐ, Z_{b} et Z_{c} soit différent d'un atome de carbone ; R₃ et R₄ peuvent également former ensemble un cycle aromatique substitué ou non tel qu'un cycle phényle.

2. Composition selon la revendication 1, **caractérisée par le fait que** les radicaux R₁ des composés de formule (I) sont choisis dans le groupe constitué par un atome d'hydrogène ; un radical alkyle en C₁-C₄, linéaire ou ramifié ; un cycle phényle ; un cycle phényle substitué par un atome d'halogène, un radical alkyle en C₁-C₄, un radical alcoxy en C₁-C₄, un groupe nitro, un groupe amino, un groupe trifluorométhyle ou par un radical alkylamino en C₁-C₄ ; un radical benzyle ; un radical benzyle substitué par un atome d'halogène, un radical alkyle en C₁-C₄, un radical alcoxy en C₁-C₄, un groupe nitro, un groupe amino, un groupe trifluorométhyle ou par un radical alkylamino en C₁-C₄ ; un hétérocycle choisi parmi les cycles thiophène, furane ou pyridine ; un radical trifluorométhyle ; un radical (CH₂)ₚ-X-(CH₂)_{q}-OR' dans lequel p et q sont des nombres entiers, identiques ou différents, compris entre 1 et 3 inclusivement, R' représente un atome d'hydrogène ou un radical méthyle et X désigne un atome d'oxygène ou un groupe NR" dans lequel R" désigne un atome d'hydrogène ou un radical méthyle ; un radical hydroxyalkyle en C₁-C₄ ; un radical aminoalkyle en C₁-C₄ ; un radical alkylamino en C₁-C₄ ; un radical dialkylamino en C₁-C₄ ; un radical arylamino ; un radical alcoxy choisi parmi méthoxy, éthoxy et phénoxy ; un atome d'halogène choisi parmi le brome, le chlore et le fluor ; un groupe carboxyle ; un radical alcoxycarbonyle en C₁-C₄ ; un radical phényloxy-carbonyle ; un radical méthylthio ; un radical éthylthio ; un radical phénylthio ; un radical méthanesulfonyle ; un radical cyano ; un radical amino ; un radical hydroxyle.

3. Composition selon la revendication 1 ou 2, **caractérisée par le fait que** les radicaux R₁ sont choisis dans le groupe constitué par un atome d'hydrogène ; un radical alkyle choisi parmi les radicaux méthyle, éthyle, isopropyle et ter-butyle ; un atome d'halogène choisi parmi le chlore et le fluor ; phényle ; toluyle ; 4-chlorophényle ; 4-méthoxyphényle ; 3-méthoxyphényle ; 2-méthoxyphényle ; benzyle ; un hétérocycle choisi parmi les cycles pyridyle, furyle et thiènyle ; trifluorométhyle ; hydroxyméthyle ; aminométhyle ; méthoxy ; éthoxy ; méthylamino ; éthylamino ; diméthylamino ; carboxyle ; méthoxycarbonyle ; éthoxycarbonyle et cyano.

4. Composition selon l'une quelconque des revendications précédentes, **caractérisée par le fait que** les radicaux R₂ des composés de formule (I) sont choisis dans le groupe constitué par un atome d'hydrogène ; un radical méthyle et un radical β-hydroxyéthyle.

5. Composition selon l'une quelconque des revendications précédentes, **caractérisée par le fait que** les radicaux R₃ et R₄ des composés de formule (I) sont choisis dans le groupe constitué par un atome d'hydrogène ; un radical hydroxyle ; un radical amino ; un radical alkyle en C₁-C₄, linéaire ou ramifié ; un radical trifluorométhyle ; un cycle phényle ; un cycle phényle substitué par un ou deux radicaux R choisis parmi un atome d'halogène, un radical alkyle en C₁-C₄, un radical alcoxy en C₁-C₄, un radical hydroxyle, un radical carboxyle, un groupe nitro, un radical alkylthio en C₁-C₄, un groupe méthylènedioxy, un groupe amino, un groupe trifluorométhyle et un radical alkylamino en C₁-C₄; un radical benzyle ; un radical benzyle substitué par un atome d'halogène, un radical méthyle ou isopropyle, un radical méthoxy ; un radical monohydroxyalkyle en C₁-C₄ ; un radical aminoalkyle en C₁-C₄ ; un radical alkyl(C₁-C₄)aminoalkyle en C₁-C₄ ; un radical alcoxy choisi parmi les radicaux méthoxy, éthoxy et phénoxy ; un radical méthylthio ; un radical éthylthio ; un radical phénylthio ; un radical méthanesulfonyle ; un cycle aromatique substitué ou non et formé conjointement par les radicaux R₃ et R₄ tel qu'un cycle phényle, pyridyle ou phényle substitué par un radical sulfonyle, un atome d'halogène, un radical alcoxy en C₁-C₄, un radical alkyle en C₁-C₄, un groupe nitro, un groupe cyano, un radical amino, un radical alkylamino en C₁-C₄, ou par un radical trifluorométhyle.

6. Composition selon la revendication 5, **caractérisée par le fait que** les radicaux R₃ et R₄ des composés de formule (I) sont choisis dans le groupe constitué par un atome d'hydrogène ; un radical alkyle choisi parmi les radicaux méthyle, éthyle, isopropyle, n-propyle et ter-butyle ; un cycle phényle ; un cycle toluyle ; un cycle 2-, 3- ou 4-chlorophényle ; un cycle 3- ou 4-hydroxyphényle ; un cycle 3- ou 4-aminophényle ; un cycle 3- ou 4-méthoxyphényle ; un cycle 4-trifluorométhylphényle ; un cycle benzyle ; un radical trifluorométhyle ; un radical hydroxyméthyle ; un radical hydroxyéthyle ; un radical hydroxyisopropyle ; un radical aminométhyle ou aminoéthyle ; un radical méthoxy ou éthoxy ; un radical méthylthio ou éthylthio ; un cycle aromatique substitué ou non et formé conjointement par les radicaux R₃ et R₄ tel qu'un cycle phényle, toluyle, sulfonylphényle et chlorophényle.

7. Composition selon l'une quelconque des revendications précédentes, **caractérisée par le fait que** les composés de formule (I) sont choisis parmi :
i) les pyrazolo [1,5-b] 1,2,4-triazoles de formule particulière (la) suivante, et leurs sels d'addition avec un acide : 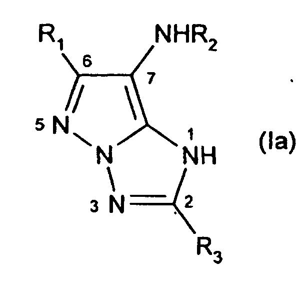
ii) les pyrazolo [3,2-c] 1,2,4-triazoles de formule particulière (Ib) suivante, et leurs sels d'addition avec un acide : 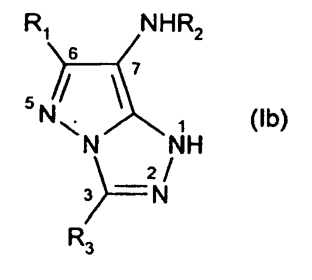
iii) les pyrazolotétrazoles de formule particulière (Ic) suivante, et leurs sels d'addition avec un acide : 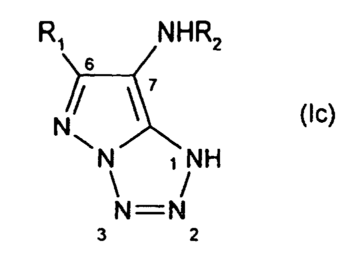
iv) les pyrazolo [1,5-a] imidazoles de formule particulière (Id) suivante, et leurs sels d'addition avec un acide : 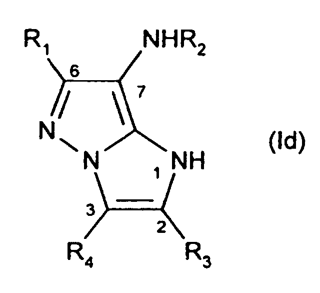
v) les pyrazolo [5,1-e] pyrazoles de formule particulière (le) suivante, et leurs sels d'addition avec un acide : 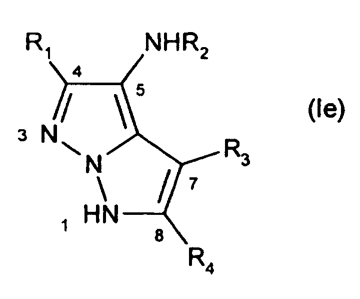
vi) les pyrazolo [5,1-e] 1,2,3-triazoles de formule particulière (If) suivante, et leurs sels d'addition avec un acide : 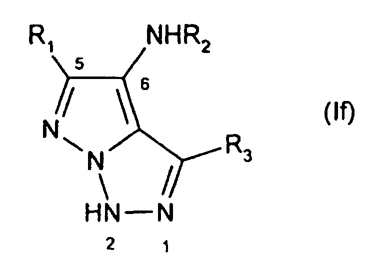 formules dans lesquelles R₁, R₂, R₃ et R₄ ont les mêmes significations que celles indiquées à l'une quelconque des revendications 1 à 6.

8. Composition selon la revendication 7, **caractérisée par le fait que** les composés de formules (la) ou (Ib) sont choisis parmi ceux pour lesquels :
- R₁ désigne un atome d'hydrogène, un radical méthyle, trifluorométhyle, carboxyle, phényle, éthoxy, cyano, éthylthio, amino ou hydroxyle ;
- R₂ désigne un atome d'hydrogène ;
- R₃ désigne un atome d'hydrogène, un radical méthyle, β-aminoéthyle, éthyle, isopropyle, phényle, β-hydroxyéthyle, méthylthio ou éthoxy.

9. Composition selon la revendication 7, **caractérisée par le fait que** les composés de formule (Ic) sont choisis parmi ceux lesquels:
- R₁ désigne un atome d'hydrogène, un radical méthyle, trifluorométhyle, carboxyle, phényle, éthoxy ou cyano ;
- R₂ désigne un atome d'hydrogène.

10. Composition selon la revendication 7, **caractérisée par le fait que** les composés de formule (Id) sont choisis parmi ceux pour lesquels:
- R₁ désigne un atome d'hydrogène, un radical méthyle, trifluorométhyle, carboxyle, phényle, éthoxy, cyano, amino, éthylthio ou hydroxyle ;
- R₂ désigne un atome d'hydrogène ;
- R₃ et R₄ désignent respectivement un atome d'hydrogène et un atome d'hydrogène, un atome d'hydrogène et un radical méthyle, un radical méthyle et un atome d'hydrogène, un atome d'hydrogène et un radical phényle ; un radical hydroxyle et un atome d'hydrogène ; R₃ et R₄ forment conjointement un cycle phényle.

11. Composition selon la revendication 7, **caractérisée par le fait que** les composés de formule (le) sont choisis parmi ceux pour lesquels :
- R₁ désigne un atome d'hydrogène, un radical méthyle, trifluorométhyle, carboxyle, phényle, éthoxy ou cyano ;
- R₂ désigne un atome d'hydrogène ;
- R₃ et R₄ désignent respectivement un atome d'hydrogène et un radical méthyle, un radical méthyle et un atome d'hydrogène, un radical méthyle et un radical méthyle, un atome d'hydrogène et un radical phényle, un atome d'hydrogène et un radical amino.

12. Composition selon la revendication 7, **caractérisée par le fait que** les composés de formule (If) sont choisis parmi ceux pour lesquels :
- R₁ désigne un atome d'hydrogène, un radical méthyle, trifluorométhyle, carboxyle, phényle, éthoxy ou cyano ;
- R₂ désigne un atome d'hydrogène ;
- R₃ désigne un atome d'hydrogène ou un radical méthyle.

13. Composition selon la revendication 7, **caractérisée par le fait que** les composés de formule (I) sont choisis parmi :
- le 7-amino-2-méthyl pyrazolo [1,5-b]-1,2,4-triazole ;
- le 7-amino-2-éthyl pyrazolo [1,5-b]-1,2,4-triazole ;
- le 7-amino-2-isopropyl pyrazolo [1,5-b]-1,2,4-triazole ;
- le 7-amino-2-phényl pyrazolo [1,5-b]-1,2,4-triazole ;
- le 7-amino-2,6-diméthyl pyrazolo [1,5-b]-1,2,4-triazole ;
- le 7-amino-6-méthyl-2-éthyl pyrazolo [1,5-b]-1,2,4-triazole ;
- le 7-amino-6-méthyl-2-isopropyl pyrazolo [1,5-b]-1,2,4-triazole ;
- le 7-amino- 6-méthyl-2-phényl pyrazolo [1,5-b]-1,2,4-triazole ;
- le 7-amino-6-carboxy-2-méthyl pyrazolo [1,5-b]-1,2,4-triazole ;
- le 7-amino-6-carboxy-2-éthyl pyrazolo [1,5-b]-1,2,4-triazole ;
- le7-amino- 6-carboxy-2-isopropyl pyrazolo [1,5-b]-1,2,4-triazole ;
- le 7-amino-6-carboxy-2-phényl pyrazolo [1,5-b]-1,2,4-triazole ;
- le 7-amino-6-phényl-2-méthyl pyrazolo [1,5-b]-1,2,4-triazole ;
- le 7-amino-6-phényl-2-éthyl pyrazolo [1,5-b]-1,2,4-triazole ;
- le 7-amino-6-phényl-2-isopropyl pyrazolo [1,5-b]-1,2,4-triazole ;
- le 6,7-diamino-2-méthyl pyrazolo [1,5-b]-1,2,4-triazole ;
- le 6,7-diamino-2-éthyl pyrazolo [1,5-b]-1,2,4-triazole ;
- le 6,7-diamino-2-isopropyl pyrazolo [1,5-b]-1,2,4-triazole ;
- le 6,7-diamino-2-phényl pyrazolo [1,5-b]-1,2,4-triazole ;
- le 7-amino-6-éthylthio-2-méthyl pyrazolo [1,5-b]-1,2,4-triazole ;
- le 7-amino-6-éthylthio-2-éthyl pyrazolo [1,5-b]-1,2,4-triazole ;
- le 7-amino-6-éthylthio-2-isopropyl pyrazolo [1,5-b]-1,2,4-triazole ;
- le 7-amino-6-éthylthio-2-phényl pyrazolo [1,5-b]-1,2,4-triazole ;
- le 7-amino-6-éthoxy-2-méthyl pyrazolo [1,5-b]-1,2,4-triazole ;
- le 7-amino-6-éthoxy-2-éthyl pyrazolo [1,5-b]-1,2,4-triazole ;
- le 7-amino-6-éthoxy-2-isopropyl pyrazolo [1,5-b]-1,2,4-triazole ;
- le 7-amino-6-éthoxy-2-phényl pyrazolo [1,5-b]-1,2,4-triazole ;
- le 7-amino-6-méthyl-2-(2'-aminoéthyl) pyrazolo [1,5-b]-1,2,4-triazole ;
- le 7-amino-6-carboxy-2-(2'-aminoéthyl) pyrazolo [1,5-b]-1,2,4-triazole ;
- le 7-amino-6-phényl-2-(2'-aminoéthyl) pyrazolo [1,5-b]-1,2,4-triazole ;
- le 7-amino-6-éthylthio-2-(2'-aminoéthyl) pyrazolo [1,5-b]-1,2,4-triazole ;
- le 7-amino-2-(2'-aminoéthyl) pyrazolo [1,5-b]-1,2,4-triazole ;
- le 7-amino-2-(2'-hydroxyéthyl) pyrazolo [1,5-b]-1,2,4-triazole ;
- le 7-amino-6-méthyl-2-(2'-hydroxyéthyl) pyrazolo [1,5-b]-1,2,4-triazole ;
- le 7-amino-6-éthylthio-2-(2'-hydroxyéthyl) pyrazolo [1,5-b]-1,2,4-triazole ;
- le 7-amino-6-carboxy-2-(2'-hydroxyéthyl) pyrazolo [1,5-b]-1,2,4-triazole ;
- le 7-amino-6-phényl-2-(2'-hydroxyéthyl) pyrazolo [1,5-b]-1,2,4-triazole ;
- le 6,7-diamino-2-(2'-aminoéthyl)-pyrazolo [1,5-b]-1,2,4-triazole ;
- le 6,7-diamino-2-(2'-hydroxyéthyl)-pyrazolo [1,5-b]-1,2,4-triazole ;
- le 7-amino-6-éthoxy-2-(2'-aminoéthyl)-pyrazolo [1,5-b]-1,2,4-triazole ;
- le 7-amino-6-éthoxy-2-(2'-hydroxyéthyl)-pyrazolo [1,5-b]-1,2,4-triazole ;
- le 7-amino-3-méthyl pyrazolo [3,2-c]-1,2,4-triazole ;
- le 7-amino-3-éthyl pyrazolo [3,2-c]-1,2,4-triazole ;
- le 7-amino-3-isopropyl pyrazolo [3,2-c]-1,2,4-triazole ;
- le 7-amino-3-phényl pyrazolo [3,2-c]-1,2,4-triazole ;
- le 7-amino-3-(2'-aminoéthyl) pyrazolo [3,2-c]-1,2,4-triazole ;
- le 7-amino-3-(2'-hydroxyéthyl) pyrazolo [3,2-c]-1,2,4-triazole ;
- le 7-amino-6-méthyl-3-éthyl pyrazolo [3,2-c]-1,2,4-triazole ;
- le 7-amino-3,6-diméthyl pyrazolo [3,2-c]-1,2,4-triazole ;
- le 7-amino-6-méthyl-3-isopropyl pyrazolo [3,2-c]-1,2,4-triazole ;
- le 7-amino-6-méthyl-3-phényl pyrazolo [3,2-c]-1,2,4-triazole ;
- le 7-amino-6-méthyl-3-(2'-aminoéthyl) pyrazolo [3,2-c]-1,2,4-triazole ;
- le 7-amino-6-méthyl-3-(2'-hydroxyéthyl) pyrazolo [3,2-c]-1,2,4-triazole ;
- le 7-amino-6-méthyl-3-méthylthio pyrazolo [3,2-c]-1,2,4-triazole ;
- le 7-amino-6-phényl-3-méthyl pyrazolo [3,2-c]-1,2,4-triazole ;
- le 7-amino-6-phényl-3-éthyl pyrazolo [3,2-c]-1,2,4-triazole ;
- le 7-amino-6-phényl-3-isopropyl pyrazolo [3,2-c]-1,2,4-triazole ;
- le 7-amino-6-phényl-3-phényl pyrazolo [3,2-c]-1,2,4-triazole ;
- le 7-amino-6-phényl-3-(2'-aminoéthyl) pyrazolo [3,2-c]-1,2,4-triazole ;
- le 7-amino-6-phényl-3-(2'-hydroxyéthyl) pyrazolo [3,2-c]-1,2,4-triazole ;
- le 7-amino-6-phényl-3-méthylthio pyrazolo [3,2-c]-1,2,4-triazole ;
- le 7-amino-6-éthylthio-3- méthyl pyrazolo [3,2-c]-1,2,4-triazole ;
- le 7-amino-6-éthylthio-3- éthyl pyrazolo [3,2-c]-1,2,4-triazole ;
- le 7-amino-6-éthylthio-3- isopropyl pyrazolo [3,2-c]-1,2,4-triazole ;
- le 7-amino-6-éthylthio-3- phényl pyrazolo [3,2-c]-1,2,4-triazole ;
- le 7-amino-6-éthylthio-3-(2'-aminoéthyl) pyrazolo [3,2-c]-1,2,4-triazole ;
- le 7-amino-6-éthylthio-3-(2'-hydroxyéthyl) pyrazolo [3,2-c]-1,2,4-triazole ;
- le 7-amino-6-trifluorométhyl-3-méthylthio pyrazolo [3,2-c]-1,2,4-triazole ;
- le 7-amino-6-trifluorométhyl pyrazolo [3,2-c]-1,2,4-triazole ;
- le 7-amino-6-carboxy-3-méthyl pyrazolo [3,2-c]-1,2,4-triazole ;
- le 7-amino-6-carboxy-3-éthyl pyrazolo [3,2-c]-1,2,4-triazole ;
- le 7-amino-6-carboxy-3-isopropyl pyrazolo [3,2-c]-1,2,4-triazole ;
- le 7-amino-6-carboxy-3-phényl pyrazolo [3,2-c]-1,2,4-triazole ;
- le 7-amino-6-carboxy-3-(2'-aminoéthyl) pyrazolo [3,2-c]-1,2,4-triazole ;
- le 7-amino-6-carboxy-3-(2'-hydroxyéthyl) pyrazolo [3,2-c]-1,2,4-triazole ;
- le 6,7-diamino-3-méthyl pyrazolo [3,2-c]-1,2,4-triazole ;
- le 6,7-diamino-3-éthyl pyrazolo [3,2-c]-1,2,4-triazole ;
- le 6,7-diamino-3-phényl pyrazolo [3,2-c]-1,2,4-triazole ;
- le 6,7-diamino-3-isopropyl pyrazolo [3,2-c]-1,2,4-triazole ;
- le 7-amino-6-éthoxy-3-méthyl pyrazolo [3,2-c]-1,2,4-triazole ;
- le 7-amino-6-éthoxy-3-éthyl pyrazolo [3,2-c]-1,2,4-triazole ;
- le 7-amino-6-éthoxy-3-isopropyl pyrazolo [3,2-c]-1,2,4-triazole ;
- le 7-amino-6-éthoxy-3-phényl pyrazolo [3,2-c]-1,2,4-triazole ;
- le 7-amino-6-éthoxy-3-(2'-aminoéthyl) pyrazolo [3,2-c]-1,2,4-triazole ;
- le 7-amino-6-éthoxy-3-(2'-hydroxyéthyl) pyrazolo [3,2-c]-1,2,4-triazole ;
- le 6,7-diamino-3-(2'-aminoéthyl) pyrazolo [3,2-c]-1,2,4-triazole ;
- le 6,7-diamino-3-(2'-hydroxyéthyl pyrazolo [3,2-c]-1,2,4-triazole ;
- le 7-amino-6-méthyl pyrazolo [5,1-e] tétrazole ;
- le 7-amino-6-phényl pyrazolo [5,1-e] tétrazole ;
- le 7-amino-6-carboxy pyrazolo [5,1-e] tétrazole ;
- le 7-amino-2-méthyl pyrazolo [1,5-a] imidazole ;
- le 7-amino-2-phényl pyrazolo [1,5-a] imidazole ;
- le 7-amino pyrazolo [1,5-a] benzimidazole ;
- le 7-amino-6-méthyl pyrazolo [1,5-a] imidazole ;
- le 7-amino-2,6-diméthyl pyrazolo [1,5-a] imidazole ;
- le 7-amino-6-méthyl-2-phényl pyrazolo [1,5-a] imidazole ;
- le 7-amino-6-méthyl pyrazolo [1,5-a] benzimidazole ;
- le 7-amino-6-phényl pyrazolo [1,5-a] imidazole ;
- le 7-amino-6-phényl-2-méthyl pyrazolo [1,5-a] imidazole ;
- le 7-amino-2,6-diphényl pyrazolo [1,5-a] imidazole ;
- le 7-amino-6-phényl pyrazolo [1,5-a] benzimidazole ;
- le 7-amino-6-carboxy pyrazolo [1,5-a] imidazole ;
- le 7-amino-6-carboxy-2-méthyl pyrazolo [1,5-a] imidazole ;
- le 7-amino-6-carboxy-2-phényl pyrazolo [1,5-a] imidazole ;
- le 7-amino-6-carboxy pyrazolo [1,5-a] benzimidazole ;
- le 7-amino-6-éthoxy pyrazolo [1,5-a] imidazole ;
- le 7-amino-6-éthoxy-2-méthyl pyrazolo [1,5-a] imidazole ;
- le 7-amino-6-éthoxy-2-phényl pyrazolo [1,5-a] imidazole ;
- le 7-amino-6-éthoxy pyrazolo [1,5-a] benzimidazole ;
- le 7-amino-6-trifluorométhyl pyrazolo [1,5-a] benzimidazole ;
- le 6,7-diamino pyrazolo [1,5-a] imidazole ;
- le 6,7-diamino-2-méthyl pyrazolo [1,5-a] imidazole ;
- le 6,7-diamino-2-phényl pyrazolo [1,5-a] imidazole ;
- le 6,7-diamino pyrazolo [1,5-a] benzimidazole ;
- le 7-amino-6-éthylthio pyrazolo [1,5-a] imidazole ;
- le 7-amino-6-éthylthio-2-méthyl pyrazolo [1,5-a] imidazole ;
- le 7-amino-6-éthylthio-2-phényl pyrazolo [1,5-a] imidazole ;
- le 7-amino-6-méthyl-2-hydroxy pyrazolo [1,5-a] imidazole ;
- le 7-amino-6-phényl-2-hydroxy pyrazolo [1,5-a] imidazole ;
- le 7-amino-6-éthoxy-2-hydroxy pyrazolo [1,5-a] imidazole ;
- le 7-amino-6-éthylthio-2-hydroxy pyrazolo [1,5-a] imidazole ;
- le 6,7-diamino-2-hydroxy pyrazolo [1,5-a] imidazole ;
- le 5,8-diamino-4-méthyl pyrazolo [5,1-e] pyrazole ;
- le 5,7,8-triamino-4-méthyl pyrazolo [5,1-e] pyrazole ;
- le 6-amino-5-méthyl pyrazolo [5,1-e]-1,2,3-triazole ;
- le 6-amino-5-phényl pyrazolo [5,1-e]-1,2,3-triazole ;
et leurs sels d'addition avec un acide.

14. Composition selon l'une quelconque des revendications précédentes, **caractérisée par le fait que** le ou les composés de formule (I) représentent de 0,0005 à 12 % en poids du poids total de la composition tinctoriale.

15. Composition selon la revendication 14, **caractérisée par le fait que** le ou les composés de formule (I) représentent de 0,005 à 6 % en poids du poids total de la composition tinctoriale.

16. Composition selon l'une quelconque des revendications précédentes, **caractérisée par le fait que** le milieu approprié pour la teinture (ou support) est constitué par de l'eau ou par un mélange d'eau et d'au moins un solvant organique.

17. Composition selon l'une quelconque des revendications précédentes, **caractérisée par le fait qu'**elle présente un pH compris entre 3 et 12 et de préférence entre 5 et 11.

18. Composition selon l'une quelconque des revendications précédentes, **caractérisée par le fait qu'**elle renferme un ou plusieurs coupleurs choisis parmi les métaphénylènediamines, les méta-aminophénols, les métadiphénols et les coupleurs hétérocycliques.

19. Composition selon la revendication 18, **caractérisée par le fait que** tes coupleurs sont choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthyl)amino 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, la 1H 3-méthyl pyrazol 5-one, la 1-phényl 3-méthyl pyrazol 5-one, le 2,6-diméthyl pyrazolo [1,5-b]-1,2,4-triazole, le 2,6-diméthyl [3,2-c]-1,2,4-triazole, le 6-méthyl pyrazolo [1,5-a] benzimidazole, et leurs sels d'addition avec un acide.

20. Composition selon l'une quelconque des revendications 18 ou 19, **caractérisée par le fait que** le ou les coupleurs représentent de 0,0001 à 10 % en poids du poids total de la composition tinctoriale.

21. Composition selon l'une quelconque des revendications précédentes, **caractérisée par le fait qu'**elle contient au moins une base d'oxydation additionnelle choisie parmi les paraphénylènediamines, les bis-phénylalkylènediamines, les para-aminophénols, les ortho-aminophénols et les bases hétérocycliques différentes des composés pyrazolo-azoles de formule (I).

22. Composition selon l'une quelconque des revendications précédentes, **caractérisée par le fait que** les sels d'addition avec un acide sont choisis parmi les chlorhydrates, les bromhydrates, les sulfates, les citrates, les succinates, les tartrates, les tosylates, les benzènesulfonates, les lactates et les acétates.

23. Utilisation des composés de formule (I) tels que définis à l'une quelconques des revendications 1 à 13 et 22, à titre de base d'oxydation dans des compositions pour la teinture d'oxydation des fibres kératiniques et en particulier des fibres kératiniques humaines telles que les cheveux.

24. Procédé de teinture des fibres kératiniques et en particulier des fibres kératiniques humaines telles que les cheveux, **caractérisé par le fait qu'**on applique sur les fibres au moins une composition tinctoriale telle que définie à l'une quelconque des revendications 1 à 22, pendant un temps suffisant pour développer la coloration désirée, soit à l'air, soit à l'aide d'un agent oxydant.

25. Procédé de teinture selon la revendication 24, **caractérisé par le fait que** la coloration des fibres est effectuée sans addition d'un agent oxydant, au seul contact de l'oxygène de l'air.

26. Procédé de teinture selon la revendication 24, **caractérisé par le fait que** la couleur est révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

27. Procédé selon la revendication 26, **caractérisé par le fait que** l'agent est choisi parmi le peroxyde d'hydrogène, le peroxyde d'urée, les bromates de métaux alcalins, les persels tels que les perborates et persulfates et les enzymes telles que les peroxydases et les oxydo-réductases à 2 électrons.

28. Dispositif à plusieurs compartiments ou "kit" de teinture à plusieurs compartiments, dont un premier compartiment renferme une composition tinctoriale telle que définie à l'une quelconque des revendications 1 à 22 et un second compartiment renferme une composition oxydante.

29. Composés de formule (I), ainsi que leurs sels d'addition avec un acide, de formules (l'a) à (I'f) suivantes : 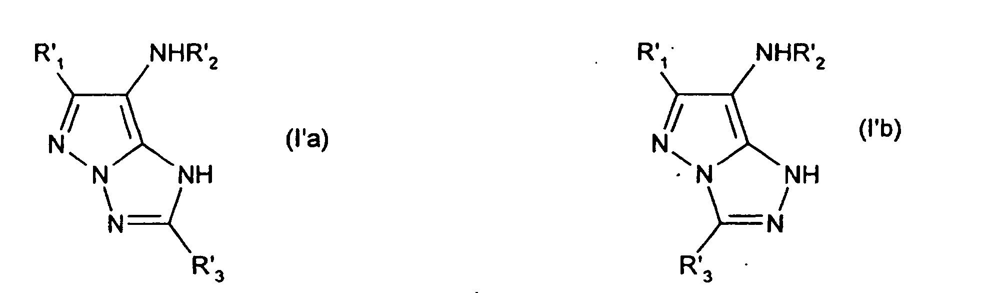 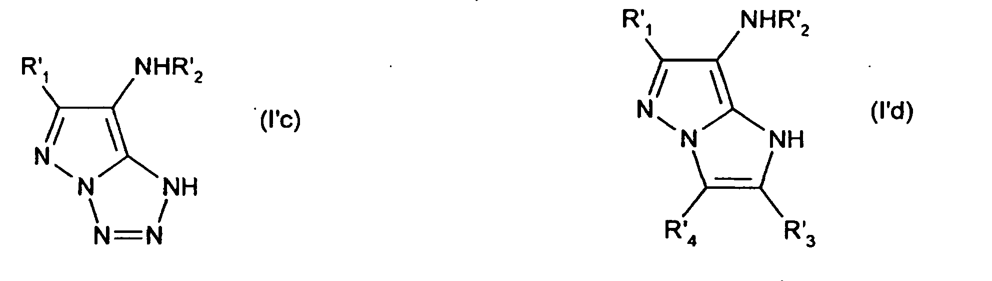 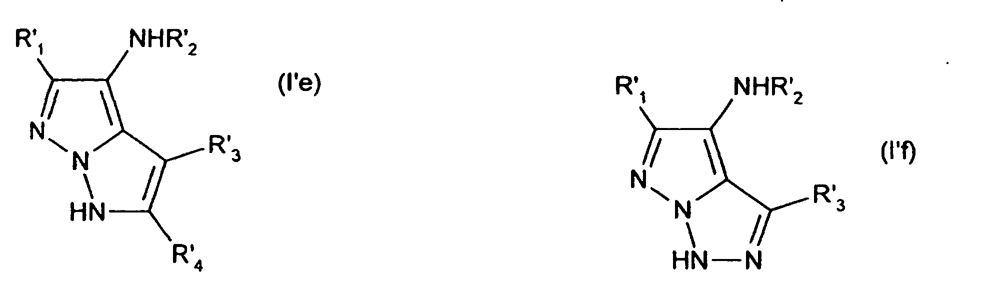 dans lesquelles les radicaux R'₁, R'₂, R'₃ et R'₄ ont les mêmes significations que celles indiquées à l'une quelconque des revendications 1 à 12 pour les radicaux R₁, R₂, R₃ et R₄ de la formule (I) ; étant entendu que :
- dans les composés de formule (I'b) :
i) lorsque R'₁ représente un radical méthyle, et que R'₂ représente un atome d'hydrogène, alors le radical R'₃ est différent d'un radical méthyle ou d'un radical (CH₂)₃-SO₂-(CH₂)₁₅-CH₃, ou CH(CH₃)-CH₂-SO₂-(CH₂)₁₁-CH₃ ;
ii) lorsque R'₁ représente un radical ter-butyle, et que R'₂ représente un atome d'hydrogène, alors R'₃ est différent d'un radical (CH₂)₃-SO₂-(CH₂)₁₁-CH₃ ;
- dans les composés de formule (I'd) :
- lorsque R'₃ et R'₄ forment ensemble un noyau benzénique, et que R'₂ représente un atome d'hydrogène, alors R'₁ est différent d'un radical méthyle, -CONH₂ ou carboxyle;
- lorsque R'₃ et R'₄ forment ensemble un noyau benzénique substitué par un radical sulfonyle en position 4, et que R'₂ représente un atome d'hydrogène, alors R'₁ est différent d'un radical phényle, d'un radical -(CH₂)₁₆-CH₃ ; ou d'un radical -COOH ;
- lorsque R'₃ et R'₄ forment ensemble un noyau benzénique substitué par un radical carboxylique en position 4, et que R'₂ représente un atome d'hydrogène, alors R'₁ est différent d'un radical carboxylique.

30. Composés de formule (I') selon la revendication 29, **caractérisé par le fait qu'**ils sont choisis parmi :
- le 7-amino-2-méthyl pyrazolo [1,5-b]-1,2,4-triazole ;
- le 7-amino-2-éthyl pyrazolo [1,5-b]-1,2,4-triazole ;
- le 7-amino-2-isopropyl pyrazolo [1,5-b]-1,2,4-triazole ;
- le 7-amino-2-phényl pyrazolo [1,5-b]-1,2,4-triazole ;
- le 7-amino-2,6-diméthyl pyrazolo [1,5-b]-1,2,4-triazole ;
- le 7-amino-6-méthyl-2-éthyl pyrazolo [1,5-b]-1,2,4-triazole ;
- le 7-amino-6-méthyl-2-isopropyl pyrazolo [1,5-b]-1,2,4-triazole ;
- le 7-amino- 6-méthyl-2-phényl pyrazolo [1,5-b]-1,2,4-triazole ;
- le 7-amino-6-carboxy-2-méthyl pyrazolo [1,5-b]-1,2,4-triazole ;
- le 7-amino-6-carboxy-2-éthyl pyrazolo [1 ,5-b]-1 ,2,4-triazole ;
- le7-amino- 6-carboxy-2-isopropyl pyrazolo [1,5-b]-1,2,4-triazole ;
- le 7-amino-6-carboxy-2-phényl pyrazolo [1,5-b]-1,2,4-triazole ;
- le 7-amino-6-phényl-2-méthyl pyrazolo [1,5-b]-1,2,4-triazole ;
- le 7-amino-6-phényl-2-éthyl pyrazolo [1,5-b]-1,2,4-triazole ;
- le 7-amino-6-phényl-2-isopropyl pyrazolo [1,5-b]-1,2,4-triazole ;
- le 6,7-diamino-2-méthyl pyrazolo [1,5-b]-1,2,4-triazole ;
- le 6,7-diamino-2-éthyl pyrazolo [1,5-b]-1,2,4-triazole ;
- le 6,7-diamino-2-isopropyl pyrazolo [1,5-b]-1,2,4-triazole ;
- le 6,7-diamino-2-phényl pyrazolo [1,5-b]-1,2,4-triazole ;
- le 7-amino-6-éthylthio-2-méthyl pyrazolo [1,5-b]-1,2,4-triazole ;
- le 7-amino-6-éthylthio-2-éthyl pyrazolo [1,5-b]-1,2,4-triazole ;
- le 7-amino-6-éthylthio-2-isopropyl pyrazolo [1,5-b]-1,2,4-triazole ;
- le 7-amino-6-éthylthio-2-phényl pyrazolo [1,5-b]-1,2,4-triazole ;
- le 7-amino-6-éthoxy-2-méthyl pyrazolo [1,5-b]-1,2,4-triazole ;
- le 7-amino-6-éthoxy-2-éthyl pyrazolo [1,5-b]-1,2,4-triazole ;
- le 7-amino-6-éthoxy-2-isopropyl pyrazolo [1,5-b]-1,2,4-triazole ;
- le 7-amino-6-éthoxy-2-phényl pyrazolo [1,5-b]-1,2,4-triazole ;
- le 7-amino-6-méthyl-2-(2'-aminoéthyl) pyrazolo [1,5-b]-1,2,4-triazole ;
- le 7-amino-6-carboxy-2-(2'-aminoéthyl) pyrazolo [1,5-b]-1,2,4-triazole ;
- le 7-amino-6-phényl-2-(2'-aminoéthyl) pyrazolo [1,5-b]-1,2,4-triazole ;
- le 7-amino-6-éthylthio-2-(2'-aminoéthyl) pyrazolo [1,5-b]-1,2,4-triazole ;
- le 7-amino-2-(2'-aminoéthyl) pyrazolo [1,5-b]-1,2,4-triazole ;
- le 7-amino-2-(2'-hydroxyéthyl) pyrazolo [1,5-b]-1,2,4-triazole ;
- le 7-amino-6-méthyl-2-(2'-hydroxyéthyl) pyrazolo [1,5-b]-1,2,4-triazole ;
- le 7-amino-6-éthylthio-2-(2'-hydroxyéthyl) pyrazolo [1,5-b]-1,2,4-triazole ;
- le 7-amino-6-carboxy-2-(2'-hydroxyéthyl) pyrazolo [1,5-b]-1,2,4-triazole ;
- le 7-amino-6-phényl-2-(2'-hydroxyéthyl) pyrazolo [1,5-b]-1,2,4-triazole ;
- le 6,7-diamino-2-(2'-aminoéthyl) pyrazolo [1,5-b]-1,2,4-triazole ;
- le 6,7-diamino-2-(2'-hydroxyéthyl) pyrazolo [1,5-b]-1,2,4-triazole ;
- le 7-amino-6-éthoxy-2-(2'-aminoéthyl) pyrazolo [1,5-b]-1,2,4-triazole ;
- le 7-amino-6-éthoxy-2-(2'-hydroxyéthyl) pyrazolo [1,5-b]-1,2,4-triazole ;
- le 7-amino-3-méthyl pyrazolo [3,2-c]-1,2,4-triazole ;
- le 7-amino-3-éthyl pyrazolo [3,2-c]-1,2,4-triazole ;
- le 7-amino-3-isopropyl pyrazolo [3,2-c]-1,2,4-triazole ;
- le 7-amino-3-phényl pyrazolo [3,2-c]-1,2,4-triazole ;
- le 7-amino-3-(2'-aminoéthyl) pyrazolo [3,2-c]-1,2,4-triazole ;
- le 7-amino-3-(2'-hydroxyéthyl) pyrazolo [3,2-c]-1,2,4-triazole ;
- le 7-amino-6-méthyl-3-éthyl pyrazolo [3,2-c]-1,2,4-triazole ;
- le 7-amino-3,6-diméthyl pyrazolo [3,2-c]-1,2,4-triazole ;
- le 7-amino-6-méthyl-3-isopropyl pyrazolo [3,2-c]-1,2,4-triazole ;
- le 7-amino-6-méthyl-3-phényl pyrazolo [3,2-c]-1,2,4-triazole ;
- le 7-amino-6-méthyl-3-(2'-aminoéthyl) pyrazolo [3,2-c]-1,2,4-triazole ;
- le 7-amino-6-méthyl-3-(2'-hydroxyéthyl) pyrazolo [3,2-c]-1,2,4-triazole ;
- le 7-amino-6-méthyl-3-méthylthio pyrazolo [3,2-c]-1,2,4-triazole ;
- le 7-amino-6-phényl-3-méthyl pyrazolo [3,2-c]-1,2,4-triazole ;
- le 7-amino-6-phényl-3-éthyl pyrazolo [3,2-c]-1,2,4-triazole ;
- le 7-amino-6-phényl-3-isopropyl pyrazolo [3,2-c]-1,2,4-triazole ;
- le 7-amino-6-phényl-3-phényl pyrazolo [3,2-c]-1,2,4-triazole ;
- le 7-amino-6-phényl-3-(2'-aminoéthyl) pyrazolo [3,2-c]-1,2,4-triazole ;
- le 7-amino-6-phényl-3-(2'-hydroxyéthyl) pyrazolo [3,2-c]-1,2,4-triazole ;
- le 7-amino-6-phényl-3-méthylthio pyrazolo [3,2-c]-1,2,4-triazole ;
- le 7-amino-6-éthylthio-3-méthyl pyrazolo [3,2-c]-1,2,4-triazole ;
- le 7-amino-6-éthylthio-3-éthyl pyrazolo [3,2-c]-1,2,4-triazole ;
- le 7-amino-6-éthylthio-3-isopropyl pyrazolo [3,2-c]-1,2,4-triazole ;
- le 7-amino-6-éthylthio-3-phényl pyrazolo [3,2-c]-1,2,4-triazole ;
- le 7-amino-6-éthylthio-3-(2'-aminoéthyl) pyrazolo [3,2-c]-1,2,4-triazole ;
- le 7-amino-6-éthylthio-3-(2'-hydroxyéthyl) pyrazolo [3,2-c]-1,2,4-triazole ;
- le 7-amino-6-trifluorométhyl-3-méthylthio pyrazolo [3,2-c]-1,2,4-triazole ;
- le 7-amino-6-trifluorométhyl pyrazolo [3,2-c]-1,2,4-triazole ;
- le 7-amino-6-carboxy-3-méthyl pyrazolo [3,2-c]-1,2,4-triazole ;
- le 7-amino-6-carboxy-3-éthyl pyrazolo [3,2-c]-1,2,4-triazole ;
- le 7-amino-6-carboxy-3-isopropyl pyrazolo [3,2-c]-1,2,4-triazole ;
- le 7-amino-6-carboxy-3-phényl pyrazolo [3,2-c]-1,2,4-triazole ;
- le 7-amino-6-carboxy-3-(2'-aminoéthyl) pyrazolo [3,2-c]-1,2,4-triazole ;
- le 7-amino-6-carboxy-3-(2'-hydroxyéthyl) pyrazolo [3,2-c]-1,2,4-triazole ;
- le 6,7-diamino-3-méthyl pyrazolo [3,2-c]-1,2,4-triazole;
- le 6,7-diamino-3-éthyl pyrazolo [3,2-c]-1,2,4-triazole ;
- le 6,7-diamino-3-phényl pyrazolo [3,2-c]-1,2,4-triazole ;
- le 6,7-diamino-3-isopropyl pyrazolo [3,2-c]-1,2,4-triazole ;
- le 7-amino-6-éthoxy-3-méthyl pyrazolo [3,2-c]-1,2,4-triazole ;
- le 7-amino-6-éthoxy-3-éthyl pyrazolo [3,2-c]-1,2,4-triazole ;
- le 7-amino-6-éthoxy-3-isopropyl pyrazolo [3,2-c]-1,2,4-triazole ;
- le 7-amino-6-éthoxy-3-phényl pyrazolo [3,2-c]-1,2,4-triazole ;
- le 7-amino-6-éthoxy-3-(2'-aminoéthyl) pyrazolo [3,2-c]-1,2,4-triazole ;
- le 7-amino-6-éthoxy-3-(2'-hydroxyéthyl) pyrazolo [3,2-c]-1,2,4-triazole ;
- le 6,7-diamino-3-(2'-aminoéthyl) pyrazolo [3,2-c]-1,2,4-triazole ;
- le 6,7-diamino-3-(2'-hydroxyéthyl) pyrazolo [3,2-c]-1,2,4-triazole ;
- le 7-amino-6-méthyl pyrazolo [5,1-e] tétrazole ;
- le 7-amino-6-phényl pyrazolo [5,1-e] tétrazole ;
- le 7-amino-6-carboxy pyrazolo [5,1-e] tétrazole ;
- le 7-amino pyrazolo [1,5-a] imidazole ;
- le 7-amino-2-méthyl pyrazolo [1,5-a] imidazole ;
- le 7-amino-2-phényl pyrazolo [1,5-a] imidazole ;
- le 7-amino pyrazolo [1,5-a] benzimidazole ;
- le 7-amino-6-méthyl pyrazolo [1,5-a] imidazole ;
- le 7-amino-2,6-diméthyl pyrazolo [1,5-a] imidazole ;
- le 7-amino-6-méthyl-2-phényl pyrazolo [1,5-a] imidazole ;
- le 7-amino-6-phényl pyrazolo [1,5-a] imidazole ;
- le 7-amino-6-phényl-2-méthyl pyrazolo [1,5-a] imidazole ;
- le 7-amino-2,6-diphényl pyrazolo [1,5-a] imidazole ;
- le 7-amino-6-phényl pyrazolo [1,5-a] benzimidazole ;
- le 7-amino-6-carboxy pyrazolo [1,5-a] imidazole ;
- le 7-amino-6-carboxy-2-méthyl pyrazolo [1,5-a] imidazole;
- le 7-amino-6-carboxy-2-phényl pyrazolo [1,5-a] imidazole ;
- le 7-amino-6-éthoxy pyrazolo [1,5-a] imidazole ;
- le 7-amino-6-éthoxy-2-méthyl pyrazolo [1,5-a] imidazole ;
- le 7-amino-6-éthoxy-2-phényl pyrazolo [1,5-a] imidazole ;
- le 7-amino-6-éthoxy pyrazolo [1,5-a] benzimidazole ;
- le 7-amino-6-trifluorométhyl pyrazolo [1,5-a] benzimidazole ;
- le 6,7-diamino pyrazolo [1,5-a] imidazole ;
- le 6,7-diamino-2-méthyl pyrazolo [1,5-a] imidazole ;
- le 6,7-diamino-2-phényl pyrazolo [1,5-a] imidazole ;
- le 6,7-diamino pyrazolo [1,5-a] benzimidazole ;
- le 7-amino-6-éthylthio pyrazolo [1,5-a] imidazole ;
- le 7-amino-6-éthylthio-2-méthyl pyrazolo [1,5-a] imidazole ;
- le 7-amino-6-éthylthio-2-phényl pyrazolo [1,5-a] imidazole ;
- le 7-amino-6-méthyl-2-hydroxy pyrazolo [1,5-a] imidazole ;
- le 7-amino-6-phényl-2-hydroxy pyrazolo [1,5-a] imidazole ;
- le 7-amino-6-éthoxy-2-hydroxy pyrazolo [1,5-a] imidazole ;
- le 7-amino-6-éthylthio-2-hydroxy pyrazolo [1,5-a] imidazole ;
- le 6,7-diamino-2-hydroxy pyrazolo [1,5-a] imidazole;
- le 5,8-diamino-4-méthyl pyrazolo [5,1-e] pyrazole ;
- le 5,7,8-triamino-4-méthyl pyrazolo [5,1-e] pyrazole ;
- le 6-amino-5-méthyl pyrazolo [5,1-e]-1,2,3-triazole ;
- le 6-amino-5-phényl pyrazolo [5,1-e]-1,2,3-triazole ;
et leurs sels d'addition avec un acide.

## Patentansprüche

1. Zusammensetzung zum oxidativen Färben von Keratinfasern und insbesondere menschlichen Keratinfasern, wie dem Haar, **dadurch gekennzeichnet, dass** sie in einem zum Färben geeigneten Träger enthält:
- als Oxidationsbase mindestens ein Pyrazolo-azol der Formel (I) und/oder mindestens ein Additionssalz dieser Verbindungen mit einer Säure: 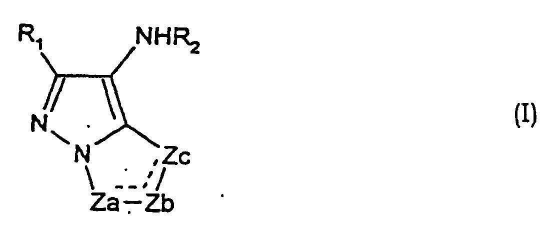
worin bedeuteten:
- R₁ ein Wasserstoffatom; eine geradkettige oder verzweigte C₁₋₂₀- Alkylgruppe, die gegebenenfalls mit einer Gruppe R oder mit zwei Gruppen R substituiert ist, die unter den folgenden Gruppen ausgewählt sind: Halogen, Nitro, Cyano, Hydroxy, Alkoxy, Aryloxy, Amino, Alkylamino, Acylamino, Carbamoyl, Sulfonamido, Sulfamoyl, Imido, Alkylthio, Arylthio, Aryl, wie Phenyl oder Naphthyl, C₁₋₄-Alkoxycarbonyl oder Acyl; eine Arylgruppe (wie Phenyl oder Naphthyl), die gegebenenfalls mit einer oder zwei Gruppen R substituiert ist, wie sie oben definiert wurden; einen 5- oder 6-gliedrigen Heterocyclus, der mindestens ein Stickstoffatom, Sauerstoffatom oder Schwefelatom enthält (wie Pyridyl, Chinolyl, Pyrrolyl, Morpholyl, Furanyl, Tetrahydrofuranyl, Pyrazolyl, Triazolyl, Tetrazolyl, Thiazolyl, Oxazolyl, Imidazolyl oder Thiadiazolyl), wobei der Heterocyclus mit einer oder zwei Gruppen R substituiert sein kann, wie sie oben definiert wurden; die Trifluormethylgruppe; eine mit Trifluormethyl substituierte Phenylgruppe; ein mit einer C₁₋₄-Alkylgruppe substituierter Phenylring;
wenn die Gruppe R₁ eine C₁₋₄-Alkylgruppe, eine Arylgruppe oder einen 5- oder 6-gliedrigen Heterocyclus (wie sie oben definiert wurden) bedeutet, kann sie auch über ein Sauerstoffatom, Stickstoffatom oder Schwefelatom an das Kohlenstoffatom des Pyrazolrings gebunden sein (in diesem Fall wird R₁ XR₁, mit X = O, NH oder S);
R₁ kann ferner bedeuten: ein Halogenatom (wie Brom, Chlor oder Fluor); Acyl; Sulfonyl; Sulfinyl; Phosphonyl; Carbamoyl; Sulfamoyl; Cyano; Siloxy; Amino; Acylamino; Acyloxy; Carbamoyloxy; Sulfonamid; Imid; Ureido; Sulfamoylamino; C₁₋₄-Alkoxycarbonylamino; Aryloxycarbonylamino; C₁₋₄-Alkoxycarbonyl; Aryloxycarbonyl; Carboxy; Hydroxy; eine Gruppe (CH₂)ₚ-X-(CH₂)_{q}-OR', wobei p und q ganze Zahlen bedeuten, die gleich oder verschieden sind und im Bereich von 1 bis 3 liegen, R' ein Wasserstoffatom oder die Methylgruppe bedeutet und X ein Sauerstoffatom oder eine Gruppe NR" ist, worin R" Wasserstoff oder Methyl bedeutet;
- R₂ ein Wasserstoffatom; C₁₋₄-Alkyl; C₁₋₄-Monohydroxyalkyl; C₂₋₄-Polyhydroxyalkyl; C₁₋₄-Aminoalkyl; eine Gruppe (CH₂)ₚ-X-(CH₂)_{q}-OR', wobei p und q ganze Zahlen bedeuten, die gleich oder verschieden sind und im Bereich von 1 bis 3 liegen, R' Wasserstoff oder Methyl bedeutet und X ein Sauerstoffatom oder eine Gruppe NR" ist, wobei R" Wasserstoff oder Methyl bedeutet; C₁₋₄-alkoxy-C₂₋₄-alkyl; oder Di-C₁₋₄-alkylamino-C₁₋₄-alkyl;
- Zₐ, Z_{b} und Z_{c} unabhängig voneinander ein Stickstoffatom oder ein Kohlenstoffatom, das eine Gruppe R₃ oder R₄, wobei R₃ und R₄ die oben für die Gruppe R₁ angegebenen Bedeutungen aufweisen, sowie eine mit einer C₁₋₄-Alkylgruppe substituierte Phenylgruppe; oder eine Methansulfonylgruppe trägt; mit der Maßgabe, dass mindestens eines der Atome Zₐ, Z_{b} und Z_{c} von einem Kohlenstoffatom verschieden ist; R₃ und R₄ können auch gemeinsam einen substituierten oder unsubstituierten aromatischen Ring bilden, wie beispielsweise einen Phenylring.

2. Zusammensetzung nach Anspruch 1, **dadurch gekennzeichnet, dass** die Gruppen R₁ der Verbindungen der Formel (I) unter dem Wasserstoffatom; einer geradkettigen oder verzweigten C₁₋₄-Alkylgruppe; Phenyl; einem mit einem Halogenatom, C₁₋₄-Alkyl, C₁₋₄-Alkoxy, Nitro, Amino, Trifluormethyl oder C₁₋₄-Alkylamino substituierten Phenylring; Benzyl; einer mit einem Halogenatom, C₁₋₄-Alkyl, C₁₋₄-Alkoxy, Nitro, Amino, Trifluormethyl oder C₁₋₄-Alkylamino substituierten Benzylgruppe; einem Heterocyclus, der unter Thiophen, Furan oder Pyridin ausgewählt ist; einer Trifluormethylgruppe; einer Gruppe (CH₂)ₚ-X-(CH₂)_{q}-OR', worin p und q ganze Zahlen bedeuten, die gleich oder verschieden sind und im Bereich von 1 bis 3 liegen, R' Wasserstoff oder Methyl bedeutet und X ein Sauerstoffatom oder eine Gruppe NR" ist, wobei R" Wasserstoff oder Methyl bedeutet; C₁₋₄-Hydroxyalkyl; C₁₋₄-Aminoalkyl; C₁₋₄-Alkylamino; C₁₋₄-Dialkylamino; Arylamino; einer Alkoxygruppe, die unter Methoxy, Ethoxy und Phenoxy ausgewählt ist; einem Halogenatom, das unter Brom, Chlor und Fluor ausgewählt ist; Carboxy; C₁₋₄-Alkoxycarbonyl; Phenyloxycarbonyl; Methylthio; Ethylthio; Phenylthio; Methansulfonyl; Cyano; Amino; oder Hydroxy ausgewählt ist.

3. Zusammensetzung nach Anspruch 1 oder 2, **dadurch gekennzeichnet, dass** die Gruppen R₁ unter Wasserstoff; einer Alkylgruppe, die unter Methyl, Ethyl, Isopropyl und *t*-Butyl ausgewählt ist; einem Halogenatom, das unter Chlor und Fluor ausgewählt ist; Phenyl; Toluyl; 4-Chlorphenyl; 4-Methoxyphenyl; 3-Methoxyphenyl; 2-Methoxyphenyl, Benzyl; einem Heterocyclus, der unter Pyridyl, Furyl und Thienyl ausgewählt ist; Trifluormethyl; Hydroxymethyl; Aminomethyl; Methoxy; Ethoxy; Methylamino; Ethylamino; Dimethylamino; Carboxy; Methoxycarbonyl; Ethoxycarbonyl und Cyano ausgewählt sind.

4. Zusammensetzung nach einem der vorhergehenden Ansprüche, **dadurch gekennzeichnet, dass** die Gruppen R₂ der Verbindungen der Formel (I) unter einem Wasserstoffatom; der Methylgruppe und der β-Hydroxyethylgruppe ausgewählt sind.

5. Zusammensetzung nach einem der vorhergehenden Ansprüche, **dadurch gekennzeichnet, dass** die Gruppen R₃ und R₄ der Verbindungen der Formel (I) unter Wasserstoff; Hydroxy; Amino; einer geradkettigen oder verzweigten C₁₋₄-Alkylgruppe; Trifluormethyl; Phenyl; einem Phenylring, der mit einer oder zwei Gruppen R substituiert ist, die unter Halogen, C₁₋₄-Alkyl, C₁₋₄-Alkoxy, Hydroxy, Carboxy, Nitro, C₁₋₄-Alkyltio, Methylendioxy, Amino, Trifluormethyl und C₁₋₄-Alkylamino ausgewählt sind; Benzyl; einer Benzylgruppe, die mit einem Halogenatom, Methyl, Isopropyl oder Methoxy substituiert ist; C₁₋₄-Monohydroxyalkyl; C₁₋₄-Aminoalkyl; C₁₋₄-Alkylamino-C₁₋₄-alkyl; einer Alkoxygruppe, die unter Methoxy, Ethoxy und Phenoxy ausgewählt ist; Methylthio; Ethylthio; Phenylthio; Methansulfonyl; einem aromatischen Ring, der substituiert oder unsubstituiert vorliegt und gemeinsam von den Gruppen R₃ und R₄ gebildet wird, wie Phenyl, Pyridyl oder eine Phenylgruppe, die mit Sulfonyl, einem Halogenatom, C₁₋₄-Alkoxy, C₁₋₄-Alkyl, Nitro, Cyano, Amino, C₁₋₄-Alkylamino oder Trifluormethyl substituiert ist, ausgewählt sind.

6. Zusammensetzung nach Anspruch 5, **dadurch gekennzeichnet, dass** die Gruppen R₃ und R₄ der Verbindungen der Formel (I) unter einem Wasserstoffatom; einer Alkylgruppe, die unter Methyl, Ethyl, Isopropyl, *n*-Propyl und *t*-Butyl ausgewählt ist; Phenyl; Toluyl; 2-, 3- oder 4-Chlorphenyl; 3- oder 4-Hydroxyphenyl; 3- oder 4-Aminophenyl; 3- oder 4-Methoxyphenyl; 4-Trifluormethylphenyl; Benzyl; Trifluormethyl; Hydroxymethyl; Hydroxyethyl; Hydroxyisopropyl; Aminomethyl oder Aminoethyl; Methoxy oder Ethoxy; Methylthio oder Ethylthio; einem aromatischen Ring, der substituiert oder unsubstituiert vorliegt und gemeinsam von den Gruppen R₃ und R₄ gebildet wird, wie Phenyl, Toluyl, Sulfonylphenyl und Chlorphenyl, ausgewählt sind.

7. Zusammensetzung nach einem der vorhergehenden Ansprüche, **dadurch gekennzeichnet, dass** die Verbindungen der Formel (I) ausgewählt sind unter:
i) Pyrazolo-[1,5-b]-1,2,4-triazolen der folgenden speziellen Formel (Ia) und deren Additionssalzen mit einer Säure: 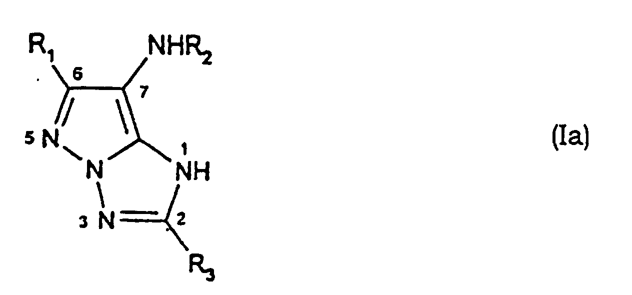
ü) den Pyrazolo-[3,2-c]-1,2,4-triazolen der folgenden speziellen Formel (Ib) und deren Additionssalzen mit einer Säure: 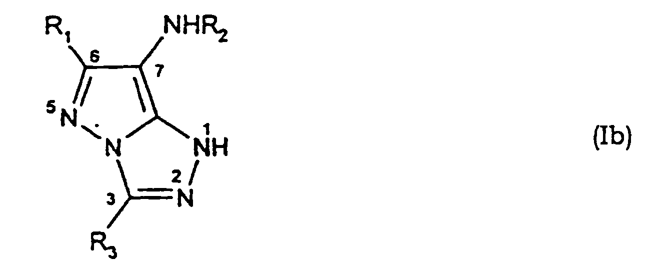
iii) die Pyrazolo-tetrazolen der folgenden speziellen Formel (Ic) und derer Additionssalzen mit einer Säure: 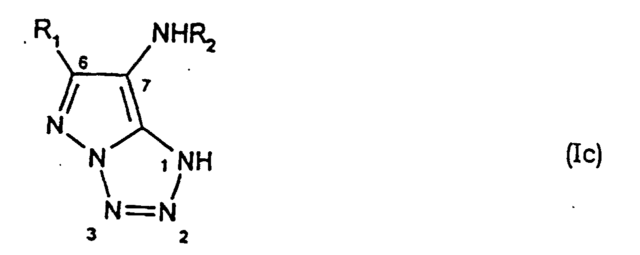
iv) den Pyrazolo-[1,5-a]-imidazolen der folgenden speziellen Formel (Id) und deren Additionssalzen mit einer Säure: 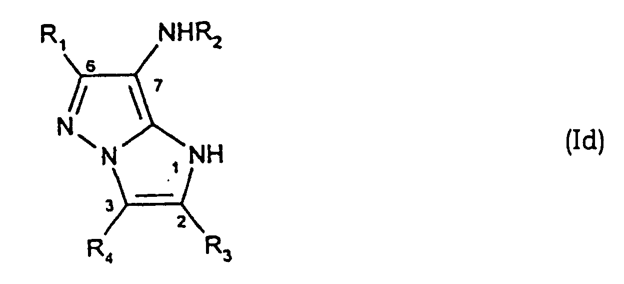
v) den Pyrazolo-[5,1-e]-pyrazolen der folgenden speziellen Formel (Ie) und deren Additionssalzen mit einer Säure: 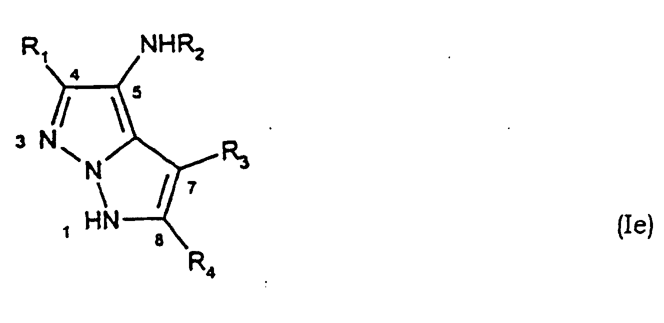
vi) den Pyrazolo-[5,1-e]-1,2,3-triazolen der folgenden speziellen Formel (If) und deren Additionssalzen mit einer Säure: 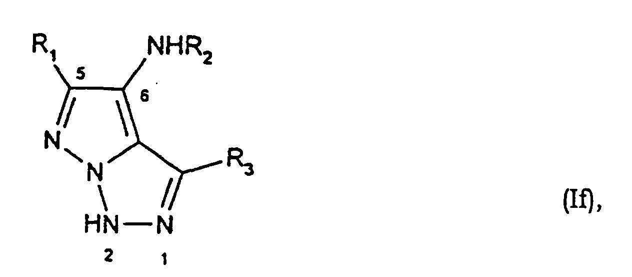
wobei in den Formeln R₁, R₂, R₃ und R₄ die in den Ansprüchen 1 bis 6 angegebenen Bedeutungen aufweisen.

8. Zusammensetzung nach Anspruch 7, **dadurch gekennzeichnet, dass** die Verbindungen der Formel (Ia) oder (Ib) unter den Verbindungen ausgewählt sind, worin:
- Ri ein Wasserstoffatom, Methyl, Trifluormethyl, Carboxy, Phenyl, Ethoxy, Cyano, Ethylthio, Amino oder Hydroxy bedeutet;
- R₂ ein Wasserstoffatom ist;
- R₃ ein Wasserstoffatom, Methyl, β-Aminoethyl, Ethyl, Isopropyl, Phenyl, β-Hydroxyethyl, Methylthio oder Ethoxy bedeutet.

9. Zusammensetzung nach Anspruch 7, **dadurch gekennzeichnet, dass** die Verbindungen der Formel (Ic) unter den Verbindungen ausgewählt sind, worin:
- R₁ ein Wasserstoffatom, Methyl, Trifluormethyl, Carboxy, Phenyl, Ethoxy oder Cyano bedeutet; und
- R₂ ein Wasserstoffatom ist.

10. Zusammensetzung nach Anspruch 7, **dadurch gekennzeichnet, dass** die Verbindungen der Formel (Id) unter den Verbindungen ausgewählt sind, worin:
- R₁ ein Wasserstoffatom, Methyl, Trifluormethyl, Carboxy, Phenyl, Ethoxy, Cyano, Amino, Ethylthio oder Hydroxy bedeutet;
- R₂ ein Wasserstoffatom bedeutet;
- R₃ und R₄ ein Wasserstoffatom und ein Wasserstoffatom, ein Wasserstoffatom und die Methylgruppe, die Methylgruppe und ein Wasserstoffatom, ein Wasserstoffatom und die Phenylgruppe; die Hydroxygruppe und ein Wasserstoffatom bedeuten; oder gemeinsam einen Phenylring bilden.

11. Zusammensetzung nach Anspruch 7, **dadurch gekennzeichnet, dass** die Verbindungen der Formel (Ie) unter den Verbindungen ausgewählt sind, worin bedeuten:
- R₁ Wasserstoff, Methyl, Trifluormethyl, Carboxy, Phenyl, Ethoxy oder Cyano;
- R₂ Wasserstoff;
- R₃ und R₄ Wasserstoff und Methyl, Methyl und Wasserstoff, Methyl und Methyl, Wasserstoff und Phenyl, Wasserstoff und Amino.

12. Zusammensetzung nach Anspruch 7, **dadurch gekennzeichnet, dass** die Verbindungen der Formel (If) unter den Verbindungen ausgewählt sind, worin bedeuten:
- R₁ Wasserstoff, Methyl, Trifluormethyl, Carboxy, Phenyl, Ethoxy oder Cyano;
- R₂ Wasserstoff;
- R₃ Wasserstoff oder Methyl.

13. Zusammensetzung nach Anspruch 7, **dadurch gekennzeichnet, dass** die Verbindungen der Formel (I) ausgewählt sind unter:
- 7-Amino-2-methyl-pyrazolo-[1,5-b]-1,2,4-triazol;
- 7-Amino-2-ethyl-pyrazolo-[1,5-b]-1,2,4-triazol;
- 7-Amino-2-isopropyl-pyrazolo-[1,5-b]-1,2,4-triazol;
- 7-Amino-2-phenyl-pyrazolo-[1,5-b]-1,2,4-triazol;
- 7-Amino-2,6-dimethyl-pyrazolo-[1,5-b]-1,2,4-triazol;
- 7-Amino-6-methyl-2-ethyl-pyrazolo-[1,5-b]-1,2,4-triazol;
- 7-Amino-6-methyl-2-isopropyl-pyrazolo-[1,5-b]-1,2,4-triazol;
- 7-Amino-6-methyl-2-phenyl-pyrazolo-[1,5-b]-1,2,4-triazol;
- 7-Amino-6-carboxy-2-methyl-pyrazolo-[1,5-b]-1,2,4-triazol;
- 7-Amino-6-carboxy-2-ethyl-pyrazolo-[1,5-b]-1,2,4-triazol;
- 7-Amino-6-carboxy-2-isopropyl-pyrazolo-[1,5-b]-1,2,4-triazol;
- 7-Amino-6-carboxy-2-phenyl-pyrazolo-[1,5-b]-1,2,4-triazol;
- 7-Amino-6-phenyl-2-methyl-pyrazolo-[1,5-b]-1,2,4-triazol;
- 7-Amino-6-phenyl-2-ethyl-pyrazolo-[1,5-b]-1,2,4-triazol;
- 7-Amino-6-phenyl-2-isopropyl-pyrazolo-[1,5-b]-1,2,4-triazol;
- 6,7-Diamino-2-methyl-pyrazolo-[1,5-b]-1,2,4-triazol;
- 6,7-Diamino-2-ethyl-pyrazolo-[1,5-b]-1,2,4-triazol;
- 6,7-Diamino-2-isopropyl-pyrazolo-[1,5-b]-1,2,4-triazol;
- 6,7-Diamino-2-phenyl-pyrazolo-[1,5-b]-1,2,4-triazol;
- 7-Amino-6-ethylthio-2-methyl-pyrazolo-[1,5-b]-1,2,4-triazol;
- 7-Amino-6-ethylthio-2-ethyl-pyrazolo-[1,5-b]-1,2,4-triazol;
- 7-Amino-5-ethylthio-2-isopropyl-pyrazolo-[1,5-b]-1,2,4-triazol;
- 7-Amino-6-ethylthio-2-phenyl-pyrazolo-[1,5-b]-1,2,4-triazol;
- 7-Amino-6-ethoxy-2-methyl-pyrazolo-[1,5-b]-1,2,4-triazol;
- 7-Amino-6-ethoxy-2-ethyl-pyrazolo-[1,5-b]-1,2,4-triazol;
- 7-Amino-6-ethoxy-2-isopropyl-pyrazolo-[1,5-b]-1,2,4-triazol;
- 7-Amino-6-ethoxy-2-phenyl-pyrazolo-[1,5-b]-1,2,4-triazol;
- 7-Amino-6-methyl-2-(2'-aminoethyl)-pyrazolo-[1,5-b]-1,2,4-triazol;
- 7-Amino-6-carboxy-2-(2'-aminoethyl)-pyrazolo-[1,5-b]-1,2,4-triazol;
- 7-Amino-6-phenyl-2-(2'-aminoethyl)-pyrazolo-[1,5-b]-1,2,4-triazol;
- 7-Amino-6-ethylthio-2-(2'-aminoethyl)-pyrazolo-[1,5-b]-1,2,4-triazol;
- 7-Amino-2-(2'-aminoethyl)-pyrazolo-[1,5-b]-1,2,4-triazol;
- 7-Amino-2-(2'-hydroxyethyl)-pyrazolo-[1,5-b]-1,2,4-triazol;
- 7-Amino-6-methyl-2-(2'-hydroxyethyl)-pyrazolo-[1,5-b]-1,2,4-triazol;
- 7-Amino-6-ethylthio-2-(2'-hydroxyethyl)-pyrazolo-[1,5-b]-1,2,4-triazol;
- 7-Amino-6-carboxy-2-(2'-hydroxyethyl)-pyrazolo-[1,5-b]-1,2,4-triazol;
- 7-Amino-6-phenyl-2-(2'-hydroxyethyl)-pyrazolo-[1,5-b]-1,2,4-triazol;
- 6,7-Diamino-2-(2'-aminoethyl)-pyrazolo-[1,5-b]-1,2,4-triazol;
- 6,7-Diamino-2-(2'-hydroxyethyl)-pyrazolo-[1,5-b]-1,2,4-triazol;
- 7-Amino-6-ethoxy-2-(2'-aminoethyl)-pyrazolo-[1,5-b]-1,2,4-triazol;
- 7-Amino-6-ethoxy-2-(2'-hydroxyethyl)-pyrazolo-[1,5-b]-1,2,4-triazol;
- 7-Amino-3-methyl-pyrazolo-[3,2-c]-1,2,4-triazol;
- 7-Amino-3-ethyl-pyrazolo-[3,2-c]-1,2,4-triazol;
- 7-Amino-3-isopropyl-pyrazolo-[3,2-c]-1,2,4-triazol;
- 7-Amino-3-phenyl-pyrazolo-[3,2-c]-1,2,4-triazol;
- 7-Amino-3-(2'-aminoethyl)-pyrazolo-[3,2-c]-1,2,4-triazol;
- 7-Amino-3-(2'-hydroxyethyl)-pyrazolo-[3,2-c]-1,2,4-triazol;
- 7-Amino-6-methyl-3-ethyl-pyrazolo-[3,2-c]-1,2,4-triazol;
- 7-Amino-3,6-dimethyl-pyrazolo-[3,2-c]-1,2,4-triazol;
- 7-Amino-6-methyl-3-isopropyl-pyrazolo-[3,2-c]-1,2,4-triazol;
- 7-Amino-6-methyl-3-phenyl-pyrazolo-[3,2-c]-1,2,4-triazol;
- 7-Amino-6-methyl-3-(2'-aminoethyl)-pyrazolo-[3,2-c]-1,2,4-triazol;
- 7-Amino-6-methyl-3-(2'-hydroxyethyl)-pyrazolo-[3,2-c]-1,2,4-triazol;
- 7-Amino-6-methyl-3-methylthio-pyrazolo-[3,2-c]-1,2,4-triazol;
- 7-Amino-6-phenyl-3-methyl-pyrazolo-[3,2-c]-1,2,4-triazol;
- 7-Amino-6-phenyl-3-ethyl-pyrazolo-[3,2-c]-1,2,4-triazol;
- 7-Amino-6-phenyl-3-isopropyl-pyrazolo-[3,2-c]-1,2,4-triazol;
- 7-Amino-6-phenyl-3-phenyl-pyrazolo-[3,2-c]-1,2,4-triazol;
- 7-Amino-6-phenyl-3-(2'-aminoethyl)-pyrazolo-[3,2-c]-1,2,4-triazol;
- 7-Amino-6-phenyl-3-(2'-hydroxyethyl)-pyrazolo-[3,2-c]-1,2,4-triazol;
- 7-Amino-6-phenyl-3-methylthio-pyrazolo-[3,2-c]-1,2,4-triazol;
- 7-Amino-6-ethylthio-3-methyl-pyrazolo-[3,2-c]-1,2,4-triazol;
- 7-Amino-6-ethylthio-3-ethyl-pyrazolo-[3,2-c]-1,2,4-triazol;
- 7-Amino-6-ethylthio-3-isopropyl-pyrazolo-[3,2-c]-1,2,4-triazol;
- 7-Amino-6-ethylthio-3-phenyl-pyrazolo-[3,2-c]-1,2,4-triazol;
- 7-Amino-6-ethylthio-3-(2'-aminoethyl)-pyrazolo-[3,2-c]-1,2,4-triazol;
- 7-Amino-6-ethylthio-3-(2'-hydroxyethyl)-pyrazolo-[3,2-c]-1,2,4-triazol;
- 7-Amino-6-trifluormethyl-3-methylthio-pyrazolo-[3,2-c]-1,2,4-triazol;
- 7-Amino-6-trifluormethyl-pyrazolo-[3,2-c]-1,2,4-triazol;
- 7-Amino-6-carboxy-3-methyl-pyrazolo-[3,2-c]-1,2,4-triazol;
- 7-Amino-6-carboxy-3-ethyl-pyrazolo-[3,2-c]-1,2,4-triazol;
- 7-Amino-6-carboxy-3-isopropyl-pyrazolo-[3,2-c]-1,2,4-triazol;
- 7-Amino-6-carboxy-3-phenyl-pyrazolo-[3,2-c]-1,2,4-triazol;
- 7-Amino-6-carboxy-3-(2'-aminoethyl)-pyrazolo-[3,2-c]-1,2,4-triazol;
- 7-Amino-6-carboxy-3-(2'-hydroxyethyl)-pyrazolo-[3,2-c]-1,2,4-triazol;
- 6,7-Diamino-3-methyl-pyrazolo-[3,2-c]-1,2,4-triazol;
- 6,7-Diamino-3-ethyl-pyrazolo-[3,2-c]-1,2,4-triazol;
- 6,7-Diamino-3-phenyl-pyrazolo-[3,2-c]-1,2,4-triazol;
- 6,7-Diamino-3-isopropyl-pyrazolo-[3,2-c]-1,2,4-triazol;
- 7-Amino-6-ethoxy-3-methyl-pyrazolo-[3,2-c]-1,2,4-triazol;
- 7-Amino-6-ethoxy-3-ethyl-pyrazolo-[3,2-c]-1,2,4-triazol;
- 7-Amino-6-ethoxy-3-isopropyl-pyrazolo-[3,2-c]-1,2,4-triazol;
- 7-Amino-6-ethoxy-3-phenyl-pyrazolo-[3,2-c]-1,2,4-triazol;
- 7-Amino-6-ethoxy-3-(2'-aminoethyl)-pyrazolo-[3,2-c]-1,2,4-triazol;
- 7-Amino-6-ethoxy-3-(2'-hydroxyethyl)-pyrazolo-[3,2-c]-1,2,4-triazol;
- 6,7-Diamino-3-(2-aminoethyl)-pyrazolo-[3,2-c]-1,2,4-triazol;
- 6,7-Diamino-3-(2'-hydroxyethyl)-pyrazolo-[3,2-c]-1,2,4-triazol;
- 7-Amino-6-methyl-pyrazolo-[5,1-e]-tetrazol;
- 7-Amino-6-phenyl-pyrazolo-[5,1-e]-tetrazol;
- 7-Amino-6-carboxy-pyrazolo-[5,1-e]-tetrazol;
- 7-Amino-2-methyl-pyrazolo-[1,5-a]-imidazol;
- 7-Amino-2-phenyl-pyrazolo-[1,5-a]-imidazol;
- 7-Amino-pyrazolo-[1,5-a]-benzimidazol;
- 7-Amino-6-methyl-pyrazolo-[1,5-a]-imidazol;
- 7-Amino-2,6-dimethyl-pyrazolo-[1,5-a]-imidazol;
- 7-Amino-6-methyl-2-phenyl-pyrazolo-[1,5-a]-imidazol;
- 7-Amino-6-methyl-pyrazolo-[1,5-a]-benzimidazol;
- 7-Amino-6-phenyl-pyrazolo-[1,5-a]-imidazol;
- 7-Amino-6-phenyl-2-methyl-pyrazolo-[1,5-a]-imidazol;
- 7-Amino-2,6-diphenyl-pyrazolo-[1,5-a]-imidazol;
- 7-Amino-6-phenyl-pyrazolo-[1,5-a]-benzimidazol;
- 7-Amino-6-carboxy-pyrazolo-[1,5-a]-imidazol;
- 7-Amino-6-carboxy-2-methyl-pyrazolo-[1,5-a]-imidazol;
- 7-Amino-6-carboxy-2-phenyl-pyrazolo-[1,5-a]-imidazol;
- 7-Amino-6-carboxy-pyrazolo-[1,5-a]-benzimidazol;
- 7-Amino-6-ethoxy-pyrazolo-[1,5-a]-imidazol;
- 7-Amino-6-ethoxy-2-methyl-pyrazolo-[1,5-a]-imidazol;
- 7-Amino-6-ethoxy-2-phenyl-pyrazolo-[1,5-a]-imidazol;
- 7-Amino-6-ethoxy-pyrazolo-[1,5-a]-benzimidazol;
- 7-Amino-6-trifluormethyl-pyrazolo-[1,5-a]-benzimidazol;
- 6,7-Diamino-pyrazolo-[1,5-a]-imidazol;
- 6,7-Diamino-2-methyl-pyrazolo-[1,5-a]-imidazol;
- 6,7-Diamino-2-phenyl-pyrazolo-[1,5-a]-imidazol;
- 6,7-Diamino-pyrazolo-[1,5-a]-benzimidazol;
- 7-Amino-6-ethylthio-pyrazolo-[1,5-a]-imidazol;
- 7-Amino-6-ethylthio-2-methyl-pyrazolo-[1,5-a]-imidazol;
- 7-Amino-6-ethylthio-2-phenyl-pyrazolo-[1,5-a]-imidazol;
- 7-Amino-6-methyl-2-hydroxy-pyrazolo-[1,5-a]-imidazol;
- 7-Amino-6-phenyl-2-hydroxy-pyrazolo-[1,5-a]-imidazol;
- 7-Amino-6-ethoxy-2-hydroxy-pyrazolo-[1,5-a]-imidazol;
- 7-Amino-6-ethylthio-2-hydroxy-pyrazolo-[1,5-a]-imidazol;
- 6,7-Diamino-2-hydroxy-pyrazolo-[1,5-a]-imidazol;
- 5,8-Diamino-4-methyl-pyrazolo-[1,5-e]-pyrazol;
- 5,7,8-Triamino-4-methyl-pyrazolo-[5,1-e]-pyrazol;
- 6-Amino-5-methyl-pyrazolo-[5,1-e]-1,2,3-triazol;
- 6-Amino-5-phenyl-pyrazolo-[5,1-e]-1,2,3-triazol;
und deren Additionssalzen mit einer Säure.

14. Zusammensetzung nach einem der vorhergehenden Ansprüche, **dadurch gekennzeichnet, dass** die Verbindung(en) der Formel (I) 0,0005 bis 12 Gew.-% des Gesamtgewichts der Farbmittelzusammensetzung ausmachen.

15. Zusammensetzung nach Anspruch 14, **dadurch gekennzeichnet, dass** die Verbindung(en) der Formel (I) 0,005 bis 6 Gew.-% des Gesamtgewichts der Farbmittelzusammensetzung ausmachen.

16. Zusammensetzung nach einem der vorhergehenden Ansprüche, **dadurch gekennzeichnet, dass** das zum Färben geeignete Medium (oder der Träger) aus Wasser oder einem Gemisch von Wasser und mindestens einem organischen Lösungsmittel besteht.

17. Zusammensetzung nach einem der vorhergehenden Ansprüche, **dadurch gekennzeichnet, dass** sie einen pH-Wert im Bereich von 3 bis 13 und vorzugsweise 5 bis 11 aufweist.

18. Zusammensetzung nach einem der vorhergehenden Ansprüche, **dadurch gekennzeichnet, dass** sie einen oder mehrere Kuppler enthält, die unter den m-Phenylendiaminen, m-Aminophenolen, m-Dihydroxybenzolen und den heterocyclischen Kupplern ausgewählt sind.

19. Zusammensetzung nach Anspruch 18, **dadurch gekennzeichnet, dass** die Kuppler unter 2-Methyl-5-aminophenol, 5-N-(β-hydroxyethyl)amino-2-methylphenol, 3-Aminophenol, 1,3-Dihydroxybenzol, 1,3-Dihydroxy-2-methylbenzol, 4-Chlor-1,3-dihydroxybenzol, 2,4-Diamino-1-(β-hydroxyethyloxy)-benzol, 2-Amino-4-(β-hydroxyethyl)-amino-1-methoxybenzol, 1,3-Diaminobenzol, 1,3-Bis-(2,4-diaminophenoxy)-propan, Sesamol, α-Naphthol, 6-Hydroxyindol, 4-Hydroxyindol, 4-Hydroxy-N-methylindol, 6-Hydroxyindolin, 2,6-Dihydroxy-4-methylpyridin, 1H-3-Methylpyrazol-5-on, 1-Phenyl-3-methylpyrazol-5-on, 2,6-Dimethyl-pyrazolo-[1,5-b]-1,2,4-triazol, 2,6-Dimethyl-[3,2-c]-1,2,4-triazol, 6-Methyl-pyrazolo-[1,5-a]-benzimidazol und ihren Additionssalzen mit einer Säure ausgewählt sind.

20. Zusammensetzung nach einem der Ansprüche 18 oder 19, **dadurch gekennzeichnet, dass** der oder die Kuppler 0,0001 bis 10 Gew.-% des Gesamtgewichts der Farbmittelzusammensetzung ausmachen.

21. Zusammensetzung nach einem der vorhergehenden Ansprüche, **dadurch gekennzeichnet, dass** sie mindestens eine zusätzliche Oxidationsbase enthält, die unter den p-Phenylendiaminen, Bisphenylalkylendiaminen, p-Aminophenolen, o-Aminophenolen und den heterocyclischen Basen, die von den Pyrazolo-azolen der Formel (I) verschieden sind, ausgewählt sind.

22. Zusammensetzung nach einem der vorhergehenden Ansprüche, **dadurch gekennzeichnet, dass** die Additionssalze mit einer Säure unter den Hydrochloriden, Hydrobromiden, Sulfaten, Citraten, Succinaten, Tartraten, Tosylaten, Benzolsulfonaten, Lactaten und Acetaten ausgewählt sind.

23. Verwendung von Verbindungen der Formel (I) nach einem der Ansprüche 1 bis 13 und 22 als Oxidationsbasen in Zusammensetzungen zum oxidativen Färben von Keratinfasern und insbesondere menschlichen Keratinfasern, wie dem Haar.

24. Verfahren zum Färben von Keratinfasern und insbesondere menschlichen Keratinfasern, wie dem Haar, **dadurch gekennzeichnet, dass** auf die Fasern mindestens eine Farbmittelzusammensetzung nach einem der Ansprüche 1 bis 22 während einer Zeitspanne, die für die Entwicklung der gewünschten Färbung entweder mit Luft oder mithilfe eines Oxidationsmittels ausreichend ist, aufgebracht wird.

25. Verfahren zum Färben nach Anspruch 24, **dadurch gekennzeichnet, dass** die Färbung der Fasern ohne Zusatz eines Oxidationsmittels nur in Kontakt mit Luftsauerstoff erfolgt.

26. Verfahren zum Färben nach Anspruch 24, **dadurch gekennzeichnet, dass** die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung enthalten ist, die gleichzeitig oder getrennt davon anschließend aufgetragen wird.

27. Verfahren nach Anspruch 26, **dadurch gekennzeichnet, dass** das Oxidationsmittel unter Wasserstoffperoxid, Harnstoffperoxid, Alkalimetallbromaten, Salzen von Persäuren, wie Perboraten, und Persulfaten und Enzymen, wie Peroxidasen und Oxidoreductasen, die zwei Elektronen übertragen, ausgewählt ist.

28. Vorrichtung in mehreren Abteilungen oder "Kit" zum Färben mit mehreren Abteilungen, wobei eine erste Abteilung eine Farbmittelzusammensetzung nach einem der Ansprüche 1 bis 22 enthält und eine zweite Abteilung eine oxidierende Zusammensetzung enthält.

29. Verbindungen der Formel (I) sowie deren Additionssalze mit einer Säure der folgenden Formeln (I'a) bis (I'f): 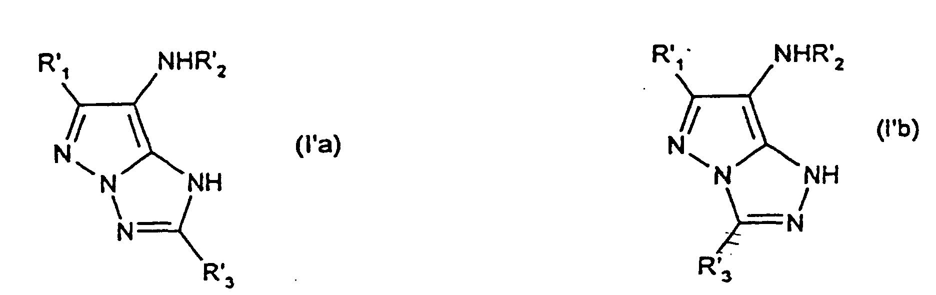 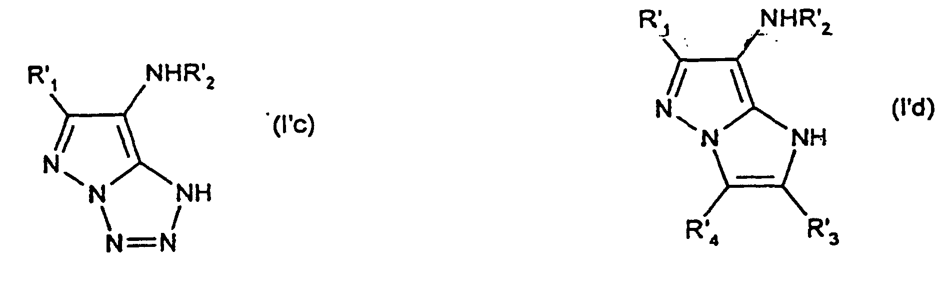 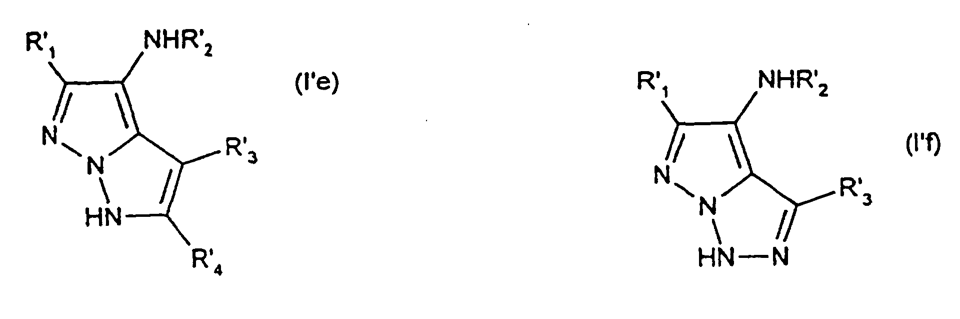 worin die Gruppen R'₁, R'₂, R'₃ und R'₄ die in einem der Ansprüche 1 bis 12 für die Gruppen R₁, R₂, R₃ und R₄ der Formel (I) angegebenen Bedeutungen aufweisen, mit der Maßgabe, dass:
- in den Verbindungen der Formel (I'b):
i) wenn R'₁ Methyl und R'₂ Wasserstoff bedeutet, die Gruppe R'₃ von Methyl oder (CH₂)₃-SO₂-(CH₂)₁₅-CH₃ oder CH(CH₃)-CH₂-SO₂-(CH₂)₁₁CH₃ verschieden ist;
ü) wenn R'₁ *t*-Butyl und R'₂ Wasserstoff bedeutet, die Gruppe R'₃ von (CH₂)₃-SO₂-(CH₂)₁₁-CH₃ verschieden ist;
- in den Verbindungen der Formel (I'd):
- wenn R'₃ und R'₄ gemeinsam einen Benzolring bilden und R'₂ Wasserstoff bedeutet, die Gruppe R'₁ von Methyl, -CONH₂ oder Carboxy verschieden ist;
- wenn R'₃ und R'₄ gemeinsam einen Benzolring bilden, der in 4-Stellung mit einer Sulfonylgruppe substituiert ist, und R'₂ Wasserstoff bedeutet, die Gruppe R'₁ von Phenyl, -(CH₂)₁₆-CH₃ oder -COOH verschieden ist;
- wenn R'₃ und R'₄ gemeinsam einen Benzolring bilden, der in 4-Stellung mit einer Carboxygruppe substituiert ist, und R'₂ Wasserstoff bedeutet, die Gruppe R'₁ von Carboxy verschieden ist.

30. Verbindungen der Formel (I') nach Anspruch 29, **dadurch gekennzeichnet, dass** sie ausgewählt sind unter:
- 7-Amino-2-methyl-pyrazolo-[1,5-b]-1,2,4-triazol;
- 7-Amino-2-ethyl-pyrazolo-[1,5-b]-1,2,4-triazol;
- 7-Amino-2-isopropyl-pyrazolo-[1,5-b]-1,2,4-triazol;
- 7-Amino-2-phenyl-pyrazolo-[1,5-b]-1,2,4-triazol;
- 7-Amino-2,6-dimethyl-pyrazolo-[1,5-b]-1,2,4-triazol;
- 7-Amino-6-methyl-2-ethyl-pyrazolo-[1,5-b]-1,2,4-triazol;
- 7-Amino-6-methyl-2-isopropyl-pyrazolo-[1,5-b]-1,2,4-triazol;
- 7-Amino-6-methyl-2-phenyl-pyrazolo-[1,5-b]-1,2,4-triazol;
- 7-Amino-6-carboxy-2-methyl-pyrazolo-[1,5-b]-1,2,4-triazol;
- 7-Amino-6-carboxy-2-ethyl-pyrazolo-[1,5-b]-1,2,4-triazol;
- 7-Amino-6-carboxy-2-isopropyl-pyrazolo-[1,5-b]-1,2,4-triazol;
- 7-Amino-6-carboxy-2-phenyl-pyrazolo-[1,5-b]-1,2,4-triazol;
- 7-Amino-6-phenyl-2-methyl-pyrazolo-[1,5-b]-1,2,4-triazol;
- 7-Amino-6-phenyl-2-ethyl-pyrazolo-[1,5-b]-1,2,4-triazol;
- 7-Amino-6-phenyl-2-isopropyl-pyrazolo-[1,5-b]-1,2,4-triazol;
- 6,7-Diamino-2-methyl-pyrazolo-[1,5-b]-1,2,4-triazol;
- 6,7-Diamino-2-ethyl-pyrazolo-[1,5-b]-1,2,4-triazol;
- 6,7-Diamino-2-isopropyl-pyrazolo-[1,5-b]-1,2,4-triazol;
- 6,7-Diamino-2-phenyl-pyrazolo-[1,5-b]-1,2,4-triazol;
- 7-Amino-6-ethylthio-2-methyl-pyrazolo-[1,5-b]-1,2,4-triazol;
- 7-Amino-6-ethylthio-2-ethyl-pyrazolo-[1,5-b]-1,2,4-triazol;
- 7-Amino-6-ethylthio-2-isopropyl-pyrazolo-[1,5-b]-1,2,4-triazol;
- 7-Amino-6-ethylthio-2-phenyl-pyrazolo-[1,5-b]-1,2,4-triazol;
- 7-Amino-6-ethoxy-2-methyl-pyrazolo-[1,5-b]-1,2,4-triazol;
- 7-Amino-6-ethoxy-2-ethyl-pyrazolo-[1,5-b]-1,2,4-triazol;
- 7-Amino-6-ethoxy-2-isopropyl-pyrazolo-[1,5-b]-1,2,4-triazol;
- 7-Amino-6-ethoxy-2-phenyl-pyrazolo-[1,5-b]-1,2,4-triazol;
- 7-Amino-6-methyl-2-(2'-aminoethyl)-pyrazolo-[1,5-b]-1,2,4-triazol;
- 7-Amino-6-carboxy-2-(2'-aminoethyl)-pyrazolo-[1,5-b]-1,2,4-triazol;
- 7-Amino-6-phenyl-2-(2'-aminoethyl)-pyrazolo-[1,5-b]-1,2,4-triazol;
- 7-Amino-6-ethylthio-2-(2'-aminoethyl)-pyrazolo-[1,5-b]-1,2,4-triazol;
- 7-Amino-2-(2'-aminoethyl)-pyrazolo-[1,5-b]-1,2,4-triazol;
- 7-Amino-2-(2'-hydroxyethyl)-pyrazolo-[1,5-b]-1,2,4-triazol;
- 7-Amino-6-methyl-2-(2'-hydroxyethyl)-pyrazolo-[1,5-b]-1,2,4-triazol;
- 7-Amino-6-ethylthio-2-(2'-hydroxyethyl)-pyrazolo-[1,5-b]-1,2,4-triazol;
- 7-Amino-6-carboxy-2-(2'-hydroxyethyl)-pyrazolo-[1,5-b]-1,2,4-triazol;
- 7-Amino-6-phenyl-2-(2'-hydroxyethyl)-pyrazolo-[1,5-b]-1,2,4-triazol;
- 6,7-Diamino-2-(2'-aminoethyl)-pyrazolo-[1,5-b]-1,2,4-triazol;
- 6,7-Diamino-2-(2'-hydroxyethyl)-pyrazolo-[1,5-b]-1,2,4-triazol;
- 7-Amino-6-ethoxy-2-(2'-aminoethyl)-pyrazolo-[1,5-b]-1,2,4-triazol;
- 7-Amino-6-ethoxy-2-(2'-hydroxyethyl)-pyrazolo-[1,5-b]-1,2,4-triazol;
- 7-Amino-3-methyl-pyrazolo-[3,2-c]-1,2,4-triazol;
- 7-Amino-3-ethyl-pyrazolo-[3,2-c]-1,2,4-triazol;
- 7-Amino-3-isopropyl-pyrazolo-[3,2-c]-1,2,4-triazol;
- 7-Amino-3-phenyl-pyrazolo-[3,2-c]-1,2,4-triazol;
- 7-Amino-3-(2'-aminoethyl)-pyrazolo-[3,2-c]-1,2,4-triazol;
- 7-Amino-3-(2'-hydroxyethyl)-pyrazolo-[3,2-c]-1,2,4-triazol;
- 7-Amino-6-methyl-3-ethyl-pyrazolo-[3,2-c]-1,2,4-triazol;
- 7-Amino-3,6-dimethyl-pyrazolo-[3,2-c]-1,2,4-triazol;
- 7-Amino-6-methyl-3-isopropyl-pyrazolo-[3,2-c]-1,2,4-triazol;
- 7-Amino-6-methyl-3-phenyl-pyrazolo-[3,2-c]-1,2,4-triazol;
- 7-Amino-6-methyl-3-(2'-aminoethyl)-pyrazolo-[3,2-c]-1,2,4-triazol;
- 7-Amino-6-methyl-3-(2'-hydroxyethyl)-pyrazolo-[3,2-c]-1,2,4-triazol;
- 7-Amino-6-methyl-3-methylthio-pyrazolo-[3,2-c]-1,2,4-triazol;
- 7-Amino-6-phenyl-3-methyl-pyrazolo-[3,2-c]-1,2,4-triazol;
- 7-Amino-6-phenyl-3-ethyl-pyrazolo-[3,2-c]-1,2,4-triazol;
- 7-Amino-6-phenyl-3-isopropyl-pyrazolo-[3,2-c]-1,2,4-triazol;
- 7-Amino-6-phenyl-3-phenyl-pyrazolo-[3,2-c]-1,2,4-triazol;
- 7-Amino-6-phenyl-3-(2'-aminoethyl)-pyrazolo-[3,2-c]-1,2,4-triazol;
- 7-Amino-6-phenyl-3-(2'-hydroxyethyl)-pyrazolo-[3,2-c]-1,2,4-triazol;
- 7-Amino-6-phenyl-3-methylthio-pyrazolo-[3,2-c]-1,2,4-triazol;
- 7-Amino-6-ethylthio-3-methyl-pyrazolo-[3,2-c]-1,2,4-triazol;
- 7-Amino-6-ethylthio-3-ethyl-pyrazolo-[3,2-c]-1,2,4-triazol;
- 7-Amino-6-ethylthio-3-isopropyl-pyrazolo-[3,2-c]-1,2,4-triazol;
- 7-Amino-6-ethylthio-3-phenyl-pyrazolo-[3,2-c]-1,2,4-triazol;
- 7-Amino-6-ethylthio-3-(2'-aminoethyl)-pyrazolo-[3,2-c]-1,2,4-triazol;
- 7-Amino-6-ethylthio-3-(2'-hydroxyethyl)-pyrazolo-[3,2-c]-1,2,4-triazol;
- 7-Amino-6-trifluormethyl-3-methylthio-pyrazolo-[3,2-c]-1,2,4-triazol;
- 7-Amino-6-trifluormethyl-pyrazolo-[3,2-c]-1,2,4-triazol;
- 7-Amino-6-carboxy-3-methyl-pyrazolo-[3,2-c]-1,2,4-triazol;
- 7-Amino-6-carboxy-3-ethyl-pyrazolo-[3,2-c]-1,2,4-triazol;
- 7-Amino-6-carboxy-3-isopropyl-pyrazolo-[3,2-c]-1,2,4-triazol;
- 7-Amino-6-carboxy-3-phenyl-pyrazolo-[3,2-c]-1,2,4-triazol;
- 7-Amino-6-carboxy-3-(2'-aminoethyl)-pyrazolo-[3,2-c]-1,2,4-triazol;
- 7-Amino-6-carboxy-3-(2'-hydroxyethyl)-pyrazolo-[3,2-c]-1,2,4-triazol;
- 6,7-Diamino-3-methyl-pyrazolo-[3,2-c]-1,2,4-triazol;
- 6,7-Diamino-3-ethyl-pyrazolo-[3,2-c]-1,2,4-triazol;
- 6,7-Diamino-3-phenyl-pyrazolo-[3,2-c]-1,2,4-triazol;
- 6,7-Diamino-3-isopropyl-pyrazolo-[3,2-c]-1,2,4-triazol;
- 7-Amino-6-ethoxy-3-methyl-pyrazolo-[3,2-c]-1,2,4-triazol;
- 7-Amino-6-ethoxy-3-ethyl-pyrazolo-[3,2-c]-1,2,4-triazol;
- 7-Amino-6-ethoxy-3-isopropyl-pyrazolo-[3,2-c]-1,2,4-triazol;
- 7-Amino-6-ethoxy-3-phenyl-pyrazolo-[3,2-c]-1,2,4-triazol;
- 7-Amino-6-ethoxy-3-(2'-aminoethyl)-pyrazolo-[3,2-c]-1,2,4-triazol;
- 7-Amino-6-ethoxy-3-(2'-hydroxyethyl)-pyrazolo-[3,2-c]-1,2,4-triazol;
- 6,7-Diamino-3-(2'-aminoethyl)-pyrazolo-[3,2-c]-1,2,4-triazol;
- 6,7-Diamino-3-(2'-hydroxyethyl)-pyrazolo-[3,2-c]-1,2,4-triazol;
- 7-Amino-6-methyl-pyrazolo-[5,1-e]-tetrazol;
- 7-Amino-6-phenyl-pyrazolo-[5,1-e]-tetrazol;
- 7-Amino-6-carboxy-pyrazolo-[5,1-e]-tetrazol;
- 7-Amino-pyrazolo-[1,5-a]-imidazol;
- 7-Amino-2-methyl-pyrazolo-[1,5-a]-imidazol;
- 7-Amino-2-phenyl-pyrazolo-[1,5-a]-imidazol;
- 7-Amino-pyrazolo-[1,5-a]-benzimidazol;
- 7-Amino-6-methyl-pyrazolo-[1,5-a]-imidazol;
- 7-Amino-2,6-dimethyl-pyrazolo-[1,5-a]-imidazol;
- 7-Amino-6-methyl-2-phenyl-pyrazolo-[1,5-a]-imidazol;
- 7-Amino-6-phenyl-pyrazolo-[1,5-a]-imidazol;
- 7-Amino-6-phenyl-2-methyl-pyrazolo-[1,5-a]-imidazol;
- 7-Amino-2,6-diphenyl-pyrazolo-[1,5-a]-imidazol;
- 7-Amino-6-phenyl-pyrazolo-[1,5-a]-benzimidazol;
- 7-Amino-6-carboxy-pyrazolo-[1,5-a]-imidazol;
- 7-Amino-6-carboxy-2-methyl-pyrazolo-[1,5-a]-imidazol;
- 7-Amino-6-carboxy-2-phenyl-pyrazolo-[1,5-a]-imidazol;
- 7-Amino-6-ethoxy-pyrazolo-[1,5-a]-imidazol;
- 7-Amino-6-ethoxy-2-methyl-pyrazolo-[1,5-a]-imidazol;
- 7-Amino-6-ethoxy-2-phenyl-pyrazolo-[1,5-a]-imidazol;
- 7-Amino-6-ethoxy-pyrazolo-[1,5-a]-benzimidazol;
- 7-Amino-6-trifluormethyl-pyrazolo-[1,5-a]-benzimidazol;
- 6,7-Diamino-pyrazolo-[1,5-a]-imidazol;
- 6,7-Diamino-2-methyl-pyrazolo-[1,5-a]-imidazol;
- 6,7-Diamino-2-phenyl-pyrazolo-[1,5-a]-imidazol;
- 6,7-Diamino-pyrazolo-[1,5-a]-benzimidazol;
- 7-Amino-6-ethylthio-pyrazolo-[1,5-a]-imidazol;
- 7-Amino-6-ethylthio-2-methyl-pyrazolo-[1,5-a]-imidazol;
- 7-Amino-6-ethylthio-2-phenyl-pyrazolo-[1,5-a]-imidazol;
- 7-Amino-6-methyl-2-hydroxy-pyrazolo-[1,5-a]-imidazol;
- 7-Amino-6-phenyl-2-hydroxy-pyrazolo-[1,5-a]-imidazol;
- 7-Amino-6-ethoxy-2-hydroxy-pyrazolo-[1,5-a]-imidazol;
- 7-Amino-6-ethylthio-2-hydroxy-pyrazolo-[1,5-a]-imidazol;
- 6,7-Diamino-2-hydroxy-pyrazolo-[1,5-a]-imidazol;
- 5,8-Diamino-4-methyl-pyrazolo-[1,5-e]-pyrazol;
- 5,7,8-Triamino-4-methyl-pyrazolo-[5,1-e]-pyrazol;
- 6-Amino-5-methyl-pyrazolo-[5,1-e]-1,2,3-triazol;
- 6-Amino-5-phenyl-pyrazolo-[5,1-e]-1,2,3-triazol;
und deren Additionssalzen mit einer Säure.

## Claims

1. Composition for the oxidation dyeing of keratinous fibres and in particular of human keratinous fibres, such as hair, **characterized in that** it comprises, in a medium appropriate for dyeing:
- as oxidation base, at least one pyrazoloazole compound of formula (I) and/or at least one of its addition salts with an acid: 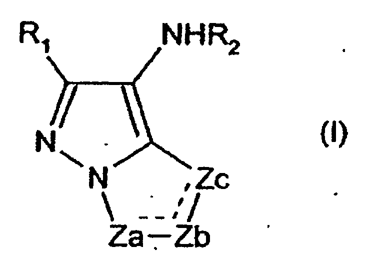
in which:
. R₁ represents: a hydrogen atom; a linear or branched C₁-C₂₀ alkyl radical, optionally substituted by 1 or 2 R radicals chosen from the group consisting of halogen, nitro, cyano, hydroxyl, alkoxy, aryloxy, amino, alkylamino, acylamino, carbamoyl, sulphonamido, sulphamoyl, imido, alkylthio, arylthio, aryl, such as phenyl or naphthyl, (C₁-C₄)alkoxycarbonyl or acyl; an aryl radical (such as phenyl or naphthyl), optionally substituted by 1 or 2 radicals R as defined above; a 5- or 6-membered heterocycle possessing at least one nitrogen, oxygen or sulphur atom (such as pyridyl, quinolyl, pyrrolyl, morpholyl, furanyl, tetrahydrofuranyl, pyrazolyl, triazolyl, tetrazolyl, thiazolyl, oxazolyl, imidazolyl or thiadiazolyl), it being possible for the said heterocycle to be substituted by 1 or 2 radicals R as defined above; a trifluoromethyl radical; a phenyl radical substituted by a trifluoromethyl; or a phenyl ring substituted by a C₁-C₄ alkyl radical;
when R₁ denotes a C₁-C₄ alkyl radical, an aryl radical or a 5- or 6-membered heterocycle (as defined above), it can then be connected to the carbon atom of the pyrazole nucleus via an oxygen, nitrogen or sulphur atom (in this case, R₁ becomes XR₁ with X = O, NH or S);
R₁ can, in addition, denote a halogen atom (such as bromine, chlorine or fluorine); an acyl radical; a sulphonyl radical; a sulphinyl radical; a phosphonyl radical; a carbamoyl radical; a sulphamoyl radical; a cyano radical; a siloxy radical; an amino radical; an acylamino radical; an acyloxy radical; a carbamoyloxy radical; a sulphonamide radical; an imide radical; a ureido radical; a sulphamoylamino radical; a (C₁-C₄)alkoxycarbonylamino radical; an aryloxycarbonylamino radical; a (C₁-C₄)alkoxycarbonyl radical; an aryloxycarbonyl radical; a carboxyl radical; a hydroxyl radical; or a (CH₂)ₚ-X-(CH₂)_{q}-OR' radical in which p and q are identical or different integers of between 1 and 3 inclusive, R' represents a hydrogen atom or a methyl radical and X denotes an oxygen atom or an NR" group in which R" denotes a hydrogen atom or a methyl radical;
. R₂ denotes a hydrogen atom; a C₁-C₄ alkyl radical; a C₁-C₄ monohydroxyalkyl radical; a C₂-C₄ polyhydroxyalkyl radical; a C₁-C₄ aminoalkyl radical; a (CH₂)ₚ-X-(CH₂)_{q}OR' radical in which p and q are identical or different integers of between 1 and 3 inclusive, R' represents a hydrogen atom or a methyl radical and X denotes an oxygen atom or an NR" group in which R" represents a hydrogen atom or a methyl radical; a (C₁-C₄)alkoxy (C₂-C₄)alkyl radical; or a di (C₁-C₄)alkylamino(C₁-C₄)alkyl radical;
. Zₐ, Z_{b} and Z_{c} represent, independently of one another, a nitrogen atom or a carbon atom carrying an R₃ or R₄ radical, R₃ and R₄ having the same meanings as those indicated above for the R₁ radical and also phenyl substituted by a C₁-C₄ alkyl radical; a methanesulphonyl radical; with the proviso that at least one of the Zₐ, Z_{b} and Z_{c} radicals is other than a carbon atom; R₃ and R₄ can also together form a substituted or unsubstituted aromatic ring, such as a phenyl ring.

2. Composition according to Claim 1, **characterized in that** the R₁ radicals of the compounds of formula (I) are chosen from the group consisting of a hydrogen atom; a linear or branched C₁-C₄ alkyl radical; a phenyl ring; a phenyl ring substituted by a halogen atom, a C₁-C₄ alkyl radical, a C₁-C₄ alkoxy radical, a nitro group, an amino group, a trifluoromethyl group or by a C₁-C₄ alkylamino radical; a benzyl radical; a benzyl radical substituted by a halogen atom, a C₁-C₄ alkyl radical, a C₁-C₄ alkoxy radical, a nitro group, an amino group, a trifluoromethyl group or by a C₁-C₄ alkylamino radical; a heterocycle chosen from the thiophene, furan or pyridine rings; a trifluoromethyl radical; a (CH₂)ₚ-X-(CH₂)_{q}-OR' radical in which p and q are identical or different integers of between 1 and 3 inclusive, R' represents a hydrogen atom or a methyl radical and X denotes an oxygen atom or an NR" group in which R" denotes a hydrogen atom or a methyl radical; a C₁-C₄ hydroxyalkyl radical; a C₁-C₄ aminoalkyl radical; a C₁-C₄ alkylamino radical; a di(C₁-C₄)alkylamino radical; an arylamino radical; an alkoxy radical chosen from methoxy, ethoxy and phenoxy; a halogen atom chosen from bromine, chlorine and fluorine; a carboxyl group; a C₁-C₄ alkoxycarbonyl radical; a phenyloxycarbonyl radical; a methylthio radical; an ethylthio radical; a phenylthio radical; a methanesulphonyl radical; a cyano radical; an amino radical; or a hydroxyl radical.

3. Composition according to Claim 1 or 2, **characterized in that** the R₁ radicals are chosen from the group consisting of a hydrogen atom; an alkyl radical chosen from the methyl, ethyl, isopropyl and tert-butyl radicals; a halogen atom chosen from chlorine and fluorine; phenyl; toluyl; 4-chlorophenyl; 4-methoxyphenyl; 3-methoxyphenyl; 2-methoxyphenyl; benzyl; a heterocycle chosen from the pyridyl, furyl and thienyl rings; trifluoromethyl, hydroxymethyl; aminomethyl; methoxy; ethoxy; methylamino; ethylamino; dimethylamino; carboxyl; methoxycarbonyl; ethoxycarbonyl and cyano.

4. Composition according to any one of the preceding claims, **characterized in that** the R₂ radicals of the compounds of formula (I) are chosen from the group consisting of a hydrogen atom; a methyl radical and a β-hydroxyethyl radical.

5. Composition according to any one of the preceding claims, **characterized in that** the R₃ and R₄ radicals of the compounds of formula (I) are chosen from the group consisting of a hydrogen atom; a hydroxyl radical; an amino radical; a linear or branched C₁-C₄ alkyl radical; a trifluoromethyl radical; a phenyl ring; a phenyl ring substituted by one or two R radicals chosen from a halogen atom, a C₁-C₄ alkyl radical, a C₁-C₄ alkoxy radical, a hydroxyl radical, a carboxyl radical, a nitro group, a C₁-C₄ alkylthio radical, a methylenedioxy group, an amino group, a trifluoromethyl group and a C₁-C₄ alkylamino radical; a benzyl radical; a benzyl radical substituted by a halogen atom, a methyl or isopropyl radical or a methoxy radical; a C₁-C₄ monohydroxyalkyl radical; a C₁-C₄ aminoalkyl radical; a (C₁-C₄)alkylamino (C₁-C₄)alkyl radical; an alkoxy radical chosen from the methoxy, ethoxy and phenoxy radicals; a methylthio radical; an ethylthio radical; a phenylthio radical; a methanesulphonyl radical; or a substituted or unsubstituted aromatic ring formed jointly by the R₃ and R₄ radicals, such as a phenyl ring, pyridyl ring or phenyl ring substituted by a sulphonyl radical, a halogen atom, a C₁-C₄ alkoxy radical, a C₁-C₄ alkyl radical, a nitro group, a cyano group, an amino radical, a C₁-C₄ alkylamino radical or by a trifluoromethyl radical.

6. Composition according to Claim 5, **characterized in that** the R₃ and R₄ radicals of the compounds of formula (I) are chosen from the group consisting of a hydrogen atom; an alkyl radical chosen from the methyl, ethyl, isopropyl, n-propyl and tert-butyl radicals; a phenyl ring; a toluyl ring; a 2-, 3-or 4-chlorophenyl ring; a 3- or 4-hydroxyphenyl ring; a 3- or 4-aminophenyl ring; a 3- or 4-methoxyphenyl ring; a 4-trifluoromethylphenyl ring; a benzyl ring; a trifluoromethyl radical; a hydroxymethyl radical; a hydroxyethyl radical; a hydroxyisopropyl radical; an aminomethyl or aminoethyl radical; a methoxy or ethoxy radical; a methylthio or ethylthio radical; or a substituted or unsubstituted aromatic ring formed jointly by the R₃ and R₄ radicals, such as a phenyl, toluyl, sulphonylphenyl and chlorophenyl ring.

7. Composition according to any one of the preceding claims, **characterized in that** the compounds of formula (I) are chosen from:
i) the pyrazolo[1,5-b]-1,2,4-triazoles of following specific formula (Ia) and their addition salts with an acid: 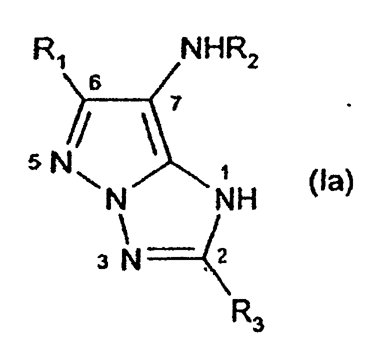
ii) the pyrazolo[3,2-c]-1,2,4-triazoles of following specific formula (Ib) and their addition salts with an acid: 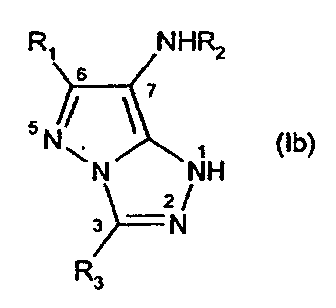
iii) the pyrazolotetrazoles of following specific formula (Ic) and their addition salts with an acid: 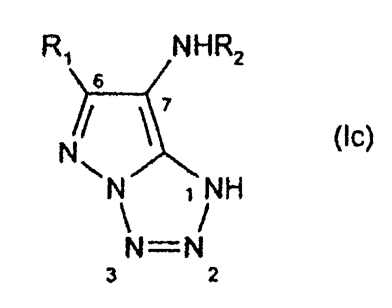
iv) the pyrazolo[1,5-a]imidazoles of following specific formula (Id) and their addition salts with an acid: 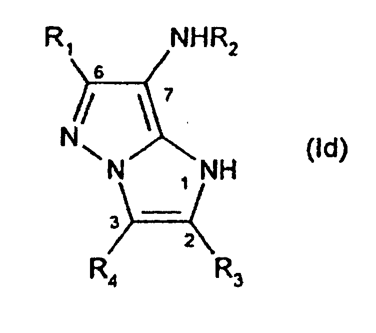
v) the pyrazolo[5,1-e]pyrazoles of following specific formula (Ie) and their addition salts with an acid: 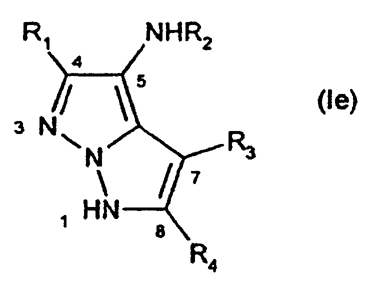
vi) the pyrazolo[5,1-e]-1,2,3-triazoles of following specific formula (If) and their addition salts with an acid: 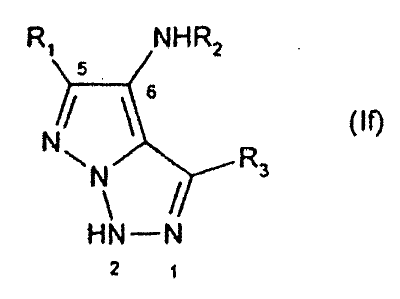
in which formulae R₁, R₂, R₃ and R₄ have the same meanings as those indicated in any one of Claims 1 to 6.

8. Composition according to Claim 7, **characterized in that** the compounds of formula (Ia) or (Ib) are chosen from those in which:
- R₁ denotes a hydrogen atom or a methyl, trifluoromethyl, carboxyl, phenyl, ethoxy, cyano, ethylthio, amino or hydroxyl radical;
- R₂ denotes a hydrogen atom;
- R₃ denotes a hydrogen atom or a methyl, β-aminoethyl, ethyl, isopropyl, phenyl, β-hydroxyethyl, methylthio or ethoxy radical.

9. Composition according to Claim 7, **characterized in that** the compounds of formula (Ic) are chosen from those in which:
- R₁ denotes a hydrogen atom or a methyl, trifluoromethyl, carboxyl, phenyl, ethoxy or cyano radical;
- R₂ denotes a hydrogen atom.

10. Composition according to Claim 7, **characterized in that** the compounds of formula (Id) are chosen from those in which:
- R₁ denotes a hydrogen atom or a methyl, trifluoromethyl, carboxyl, phenyl, ethoxy, cyano, amino, ethylthio or hydroxyl radical;
- R₂ denotes a hydrogen atom;
- R₃ and R₄ respectively denote a hydrogen atom and a hydrogen atom, a hydrogen atom and a methyl radical, a methyl radical and a hydrogen atom, a hydrogen atom and a phenyl radical, or a hydroxyl radical and a hydrogen atom; or R₃ and R₄ jointly form a phenyl ring.

11. Composition according to Claim 7, **characterized in that** the compounds of formula (Ie) are chosen from those in which:
- R₁ denotes a hydrogen atom or a methyl, trifluoromethyl, carboxyl, phenyl, ethoxy or cyano radical;
- R₂ denotes a hydrogen atom;
- R₃ and R₄ respectively denote a hydrogen atom and a methyl radical, a methyl radical and a hydrogen atom, a methyl radical and a methyl radical, a hydrogen atom and a phenyl radical, or a hydrogen atom and an amino radical.

12. Composition according to Claim 7, **characterized in that** the compounds of formula (If) are chosen from those in which:
- R₁ denotes a hydrogen atom or a methyl, trifluoromethyl, carboxyl, phenyl, ethoxy or cyano radical;
- R₂ denotes a hydrogen atom;
- R₃ denotes a hydrogen atom or a methyl radical.

13. Composition according to Claim 7, **characterized in that** the compounds of formula (I) are chosen from:
- 7-amino-2-methylpyrazolo[1,5-b]-1,2,4-triazole;
- 7-amino-2-ethylpyrazolo[1,5-b]-1,2,4-triazole;
- 7-amino-2-isopropylpyrazolo[1,5-b]-1,2,4-triazole;
- 7-amino-2-phenylpyrazolo[1,5-b]-1,2,4-triazole;
- 7-amino-2,6-dimethylpyrazolo[1,5-b]-1,2,4-triazole;
- 7-amino-6-methyl-2-ethylpyrazolo[1,5-b]-1,2,4-triazole;
- 7-amino-6-methyl-2-isopropylpyrazolo[1,5-b]-1,2,4-triazole;
- 7-amino-6-methyl-2-phenylpyrazolo[1,5-b]-1,2,4-triazole;
- 7-amino-6-carboxy-2-methylpyrazolo[1,5-b]-1,2,4-triazole;
- 7-amino-6-carboxy-2-ethylpyrazolo[1,5-b]-1,2,4-triazole;
- 7-amino-6-carboxy-2-isopropylpyrazolo[1,5-b]-1,2,4-triazole;
- 7-amino-6-carboxy-2-phenylpyrazolo[1,5-b]-1,2,4-triazole;
- 7-amino-6-phenyl-2-methylpyrazolo[1,5-b]-1,2,4-triazole;
- 7-amino-6-phenyl-2-ethylpyrazolo[1,5-b]-1,2,4-triazole;
- 7-amino-6-phenyl-2-isopropylpyrazolo[1,5-b]-1,2,4-triazole;
- 6,7-diamino-2-methylpyrazolo[1,5-b]-1,2,4-triazole;
- 6,7-diamino-2-ethylpyrazolo[1,5-b]-1,2,4-triazole;
- 6,7-diamino-2-isopropylpyrazolo[1,5-b]-1,2,4-triazole;
- 6,7-diamino-2-phenylpyrazolo[1,5-b]-1,2,4-triazole;
- 7-amino-6-ethylthio-2-methylpyrazolo[1,5-b]-1,2,4-triazole;
- 7-amino-6-ethylthio-2-ethylpyrazolo[1,5-b]-1,2,4-triazole;
- 7-amino-6-ethylthio-2-isopropylpyrazolo[1,5-b]-1,2,4-triazole;
- 7-amino-6-ethylthio-2-phenylpyrazolo[1,5-b]-1,2,4-triazole;
- 7-amino-6-ethoxy-2-methylpyrazolo[1,5-b]-1,2,4-triazole;
- 7-amino-6-ethoxy-2-ethylpyrazolo[1,5-b]-1,2,4-triazole;
- 7-amino-6-ethoxy-2-isopropylpyrazolo[1,5-b]-1,2,4-triazole;
- 7-amino-6-ethoxy-2-phenylpyrazolo[1,5-b]-1,2,4-triazole;
- 7-amino-6-methyl-2-(2'-aminoethyl)pyrazolo[1,5-b]-1,2,4-triazole;
- 7-amino-6-carboxy-2-(2'-aminoethyl)pyrazolo[1,5-b]-1,2,4-triazole;
- 7-amino-6-phenyl-2-(2'-aminoethyl)pyrazolo[1,5-b]-1,2,4-triazole;
- 7-amino-6-ethylthio-2-(2'-aminoethyl)pyrazolo[1,5-b]-1,2,4-triazole;
- 7-amino-2-(2'-aminoethyl)pyrazolo[1,5-b]-1,2,4-triazole;
- 7-amino-2-(2'-hydroxyethyl)pyrazolo[1,5-b]-1,2,4-triazole;
- 7-amino-6-methyl-2-(2'-hydroxyethyl)pyrazolo[1,5-b]-1,2,4-triazole;
- 7-amino-6-ethylthio-2-(2'-hydroxyethyl)pyrazolo[1,5-b]-1,2,4-triazole;
- 7-amino-6-carboxy-2-(2'-hydroxyethyl)pyrazolo[1,5-b]-1,2,4-triazole;
- 7-amino-6-phenyl-2-(2'-hydroxyethyl)pyrazolo[1,5-b]-1,2,4-triazole;
- 6,7-diamino-2-(2'-aminoethyl)pyrazolo[1,5-b]-1,2,4-triazole;
- 6,7-diamino-2-(2'-hydroxyethyl)pyrazolo[1,5-b]-1,2,4-triazole;
- 7-amino-6-ethoxy-2-(2'-aminoethyl)pyrazolo[1,5-b]-1,2,4-triazole;
- 7-amino-6-ethoxy-2-(2'-hydroxyethyl)pyrazolo[1,5-b]-1,2,4-triazole;
- 7-amino-3-methylpyrazolo[3,2-c]-1,2,4-triazole;
- 7-amino-3-ethylpyrazolo[3,2-c]-1,2,4-triazole;
- 7-amino-3-isopropylpyrazolo[3,2-c]-1,2,4-triazole;
- 7-amino-3-phenylpyrazolo[3,2-c]-1,2,4-triazole;
- 7-amino-3-(2'-aminoethyl)pyrazolo[3,2-c]-1,2,4-triazole;
- 7-amino-3-(2'-hydroxyethyl)pyrazolo[3,2-c]-1,2,4-triazole;
- 7-amino-6-methyl-3-ethylpyrazolo[3,2-c]-1,2,4-triazole;
- 7-amino-3,6-dimethylpyrazolo[3,2-c]-1,2,4-triazole;
- 7-amino-6-methyl-3-isopropylpyrazolo[3,2-c]-1,2,4-triazole;
- 7-amino-6-methyl-3-phenylpyrazolo[3,2-c]-1,2,4-triazole;
- 7-amino-6-methyl-3-(2'-aminoethyl)pyrazolo[3,2-c]-1,2,4-triazole;
- 7-amino-6-methyl-3-(2'-hydroxyethyl)pyrazolo[3,2-c]-1,2,4-triazole;
- 7-amino-6-methyl-3-(methylthio)pyrazolo[3,2-c]-1,2,4-triazole;
- 7-amino-6-phenyl-3-methylpyrazolo[3,2-c]-1,2,4-triazole;
- 7-amino-6-phenyl-3-ethylpyrazolo[3,2-c]-1,2,4-triazole;
- 7-amino-6-phenyl-3-isopropylpyrazolo[3,2-c]-1,2,4-triazole;
- 7-amino-6-phenyl-3-phenylpyrazolo[3,2-c]-1,2,4-triazole;
- 7-amino-6-phenyl-3-(2'-aminoethyl)pyrazolo[3,2-c]-1,2,4-triazole;
- 7-amino-6-phenyl-3-(2'-hydroxyethyl)pyrazolo[3,2-c]-1,2,4-triazole;
- 7-amino-6-phenyl-3-(methylthio)pyrazolo[3,2-c]-1,2,4-triazole;
- 7-amino-6-ethylthio-3-methylpyrazolo[3,2-c]-1,2,4-triazole;
- 7-amino-6-ethylthio-3-ethylpyrazolo[3,2-c]-1,2,4-triazole;
- 7-amino-6-ethylthio-3-isopropylpyrazolo[3,2-c]-1,2,4-triazole;
- 7-amino-6-ethylthio-3-phenylpyrazolo[3,2-c]-1,2,4-triazole;
- 7-amino-6-ethylthio-3-(2'-aminoethyl)pyrazolo[3,2-c]-1,2,4-triazole;
- 7-amino-6-ethylthio-3-(2'-hydroxyethyl)pyrazolo[3,2-c]-1,2,4-triazole;
- 7-amino-6-trifluoromethyl-3-(methylthio)pyrazolo[3,2-c]-1,2,4-triazole;
- 7-amino-6-(trifluoromethyl)pyrazolo[3,2-c]-1,2,4-triazole;
- 7-amino-6-carboxy-3-methylpyrazolo[3,2-c]-1,2,4-triazole;
- 7-amino-6-carboxy-3-ethylpyrazolo[3,2-c]-1,2,4-triazole;
- 7-amino-6-carboxy-3-isopropylpyrazolo[3,2-c]-1,2,4-triazole;
- 7-amino-6-carboxy-3-phenylpyrazolo[3,2-c]-1,2,4-triazole;
- 7-amino-6-carboxy-3-(2'-aminoethyl)pyrazolo[3,2-c]-1,2,4-triazole;
- 7-amino-6-carboxy-3-(2'-hydroxyethyl)pyrazolo[3,2-c]-1,2,4-triazole;
- 6,7-diamino-3-methylpyrazolo[3,2-c]-1,2,4-triazole;
- 6,7-diamino-3-ethylpyrazolo[3,2-c]-1,2,4-triazole;
- 6,7-diamino-3-phenylpyrazolo[3,2-c]-1,2,4-triazole;
- 6,7-diamino-3-isopropylpyrazolo[3,2-c]-1,2,4-triazole;
- 7-amino-6-ethoxy-3-methylpyrazolo[3,2-c]-1,2,4-triazole;
- 7-amino-6-ethoxy-3-ethylpyrazolo[3,2-c]-1,2,4-triazole;
- 7-amino-6-ethoxy-3-isopropylpyrazolo[3,2-c]-1,2,4-triazole;
- 7-amino-6-ethoxy-3-phenylpyrazolo[3,2-c]-1,2,4-triazole;
- 7-amino-6-ethoxy-3-(2'-aminoethyl)pyrazolo[3,2-c]-1,2,4-triazole;
- 7-amino-6-ethoxy-3-(2'-hydroxyethyl)pyrazolo[3,2-c]-1,2,4-triazole;
- 6,7-diamino-3-(2'-aminoethyl)pyrazolo[3,2-c]-1,2,4-triazole;
- 6,7-diamino-3-(2'-hydroxyethyl)pyrazolo[3,2-c]-1,2,4-triazole;
- 7-amino-6-methylpyrazolo[5,1-e]tetrazole;
- 7-amino-6-phenylpyrazolo[5,1-e]tetrazole;
- 7-amino-6-carboxypyrazolo[5,1-e]tetrazole;
- 7-amino-2-methylpyrazolo[1,5-a]imidazole;
- 7-amino-2-phenylpyrazolo[1,5-a]imidazole;
- 7-aminopyrazolo[1,5-a]benzimidazole;
- 7-amino-6-methylpyrazolo[1,5-a]imidazole;
- 7-amino-2,6-dimethylpyrazolo[1,5-a]imidazole;
- 7-amino-6-methyl-2-phenylpyrazolo[1,5-a]imidazole;
- 7-amino-6-methylpyrazolo[1,5-a]benzimidazole;
- 7-amino-6-phenylpyrazolo[1,5-a]imidazole;
- 7-amino-6-phenyl-2-methylpyrazolo[1,5-a]imidazole;
- 7-amino-2,6-diphenylpyrazolo[1,5-a]imidazole;
- 7-amino-6-phenylpyrazolo[1,5-a]benzimidazole;
- 7-amino-6-carboxypyrazolo[1,5-a]imidazole;
- 7-amino-6-carboxy-2-methylpyrazolo[1,5-a]imidazole;
- 7-amino-6-carboxy-2-phenylpyrazolo[1,5-a]imidazole;
- 7-amino-6-carboxypyrazolo[1,5-a]benzimidazole;
- 7-amino-6-ethoxypyrazolo[1,5-a]imidazole;
- 7-amino-6-ethoxy-2-methylpyrazolo[1,5-a]imidazole;
- 7-amino-6-ethoxy-2-phenylpyrazolo[1,5-a]imidazole;
- 7-amino-6-ethoxypyrazolo[1,5-a]benzimidazole;
- 7-amino-6-(trifluoromethyl)pyrazolo[1,5-a]-benzimidazole;
- 6,7-diaminopyrazolo[1,5-a]imidazole;
- 6,7-diamino-2-methylpyrazolo[1,5-a]imidazole;
- 6,7-diamino-2-phenylpyrazolo[1,5-a]imidazole;
- 6,7-diaminopyrazolo[1,5-a]benzimidazole;
- 7-amino-6-(ethylthio)pyrazolo[1,5-a]imidazole;
- 7-amino-6-ethylthio-2-methylpyrazolo[1,5-a]imidazole;
- 7-amino-6-ethylthio-2-phenylpyrazolo[1,5-a]imidazole;
- 7-amino-6-methyl-2-hydroxypyrazolo[1,5-a]imidazole;
- 7-amino-6-phenyl-2-hydroxypyrazolo[1,5-a]imidazole;
- 7-amino-6-ethoxy-2-hydroxypyrazolo[1,5-a]imidazole;
- 7-amino-6-ethylthio-2-hydroxypyrazolo[1,5-a]imidazole;
- 6,7-diamino-2-hydroxypyrazolo[1,5-a]imidazole;
- 5,8-diamino-4-methylpyrazolo[5,1-e]pyrazole;
- 5,7,8-triamino-4-methylpyrazolo[5,1-e]pyrazole;
- 6-amino-5-methylpyrazolo[5,1-e]-1,2,3-triazole;
- 6-amino-5-phenylpyrazolo[5,1-e]-1,2,3-triazole;
and their addition salts with an acid.

14. Composition according to any one of the preceding claims, **characterized in that** the compound or compounds of formula (I) represent from 0.0005 to 12% by weight of the total weight of the dyeing composition.

15. Composition according to Claim 14, **characterized in that** the compound or compounds of formula (I) represent from 0.005 to 6% by weight of the total weight of the dyeing composition.

16. Composition according to any one of the preceding claims, **characterized in that** the medium appropriate for dyeing (or vehicle) is composed of water or of a mixture of water and of at least one organic solvent.

17. Composition according to any one of the preceding claims, **characterized in that** it exhibits a pH of between 3 and 12 and preferably between 5 and 11.

18. Composition according to any one of the preceding claims, **characterized in that** it includes one or more couplers chosen from meta-phenylenediamines, meta-aminophenols, meta-diphenols and heterocyclic couplers.

19. Composition according to Claim 18, **characterized in that** the couplers are chosen from 2-methyl-5-aminophenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethyl)amino-1-methoxybenzene, 1,3-diaminobenzene, 1,3-bis(2,4-diaminophenoxy)-propane, sesamol, α-naphthol, 6-hydroxyindole, 4-hydroxyindole, 4-hydroxy-N-methylindole, 6-hydroxyindoline, 2,6-dihydroxy-4-methylpyridine, 1H-3-methylpyrazol-5-one, 1-phenyl-3-methylpyrazol-5-one, 2,6-dimethylpyrazolo[1,5-b]-1,2,4-triazole, 2,6-dimethyl[3,2-c]-2,2,4-triazole, 6-methylpyrazolo[1,5-a]benzimidazole, and their addition salts with an acid.

20. Composition according to either one of Claims 18 and 19, **characterized in that** the coupler or couplers represent from 0.0001 to 10% by weight of the total weight of the dyeing composition.

21. Composition according to any one of the preceding claims, **characterized in that** it comprises at least one additional oxidation base chosen from para-phenylenediamines, bisphenylalkylenediamines, para-aminophenols, ortho-aminophenols and heterocyclic bases other than the pyrazoloazole compounds of formula (I).

22. Composition according to any one of the preceding claims, **characterized in that** the addition salts with an acid are chosen from hydrochlorides, hydrobromides, sulphates, citrates, succinates, tartrates, tosylates, benzenesulphonates, lactates and acetates.

23. Use of the compounds of formula (I) as defined in any one of Claims 1 to 13 and 22 as oxidation base in compositions for the oxidation dyeing of keratinous fibres and in particular of human keratinous fibres, such as hair.

24. Process for the dyeing of keratinous fibres and in particular of human keratinous fibres, such as hair, **characterized in that** at least one dyeing composition as defined in any one of Claims 1 to 22 is applied to the fibres for a time sufficient to develop the desired colouring, either with air or using an oxidizing agent.

25. Dyeing process according to Claim 24, **characterized in that** the fibres are coloured without addition of an oxidizing agent, with contact only with atmospheric oxygen.

26. Dyeing process according to Claim 24, **characterized in that** the colour is developed at acidic, neutral or alkaline pH using an oxidizing agent which is added only at the time of use to the dyeing composition or which is present in an oxidizing composition applied simultaneously or sequentially in a separate fashion.

27. Process according to Claim 26, **characterized in that** the agent is chosen from hydrogen peroxide, urea hydrogen peroxide, alkali metal bromates, persalts, such as perborates and persulphates, and enzymes, such as peroxidases and 2-electron oxidoreductases.

28. Dyeing multi-compartment device or kit with several compartments, a first compartment of which includes a dyeing composition as defined in any one of Claims 1 to 22 and a second compartment of which includes an oxidizing composition.

29. Compounds of formula (I) and their addition salts with an acid of following formulae (I'a) to (I'f): 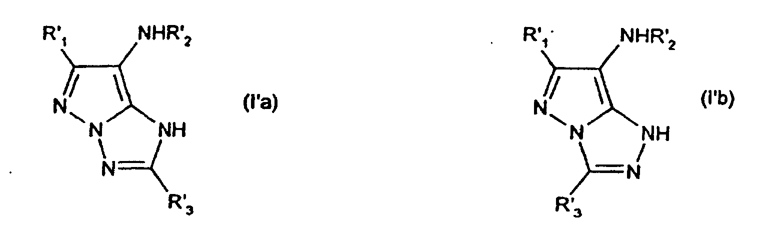 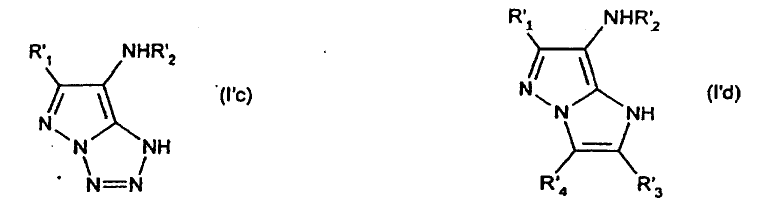 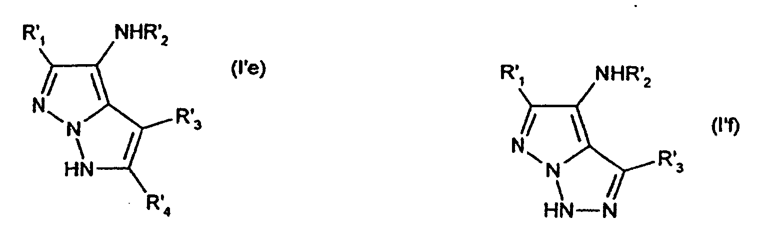 in which the R'₁, R'₂, R'₃ and R'₄ radicals have the same meanings as those indicated in any one of Claims 1 to 12 for the R₁, R₂, R₃ and R₄ radicals of the formula (I); it being understood that:
- in the compounds of formula (I'b) :
i) when R'₁ represents a methyl radical and when R'₂ represents a hydrogen atom, then the R'₃ radical is other than a methyl radical or than a (CH₂)₃-SO₂-(CH₂)₁₅-CH₃ or CH(CH₃)-CH₂-SO₂-(CH₂)₁₁-CH₃ radical;
ii) when R'₁ represents a tert-butyl radical and when R'₂ represents a hydrogen atom, then R'₃ is other than a (CH₂)₃-SO₂-(CH₂)₁₁-CH₃ radical;
- in the compounds of formula (I'd):
- when R'₃ and R'₄ together form a benzene nucleus and when R'₂ represents a hydrogen atom, then R'₁ is other than a methyl, -CONH₂ or carboxyl radical;
- when R'₃ and R'₄ together form a benzene nucleus substituted by a sulphonyl radical in the 4 position and when R'₂ represents a hydrogen atom, then R'₁ is other than a phenyl radical, than a -(CH₂)₁₆-CH₃ radical or than a
- COOH radical;
- when R'₃ and R'₄ together form a benzene nucleus substituted by a carboxyl radical in the 4 position and when R'₂ represents a hydrogen atom, then R'₁ is other than a carboxyl radical.

30. Compounds of formula (I') according to Claim 29, **characterized in that** they are chosen from:
- 7-amino-2-methylpyrazolo[1,5-b]-1,2,4-triazole;
- 7-amino-2-ethylpyrazolo[1,5-b]-1,2,4-triazole;
- 7-amino-2-isopropylpyrazolo[1,5-b]-1,2,4-triazole;
- 7-amino-2-phenylpyrazolo[1,5-b]-1,2,4-triazole;
- 7-amino-2,6-dimethylpyrazolo[1,5-b]-1,2,4-triazole;
- 7-amino-6-methyl-2-ethylpyrazolo[1,5-b]-1,2,4-triazole;
- 7-amino-6-methyl-2-isopropylpyrazolo[1,5-b]-1,2,4-triazole;
- 7-amino-6-methyl-2-phenylpyrazolo[1,5-b]-1,2,4-triazole;
- 7-amino-6-carboxy-2-methylpyrazolo[1,5-b]-1,2,4-triazole;
- 7-amino-6-carboxy-2-ethylpyrazolo[1,5-b]-1,2,4-triazole;
- 7-amino-6-carboxy-2-isopropylpyrazolo[1,5-b]-1,2,4-triazole;
- 7-amino-6-carboxy-2-phenylpyrazolo[1,5-b]-1,2,4-triazole;
- 7-amino-6-phenyl-2-methylpyrazolo[1,5-b]-1,2,4-triazole;
- 7-amino-6-phenyl-2-ethylpyrazolo[1,5-b]-2,2,4-triazole;
- 7-amino-6-phenyl-2-isopropylpyrazolo[1,5-b]-1,2,4-triazole;
- 6,7-diamino-2-methylpyrazolo[1,5-b]-1,2,4-triazole;
- 6,7-diamino-2-ethylpyrazolo[1,5-b]-1,2,4-triazole;
- 6,7-diamino-2-isopropylpyrazolo[1,5-b]-1,2,4-triazole;
- 6,7-diamino-2-phenylpyrazolo[1,5-b]-1,2,4-triazole;
- 7-amino-6-ethylthio-2-methylpyrazolo[1,5-b]-1,2,4-triazole;
- 7-amino-6-ethylthio-2-ethylpyrazolo[1,5-b]-1,2,4-triazole;
- 7-amino-6-ethylthio-2-isopropylpyrazolo[1,5-b]-1,2,4-triazole;
- 7-amino-6-ethylthio-2-phenylpyrazolo[1,5-b]-2,2,4-triazole;
- 7-amino-6-ethoxy-2-methylpyrazolo[1,5-b]-1,2,4-triazole;
- 7-amino-6-ethoxy-2-ethylpyrazolo[1,5-b]-1,2,4-triazole;
- 7-amino-6-ethoxy-2-isopropylpyrazolo[1,5-b]-1,2,4-triazole;
- 7-amino-6-ethoxy-2-phenylpyrazolo[1,5-b]-1,2,4-triazole;
- 7-amino-6-methyl-2-(2'-aminoethyl)pyrazolo[1,5-b]-1,2,4-triazole;
- 7-amino-6-carboxy-2-(2'-aminoethyl)pyrazolo[2,5-b]-1,2,4-triazole;
- 7-amino-6-phenyl-2-(2'-aminoethyl)pyrazolo[1,5-b]-1,2,4-triazole;
- 7-amino-6-ethylthio-2-(2'-aminoethyl)pyrazolo[1,5-b]-1,2,4-triazole;
- 7-amino-2-(2'-aminoethyl)pyrazolo[1,5-b]-1,2,4-triazole;
- 7-amino-2-(2'-hydroxyethyl)pyrazolo[1,5-b]-1,2,4-triazole;
- 7-amino-6-methyl-2-(2'-hydroxyethyl)pyrazolo[1,5-b]-1,2,4-triazole;
- 7-amino-6-ethylthio-2-(2'-hydroxyethyl)pyrazolo[1,5-b]-1,2,4-triazole;
- 7-amino-6-carboxy-2-(2'-hydroxyethyl)pyrazolo[1,5-b]-1,2,4-triazole;
- 7-amino-6-phenyl-2-(2'-hydroxyethyl)pyrazolo[1,5-b]-1,2,4-triazole;
- 6,7-diamino-2-(2'-aminoethyl)pyrazolo[1,5-b]-1,2,4-triazole;
- 6,7-diamino-2-(2'-hydroxyethyl)pyrazolo[1,5-b]-1,2,4-triazole;
- 7-amino-6-ethoxy-2-(2'-aminoethyl)pyrazolo[1,5-b]-1,2,4-triazole;
- 7-amino-6-ethoxy-2-(2'-hydroxyethyl)pyrazolo[1,5-b]-1,2,4-triazole;
- 7-amino-3-methylpyrazolo[3,2-c]-1,2,4-triazole;
- 7-amino-3-ethylpyrazolo[3,2-c]-1,2,4-triazole;
- 7-amino-3-isopropylpyrazolo[3,2-c]-1,2,4-triazole;
- 7-amino-3-phenylpyrazolo[3,2-c]-1,2,4-triazole;
- 7-amino-3-(2'-aminoethyl)pyrazolo[3,2-c]-1,2,4-triazole;
- 7-amino-3-(2'-hydroxyethyl)pyrazolo[3,2-c]-1,2,4-triazole;
- 7-amino-6-methyl-3-ethylpyrazolo[3,2-c]-1,2,4-triazole;
- 7-amino-3,6-dimethylpyrazolo[3,2-c]-1,2,4-triazole;
- 7-amino-6-methyl-3-isopropylpyrazolo[3,2-c]-1,2,4-triazole;
- 7-amino-6-methyl-3-phenylpyrazolo[3,2-c]-1,2,4-triazole;
- 7-amino-6-methyl-3-(2'-aminoethyl)pyrazolo[3,2-c]-1,2,4-triazole;
- 7-amino-6-methyl-3-(2'-hydroxyethyl)pyrazolo[3,2-c]-1,2,4-triazole;
- 7-amino-6-methyl-3-(methylthio)pyrazolo[3,2-c]-1,2,4-triazole;
- 7-amino-6-phenyl-3-methylpyrazolo[3,2-c]-1,2,4-triazole;
- 7-amino-6-phenyl-3-ethylpyrazolo[3,2-c]-1,2,4-triazole;
- 7-amino-6-phenyl-3-isopropylpyrazolo[3,2-c]-1,2,4-triazole;
- 7-amino-6-phenyl-3-phenylpyrazolo[3,2-c]-1,2,4-triazole;
- 7-amino-6-phenyl-3-(2'-aminoethyl)pyrazolo[3,2-c]-1,2,4-triazole;
- 7-amino-6-phenyl-3-(2'-hydroxyethyl)pyrazolo[3,2-c]-1,2,4-triazole;
- 7-amino-6-phenyl-3-(methylthio)pyrazolo[3,2-c]-1,2,4-triazole;
- 7-amino-6-ethylthio-3-methylpyrazolo[3,2-c]-1,2,4-triazole;
- 7-amino-6-ethylthio-3-ethylpyrazolo[3,2-c]-1,2,4-triazole;
- 7-amino-6-ethylthio-3-isopropylpyrazolo[3,2-c]-1,2,4-triazole;
- 7-amino-6-ethylthio-3-phenylpyrazolo[3,2-c]-1,2,4-triazole;
- 7-amino-6-ethylthio-3-(2'-aminoethyl)pyrazolo[3,2-c]-1,2,4-triazole;
- 7-amino-6-ethylthio-3-(2'-hydroxyethyl)pyrazolo[3,2-c]-1,2,4-triazole;
- 7-amino-6-trifluoromethyl-3-(methylthio)pyrazolo[3,2-c]-1,2,4-triazole;
- 7-amino-6-(trifluoromethyl)pyrazolo[3,2-c]-1,2,4-triazole;
- 7-amino-6-carboxy-3-methylpyrazolo[3,2-c]-1,2,4-triazole;
- 7-amino-6-carboxy-3-ethylpyrazolo[3,2-c]-1,2,4-triazole;
- 7-amino-6-carboxy-3-isopropylpyrazolo[3,2-c]-1,2,4-triazole;
- 7-amino-6-carboxy-3-phenylpyrazolo[3,2-c]-1,2,4-triazole;
- 7-amino-6-carboxy-3-(2'-aminoethyl)pyrazolo[3,2-c]-1,2,4-triazole;
- 7-amino-6-carboxy-3-(2'-hydroxyethyl)pyrazolo[3,2-c]-1,2,4-triazole;
- 6,7-diamino-3-methylpyrazolo[3,2-c]-1,2,4-triazole;
- 6,7-diamino-3-ethylpyrazolo[3,2-c]-1,2,4-triazole;
- 6,7-diamino-3-phenylpyrazolo[3,2-c]-1,2,4-triazole;
- 6,7-diamino-3-isopropylpyrazolo[3,2-c]-1,2,4-triazole;
- 7-amino-6-ethoxy-3-methylpyrazolo[3,2-c]-1,2,4-triazole;
- 7-amino-6-ethoxy-3-ethylpyrazolo[3,2-c]-2,2,4-triazole;
- 7-amino-6-ethoxy-3-isopropylpyrazolo[3,2-c]-1,2,4-triazole;
- 7-amino-6-ethoxy-3-phenylpyrazolo[3,2-c]-1,2,4-triazole;
- 7-amino-6-ethoxy-3-(2'-aminoethyl)pyrazolo[3,2-c]-1,2,4-triazole;
- 7-amino-6-ethoxy-3-(2'-hydroxyethyl)pyrazolo[3,2-c]-1,2,4-triazole;
- 6,7-diamino-3-(2'-aminoethyl)pyrazolo[3,2-c]-1,2,4-triazole;
- 6,7-diamino-3-(2'-hydroxyethyl)pyrazolo[3,2-c]-1,2,4-triazole;
- 7-amino-6-methylpyrazolo[5,1-e]tetrazole;
- 7-amino-6-phenylpyrazolo[5,1-e]tetrazole;
- 7-amino-6-carboxypyrazolo[5,1-e]tetrazole;
- 7-aminopyrazolo[1,5-a]imidazole;
- 7-amino-2-methylpyrazolo[1,5-a]imidazole;
- 7-amino-2-phenylpyrazolo[1,5-a]imidazole;
- 7-aminopyrazolo[1,5-a]benzimidazole;
- 7-amino-6-methylpyrazolo[1,5-a]imidazole;
- 7-amino-2,6-dimethylpyrazolo[1,5-a]imidazole;
- 7-amino-6-methyl-2-phenylpyrazolo[1,5-a]imidazole;
- 7-amino-6-phenylpyrazolo[1,5-a]imidazole;
- 7-amino-6-phenyl-2-methylpyrazolo[1,5-a]imidazole;
- 7-amino-2,6-diphenylpyrazolo[1,5-a]imidazole;
- 7-amino-6-phenylpyrazolo[1,5-a]benzimidazole;
- 7-amino-6-carboxypyrazolo[1,5-a]imidazole;
- 7-amino-6-carboxy-2-methylpyrazolo[1,5-a]imidazole;
- 7-amino-6-carboxy-2-phenylpyrazolo[1,5-a]imidazole;
- 7-amino-6-ethoxypyrazolo[1,5-a]imidazole;
- 7-amino-6-ethoxy-2-methylpyrazolo[1,5-a]imidazole;
- 7-amino-6-ethoxy-2-phenylpyrazolo[1,5-a]imidazole;
- 7-amino-6-ethoxypyrazolo[1,5-a]benzimidazole;
- 7-amino-6-(trifluoromethyl)pyrazolo[1,5-a]-benzimidazole;
- 6,7-diaminopyrazolo[1,5-a]imidazole;
- 6,7-diamino-2-methylpyrazolo[1,5-a]imidazole;
- 6,7-diamino-2-phenylpyrazolo[1,5-a]imidazole;
- 6,7-diaminopyrazolo[1,5-a]benzimidazole;
- 7-amino-6-(ethylthio)pyrazolo[1,5-a]imidazole;
- 7-amino-6-ethylthio-2-methylpyrazolo[1,5-a]imidazole;
- 7-amino-6-ethylthio-2-phenylpyrazolo[1,5-a]imidazole;
- 7-amino-6-methyl-2-hydroxypyrazolo[1,5-a]imidazole;
- 7-amino-6-phenyl-2-hydroxypyrazolo[1,5-a]imidazole;
- 7-amino-6-ethoxy-2-hydroxypyrazolo[1,5-a]imidazole;
- 7-amino-6-ethylthio-2-hydroxypyrazolo[1,5-a]imidazole;
- 6,7-diamino-2-hydroxypyrazolo[1,5-a]imidazole;
- 5,8-diamino-4-methylpyrazolo[5,1-e]pyrazole;
- 5,7,8-triamino-4-methylpyrazolo[5,1-e]pyrazole;
- 6-amino-5-methylpyrazolo[5,1-e]-1,2,3-triazole;
- 6-amino-5-phenylpyrazolo[5,1-e]-1,2,3-triazole;
and their addition salts with an acid.
